# EUROPEAN PATENT SPECIFICATION

(11) **EP 4 565 870 B1**
(45) Date of publication and mention of the grant of the patent: **07.10.2026**
(21) Application number: 23761405.2
(22) Date of filing: 04.08.2023
(51) Int. Cl.: G01N 21/64, C12Q 1/6806, G16B 25/10

(54) **SYSTEMS AND METHODS FOR IMMUNOFLUORESCENCE QUANTIFICATION**
SYSTEME UND VERFAHREN ZUR IMMUNFLUORESZENZQUANTIFIZIERUNG
SYSTÈMES ET PROCÉDÉS DE QUANTIFICATION D'IMMUNOFLUORESCENCE

(30) Priority: 05.08.2022 US 202263395722 P
(43) Date of publication of application: 11.06.2025
(73) Proprietor: 10X Genomics, Inc., Pleasanton, CA 94588-3260 (US)
(72) Inventor: WILLIAMS, Stephen, R., Pleasanton, CA 94588-3260 (US); NITSCH, William, Pleasanton, CA 94588-3260 (US)
(74) Representative: Grund, Martin
(86) International application number: PCT/US2023/071703
(87) International publication number: WO 2024/031068

(56) References cited:
- US-A1- 2021 155 982
- US-A1- 2021 158 522

## Description

### CROSS REFERENCE TO RELATED PATENT APPLICATIONS

The present application claims priority to United States Provisional Patent Application No. 63/395,722, entitled "SYSTEMS AND METHODS FOR IMMUNOFLUORESCENCE QUANTIFICATION," filed August 5, 2022.

### TECHNICAL FIELD

This specification describes technologies relating to analyzing tissue samples, particularly for use in analyzing spatial analyte data.

### BACKGROUND

Cells within a tissue of a subject have differences in cell morphology and/or function due to varied analyte levels (*e.g.,* gene and/or protein expression) within the different cells. The specific position of a cell within a tissue (*e.g.,* the cell's position relative to neighboring cells or the cell's position relative to the tissue microenvironment) can affect, for instance, the cell's morphology, differentiation, fate, viability, proliferation, behavior, and signaling and cross-talk with other cells in the tissue.

Spatial heterogeneity has been previously studied using techniques that can provide data for a small handful of analytes in the context of an intact tissue or a portion of a tissue or provide a large amount of analyte data for single cells, but do not provide information regarding the position of the single cell or analyte in a parent biological sample (*e.g.,* a tissue sample).

Image data can be utilized to assess the spatial heterogeneity of analyte levels for cells and tissues. To accurately determine the degree of spatial heterogeneity and analyte activity (*e.g.,* transcriptomic, proteomic, *etc*.) within a cell or tissue, it is desirable to align image data associated with a sample of a cell or a tissue with data associated with an array (*e.g.,* a capture spot array) configured to capture analytes from the cell or tissue sample (*e.g.,* spatial analyte data). Such an alignment would provide spatial mapping of the analyte within the sample.

Thus, there is a need in the art for systems and methods that provide analysis of tissue samples using spatial analyte data, and particularly for the alignment and analysis of image data and spatial analyte data.

### SUMMARY

Technical solutions (*e.g.,* computing systems, methods, and non-transitory computer readable storage mediums) for addressing the above-identified need for analysis of spatial analyte data are provided in the present disclosure.

It should be understood that this summary is not an extensive overview of the present disclosure. It is not intended to identify key/critical elements of the present disclosure or to delineate the scope of the present disclosure. Its sole purpose is to present some of the aspects of the present disclosure in a simplified form as a prelude to the more detailed description that is presented later. The invention is defined by the appended claims.

One aspect of the present disclosure provides a method for analyzing a tissue sample. The method comprises obtaining a set of images of the tissue sample while the tissue sample is overlaid on a substrate in a first orientation. The substrate comprises a plurality of capture spots (*e.g.,* at least 1000 capture spots) and each respective image in the set of images is an emission image of the tissue sample upon excitation of the tissue sample at a corresponding excitation wavelength of a corresponding detectable marker, in a set of one or more detectable markers, associated with the respective image.

A first spatial dataset is acquired comprising, for each respective capture spot in the plurality of capture spots, for each respective detectable marker in the set of detectable markers, a measured intensity of the respective capture spot, in the corresponding image in the set of images, indexed by a spatial barcode in a plurality of spatial barcodes that represents the respective capture spot.

A second spatial dataset is acquired comprising nucleic acid quantification data. The nucleic acid quantification data comprises, for each respective capture spot in the plurality of capture spots, for each respective nucleic acid in a plurality of nucleic acids, a corresponding representation of a number of molecules of the respective nucleic acid originating from the tissue sample while the tissue sample is overlaid on a substrate in the first orientation and localized to the respective capture spot by a corresponding spatial barcode, in the plurality of spatial barcodes, associated with the respective capture spot.

The method includes spatially displaying all or a portion of the first spatial dataset and a corresponding all or a portion of the second spatial dataset co-registered to each other by the plurality of spatial barcodes on a display.

In some embodiments, the spatially displaying is interactive. In some embodiments, the method further comprises, responsive to a user interaction, performing an action on all or a portion of the first spatial dataset and the corresponding all or a portion of the second spatial dataset co-registered to each other selected from the group consisting of: (i) zooming, (ii) panning, and (iii) adjusting an opacity of all or a portion of the first spatial dataset or the corresponding all or a portion of the second spatial dataset.

In some embodiments, the obtaining uses fluorescence microscopy to obtain each image in the set of images.

In some embodiments, the method further comprising exposing, prior to the obtaining, the tissue sample on the substrate with each respective detectable marker in the set of detectable markers. In some embodiments, each respective detectable marker in the set of detectable markers is a different fluorescent dye attached to a different antibody. In some embodiments, each respective detectable marker in the set of detectable markers is a fluorophore labeled antibody, a fluorescent label, a radioactive label, a chemiluminescent label, a colorimetric label, or a combination thereof. In some embodiments, a respective detectable marker in the set of detectable markers is live/dead stain, trypan blue, periodic acid-Schiff reaction stain, Masson's trichrome, Alcian blue, van Gieson, reticulin, Azan, Giemsa, Toluidine blue, isamin blue, sudan black and osmium, acridine orange, Bismarck brown, carmine, Coomassie blue, cresyl violet, DAPI, eosin, ethidium bromide, acid fuchsine, hematoxylin, Hoechst stains, iodine, methyl green, methylene blue, neutral red, Nile blue, Nile red, osmium tetroxide, propidium iodide, rhodamine, safranin, or a combination thereof.

In some embodiments, each image in the set of images comprises a corresponding plurality of pixels in the form of an array of pixel values, where the array of pixel values comprises at least 100,000 pixel values.

In some embodiments, the acquiring the second spatial dataset comprises obtaining a plurality of sequence reads, in electronic form, from the plurality of capture spots, where each respective capture spot in the plurality of capture spots includes a corresponding set of 1000 or more capture probes, 2000 or more capture probes, 10,000 or more capture probes, 100,000 or capture more probes, 1 x 10⁶ or more capture probes, 2 x 10⁶ or more capture probes, 5 x 10⁶ capture probes, or 1 x 10⁷ or more capture probes that directly or indirectly associates with one or more nucleic acids from the tissue sample, the plurality of sequence reads comprises sequence reads corresponding to all or portions of the plurality of nucleic acids, the plurality of sequence reads comprises at least 10,000 sequence reads, and each respective sequence read in the plurality of sequence reads includes a spatial barcode of the corresponding capture spot in the plurality of capture spots or a complement thereof. In some such embodiments, all or a subset of the plurality of spatial barcodes is used to localize respective sequence reads in the plurality of sequence reads to corresponding capture spots in the plurality of capture spots, thereby dividing the plurality of sequence reads into a plurality of subsets of sequence reads, each respective subset of sequence reads corresponding to a different capture spot in the plurality of capture spots.

In some embodiments, each capture probe in the respective capture spot includes a poly-A sequence or a poly-T sequence and a unique spatial barcode that characterizes the respective capture spot. In some embodiments, each capture probe in the respective capture spot includes the same spatial barcode from the plurality of spatial barcodes. In some embodiments, each capture probe in the respective capture spot includes a different spatial barcode from the plurality of spatial barcodes. In some embodiments, the obtaining the plurality of sequence reads comprises high-throughput sequencing. In some embodiments, each capture probe in the corresponding set of capture probes of a respective capture spot includes the same spatial barcode from the plurality of spatial barcodes.

In some embodiments, the corresponding representation of a number of molecules of the respective nucleic acid originating from the tissue sample while the tissue sample is overlaid on the substrate in the first orientation and localized to the respective capture spot by a corresponding spatial barcode, in the plurality of barcodes, associated with the respective capture spot is a count of a number of unique sequence reads in the plurality of sequence reads that have the spatial barcode associated with the respective capture spot.

In some embodiments, the plurality of nucleic acids comprises five or more nucleic acids, ten or more nucleic acids, fifty or more nucleic acids, one hundred or more nucleic acids, five hundred or more nucleic acids, 1000 or more nucleic acids, 2000 or more nucleic acids, between 2000 and 100,000 nucleic acids, between 100,000 and 1 x 10⁶ nucleic acids, or more than 1 x 10⁶ nucleic acids.

In some embodiments, the plurality of nucleic acids comprises DNA, RNA, or a combination thereof. In some embodiments, the plurality of nucleic acids comprises mRNA, microRNA, piRNA, nuclear RNA, or a combination thereof.

In some embodiments, the tissue sample is a sectioned tissue sample that has a depth of 30 microns or less. In some embodiments, the tissue sample is a sectioned tissue sample that has a depth of 10 microns or less. In some embodiments, the tissue sample is a sectioned tissue sample that has a depth of between 4 microns and 30 microns.

In some embodiments, each respective capture spot in the plurality of capture spots is contained within a 2 micron by 2 micron square on the substrate. In some embodiments, a distance between a center of each respective capture spot to a neighboring capture spot in the plurality of capture spots on the substrate is between 2 microns and 8 microns. In some embodiments, a shape of each capture spot in the plurality of capture spots on the substrate is a closed-form shape. In some embodiments, the closed-form shape is square or rectangular. In some embodiments, the closed-form shape is elliptic or circular and each capture spot in the plurality of capture spots has a diameter of between 3 microns and 90 microns. In some embodiments, the closed-form shape is elliptic or circular and each capture spot in the plurality of capture spots has a diameter of between 3 microns and 20 microns. In some embodiments, the closed-form shape is square or rectangular and, for each capture spot in the plurality of capture spots, a dimension (*e.g.,* a length and/or a width) of the respective capture spot is between 2 microns and 90 microns. In some embodiments, the closed-form shape is square or rectangular and, for each capture spot in the plurality of capture spots, a dimension (*e.g.,* a length and/or a width) of the respective capture spot is between 2 microns and 20 microns.

In some embodiments, each spatial barcode in the plurality of spatial barcodes encodes a unique predetermined value selected from the set {1, ..., 1024}, {1, ..., 4096}, {1, ..., 16384}, {1, ..., 65536}, {1, ..., 262144}, {1, ..., 1048576}, {1, ..., 4194304}, {1, ..., 16777216}, {1, ..., 67108864}, or {1, ..., 1 x 10¹²}. In some embodiments, each respective capture spot in the plurality of capture spots is at a different position in a two-dimensional array on the substrate. In some embodiments, each capture spot in the plurality of capture spots is attached directly or attached indirectly to the substrate.

In some embodiments, a first detectable marker in the set of detectable markers is indicative of a particular cell type, and the method further comprises removing from the display those portions of the first spatial dataset and the second spatial dataset that are not associated with capture spots in the plurality of capture spots that exhibit at least a threshold amount of the first detectable marker. In some embodiments, the first detectable marker comprises a dye-labeled antibody to a protein that is expressed in or on the particular cell type.

According to the claimed invention, a first detectable marker in the set of detectable markers is indicative of a presence of a cell nucleus, and the method further comprises using a measured intensity of the first detectable marker in each capture spot in the plurality of capture spots as indicated within the first spatial dataset to determine a corresponding estimate of a number of cells in each respective capture spot in the plurality of capture spots. In some embodiments, the method further comprises using the corresponding estimate of a number of cells in each respective capture spot in the plurality of capture spots to exclude, in a clustering or dimension reduction of the second spatial dataset, nucleic acid quantification data from capture spots in the plurality of capture spots that fail to satisfy a cell number threshold.

In some embodiments, a first detectable marker in the set of detectable markers is indicative of a presence of cell nucleus, a second detectable marker in the set of detectable markers is indicative of a presence of cell membrane, and the method further comprises using a measured intensity of the first detectable marker in each capture spot in the plurality of capture spots as indicated within the first spatial dataset to determine a corresponding estimate of a number of cells in each respective capture spot in the plurality of capture spots, and using a pattern of abundance of the second detectable marker in each capture spot in the plurality of capture spots as indicated within the first spatial dataset to validate the corresponding estimate of the number of cells in each respective capture spot in the plurality of capture spots.

In some embodiments, the method further comprises using display of all or the portion of the first spatial dataset and the corresponding all or the portion of the second spatial dataset co-registered to each other to characterize a biological condition in a subject.

Another aspect of the present disclosure provides a computing system including one or more processors and memory storing one or more programs for analyzing a tissue sample. It will be appreciated that this memory can be on a single computer, a network of computers, one or more virtual machines, or in a cloud computing architecture. The one or more programs are configured for execution by the one or more processors and include instructions for performing any of the methods disclosed above.

Still another aspect of the present disclosure provides a computer readable storage medium storing one or more programs to be executed by an electronic device. The one or more programs include instructions for the electronic device to perform a method for analyzing a tissue sample using any of the methods disclosed above. It will be appreciated that the computer readable storage medium can exist as a single computer readable storage medium or any number of component computer readable storage mediums that are physically separated from each other.

Other embodiments are directed to systems, portable consumer devices, and computer readable media associated with methods described herein.

As disclosed herein, any embodiment disclosed herein, when applicable, can be applied to any aspect.

Various embodiments of systems, methods, and devices within the scope of the appended claims each have several aspects, no single one of which is solely responsible for the desirable attributes described herein. Without limiting the scope of the appended claims, some prominent features are described herein. After considering this discussion, and particularly after reading the section entitled "Detailed Description" one will understand how the features of various embodiments are used.

### DESCRIPTION OF DRAWINGS

The following drawings illustrate certain embodiments of the features and advantages of this disclosure. These embodiments are not intended to limit the scope of the appended claims in any manner. Like reference symbols throughout the several views of the patent application indicate like elements.
**FIG. 1** shows an exemplary spatial analysis workflow in accordance with an embodiment of the present disclosure.
**FIG. 2** shows an exemplary spatial analysis workflow in which optional steps are indicated by dashed boxes in accordance with an embodiment of the present disclosure.
**FIGS. 3A** and **3B** show exemplary spatial analysis workflows in which, in FIG. 3A, optional steps are indicated by dashed boxes in accordance with embodiments of the present disclosure.
**FIG. 4** shows an exemplary spatial analysis workflow in which optional steps are indicated by dashed boxes in accordance with an embodiment of the present disclosure.
**FIG. 5** shows an exemplary spatial analysis workflow in which optional steps are indicated by dashed boxes in accordance with an embodiment of the present disclosure.
**FIG. 6** is a schematic diagram showing an example of a barcoded capture probe, as described herein in accordance with an embodiment of the present disclosure.

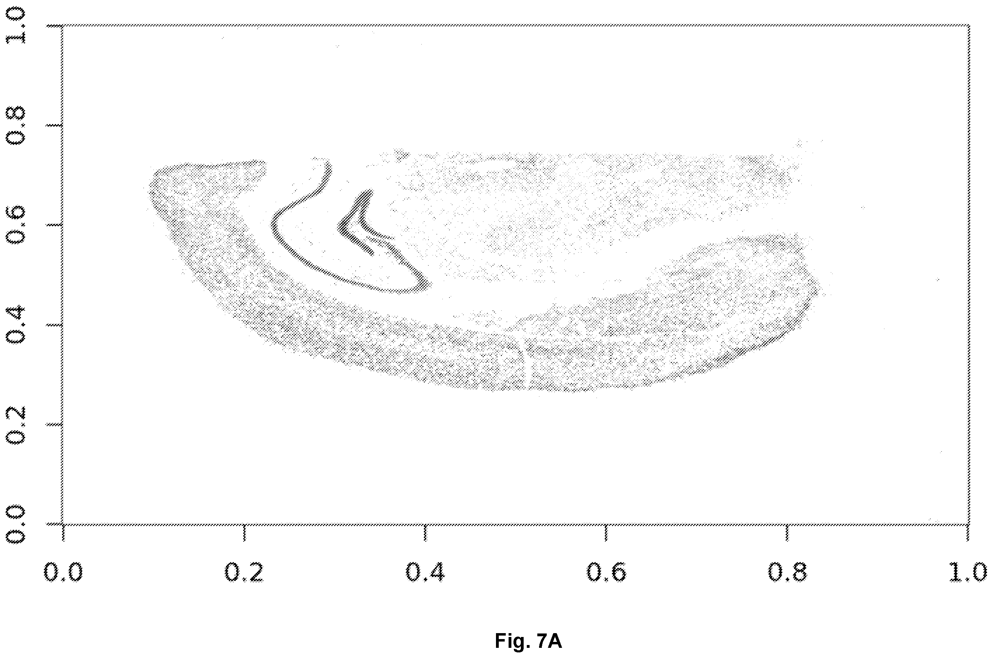

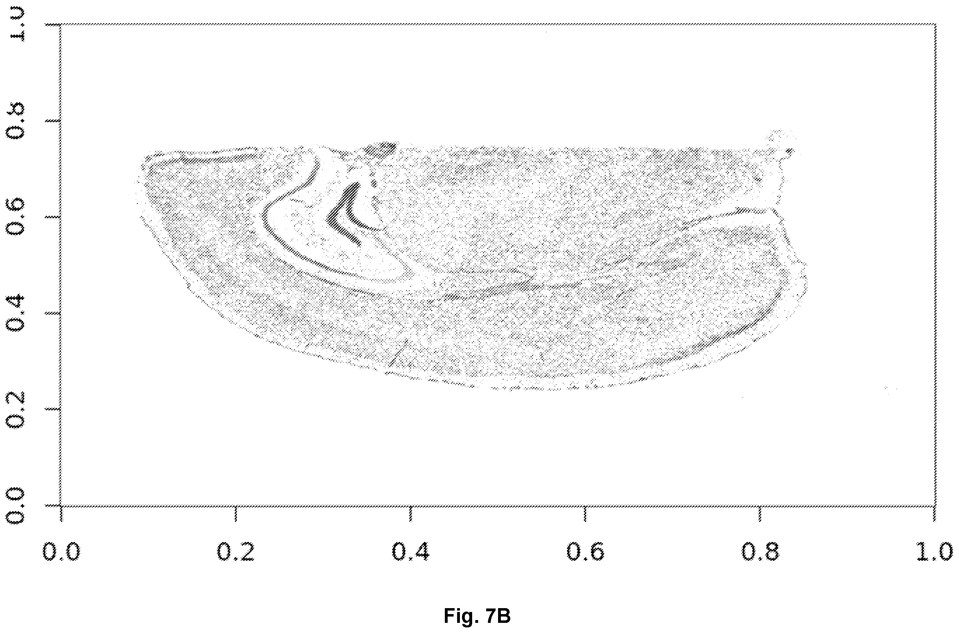

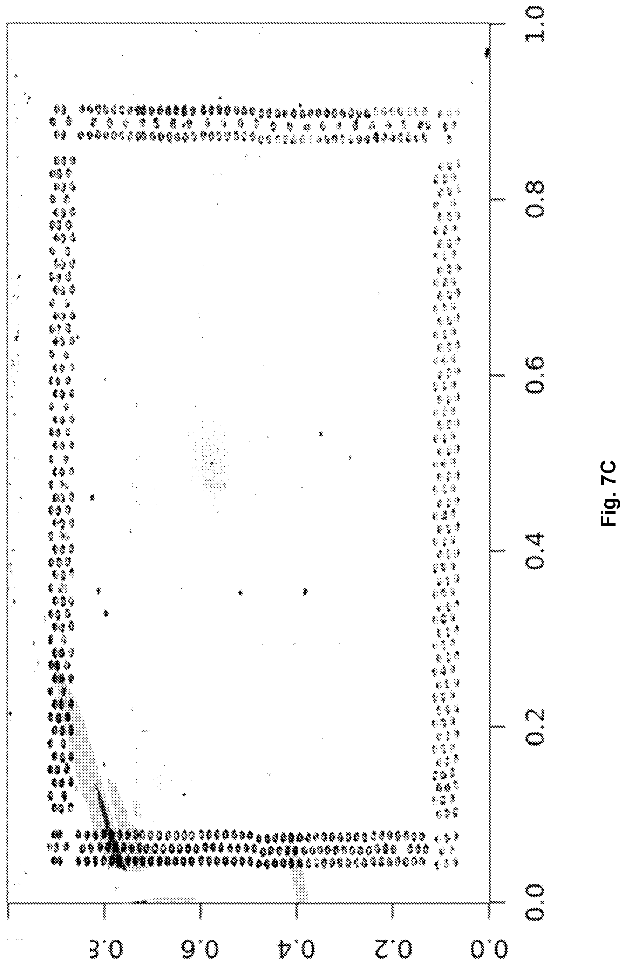

**FIGS. 7A****,** **7B****,** and **7C** collectively show a set of images of an adult mouse brain coronal section on a substrate, where each image in the set of images is obtained at a different excitation wavelength. **FIG. 7A** illustrates a first image obtained at a first excitation wavelength of a corresponding detectable marker FITC and shows staining of the neuronal protein NeuN. **FIG. 7B** illustrates a second image obtained at a second excitation wavelength of a corresponding detectable marker DAPI and shows staining of nucleic acids. **FIG. 7C** illustrates a third image obtained at a third excitation wavelength of a corresponding detectable marker TRITC and shows a plurality of fiducial markers on the substrate.
**FIGS. 8A, 8B****,** **8C,** and **8D** show representations of a first spatial dataset indexed by a plurality of spatial barcodes, where the first spatial dataset comprises measured intensities of detectable markers FITC and DAPI obtained in FIGS. 7A**-C,** for each respective capture spot in the plurality of capture spots.
**FIG. 9** illustrates details of a spatial capture spot and capture probe in accordance with an embodiment of the present disclosure.
**FIGS. 10A****,** **10B****,** **10C****,** **10D****,** and **10E** illustrate non-limiting methods for analyzing a tissue sample, in accordance with some embodiments of the present disclosure, in which optional steps are illustrated by dashed line boxes.
**FIGS. 11A** and **11B** collectively illustrate an example block diagram illustrating a computing device in accordance with some embodiments of the present disclosure.
**FIG. 12** is a schematic showing the arrangement of barcoded capture spots within an array in accordance with some embodiments of the present disclosure.
**FIG. 13** illustrates a biological sample on a first substrate overlayed on a second substrate, in accordance with some embodiments of the present disclosure.
**FIG. 14** illustrates a substrate with an image of a biological sample (e.g., tissue sample) on the substrate, in accordance with an embodiment of the present disclosure.
**FIG. 15** illustrates a substrate that has a number of capture areas and a substrate identifier, in accordance with an embodiment of the present disclosure.
**FIG. 16** illustrates a substrate that has a plurality of fiducial markers and a plurality of capture spots, in accordance with an embodiment of the present disclosure.
**FIG. 17** illustrates a non-limiting method for analyzing a tissue sample, in accordance with some embodiments of the present disclosure, in which optional steps are illustrated by dashed line boxes.

### DETAILED DESCRIPTION

### I. Introduction

This disclosure describes apparatus, systems, methods, and compositions for spatial analysis of biological samples using image registration. This section in particular describes certain general terminology, analytes, sample types, and preparative steps that are referred to in later sections of the disclosure.

Spatial resolution of analytes in tissues provides new insights into the processes underlying biological function and morphology, such as cell fate and development, disease progression and detection, and cellular and tissue-level regulatory networks. *See,* Satija et al., 2015, "Spatial reconstruction of single-cell gene expression data," Nature Biotechnology. 33, 495-502, doi:10.1038.nbt.3192 and Achim et al., 2015, "High-throughput spatial mapping of single-cell RNA-seq data to tissue of origin," Nature Biotechnology 33: 503-509, doi:10.1038/nbt.3209. An understanding of the spatial patterns or other forms of relationships between analytes can provide information on differential cell behavior. This, in turn, can help to elucidate conditions such as diseases.

For example, tissues and cells obtained from a subject (*e.g.,* a human) often have varied analyte levels (*e.g.,* gene and/or protein expression) that can result in differences in cell morphology and/or function. The position of a cell or subset of cells (*e.g.,* neighboring cells and/or non-neighboring cells) within a tissue can affect, for example, the cell's fate, behavior, morphology, signaling and cross-talk with other cells in the tissue. Specifically, in some implementations, the determination that the abundance of an analyte (*e.g*., a gene) is associated with a tissue subpopulation of a particular tissue class (*e.g.,* disease tissue, healthy tissue, the boundary of disease and healthy tissue, etc.) provides inferential evidence of the association of the analyte with a condition such as complex disease. Likewise, the determination that the abundance of an analyte is associated with a particular subpopulation of a heterogeneous cell population in a complex 2-dimensional or 3-dimensional tissue (*e.g.,* a mammalian brain, liver, kidney, heart, a tumor, or a developing embryo of a model organism) provides inferential evidence of the association of the analyte in the particular subpopulation.

Advantageously, information regarding the differences in analyte levels (*e.g.,* gene and/or protein expression) within different cells in a tissue of a mammal can also help physicians select or administer a treatment that will be effective and can allow researchers to identify and elucidate differences in cell morphology and/or cell function in single-cell or multicellular organisms (*e.g.,* a mammal) based on the detected differences in analyte levels within different cells in the tissue. For instance, differences in analyte levels within different cells in a tissue of a mammal can provide information on how tissues (*e.g.,* healthy and diseased tissues) function and/or develop. Differences in analyte levels within different cells in a tissue of a mammal can also provide information on mechanisms of disease pathogenesis, mechanisms of action of therapeutic treatments, and/or drug resistance mechanisms and the development of the same in the tissue. Moreover, differences in the presence or absence of analytes within different cells in a tissue of a multicellular organism (*e.g.,* a mammal) can provide information on drug resistance mechanisms in a tissue of a multicellular organism. Thus, in some embodiments, spatial analysis of analytes can provide information for the early detection of disease by identifying at-risk regions in complex tissues and characterizing the analyte profiles present in these regions through spatial reconstruction (*e.g.,* of gene expression, protein expression, DNA methylation, and/or single nucleotide polymorphisms, among others).

Spatial analysis of analytes can be performed by capturing analytes and/or analyte capture agents or analyte binding domains and mapping them to known locations (*e.g.,* using barcoded capture probes attached to a substrate) using a reference image indicating the tissues or regions of interest that correspond to the known locations. For example, in some implementations of spatial analysis, a sample is prepared (*e.g.,* fresh-frozen tissue is sectioned, placed onto a slide, fixed, and/or stained for imaging). The imaging of the sample provides the reference image to be used for spatial analysis. Analyte detection is then performed using, *e.g.,* analyte or analyte ligand capture via barcoded capture probes, library construction, and/or sequencing. The resulting barcoded analyte data and the reference image can be combined during data visualization for spatial analysis. See, *e.g.,* 10X, 2019, "Inside Visium Spatial Technology." Non-limiting aspects of spatial analysis methodologies are described herein and in WO 2011/127099, WO 2014/210233, WO 2014/210225, WO 2016/162309, WO 2018/091676, WO 2012/140224, WO 2014/060483, U.S. Patent No. 10,002,316, U.S. Patent No. 9,727,810, U.S. Patent No. 10,640,816, Rodriques et al., Science 363(6434):1463-1467, 2019; WO 2018/045186, Lee et al., Nat. Protoc. 10(3):442-458, 2015; WO 2016/007839, WO 2018/045181, WO 2014/163886, Trejo et al., PLoS ONE 14(2):e0212031, 2019, U.S. Patent No. 10,913,975, Chen et al., Science 348(6233):aaa6090, 2015, Gao et al., BMC Biol. 15:50, 2017, WO 2017/144338, WO 2018/107054, WO 2017/222453, WO 2019/068880, WO 2011/094669, U.S. Patent No. 7,709,198, U.S. Patent No. 8,604,182, U.S. Patent No. 8,951,726, U.S. Patent No. 9,783,841, U.S. Patent No. 10,041,949, WO 2016/057552, WO 2017/147483, WO 2018/022809, WO 2016/166128, WO 2017/027367, WO 2017/027368, WO 2018/136856, WO 2019/075091, U.S. Patent No. 10,059,990, WO 2018/057999, WO 2015/161173, Gupta et al., Nature Biotechnol. 36:1197-1202, 2018, and U.S. Patent Publication No. 20210062272A1, entitled "Systems and Methods for Using Spatial Distribution of Haplotypes to Determine a Biological Condition," filed August 13, 2020, and can be used herein in any combination. Spatial analysis of analytes is further described in U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue".

Furthermore, high-resolution spatial mapping of analytes to their specific location within a region or subregion can reveal spatial expression patterns of analytes, provide relational data, and further implicate analyte network interactions relating to disease or other morphologies or phenotypes of interest, resulting in a holistic understanding of cells in their morphological context. See, *e.g.,* 10X, 2019, "Spatially-Resolved Transcriptomics," 10X, 2019, "Inside Visium Spatial Technology," and 10X, 2019, "Visium Spatial Gene Expression Solution".

In some instances, it is advantageous to obtain one or more images (*e.g.,* microscopy images) of a biological sample, such as to obtain histological or morphological information about the biological sample that is preferentially visualized using techniques separate from or prior to preparation of the biological sample for spatial analyte analysis. These techniques can include high-resolution imaging and/or staining methods for detection or localization of particular cells or analyte expression patterns (*e.g.,* immunofluorescence).

An image of a biological sample can be superimposed over spatial analyte data for comparison of the barcoded analyte data with histological, morphological, and/or analyte expression patterns visible by imaging. For instance, as described above, barcoded analyte data and a reference image can be combined during data visualization for spatial analysis of analytes. In some implementations, an image of a biological sample includes multiple "pages" showing different representations of the biological sample. For example, each representation can include an image of the biological sample obtained at a different excitation wavelength. Different excitation wavelengths can be specific for different detectable markers, such as dyes specific for analytes of interest (*e.g.,* fluorophore-coupled antibodies specific for proteins of interest). A user can toggle through different pages of the image to view an overlay of the barcoded analyte data with each of the various representations of the biological sample, or the analyte data can be superimposed over multiple pages simultaneously. Analyte data can thus be viewed spatially by localizing analyte abundance levels within the one or more images of the sample.

In some cases, comparison of barcoded analyte data with the one or more reference images is performed qualitatively. In some such instances, the user performs a visual inspection of the analyte expression patterns, obtained from barcoded analyte data, overlaid on one or more reference images. However, in some instances, it is desirable to perform a quantitative assessment of the relationship between spatial analyte data and any histological, morphological, and/or analyte expression patterns visible in an image of a sample. Thus, there is a need in the art for systems and methods that provide improved analysis of tissue samples by mapping image characteristics (*e.g.,* measurements of pixel intensities) obtained from a reference image to barcoded analyte data.

Advantageously, the present disclosure provides systems and methods for overlaying image data for a biological sample onto spatial analyte data for a plurality of analytes of the biological sample. For example, the presently disclosed methods assign image characteristics (*e.g.,* measurements of pixel intensities) from reference images to barcoded analyte data by aligning the image characteristics to each capture spot in a plurality of capture spots in contact with the tissue sample.

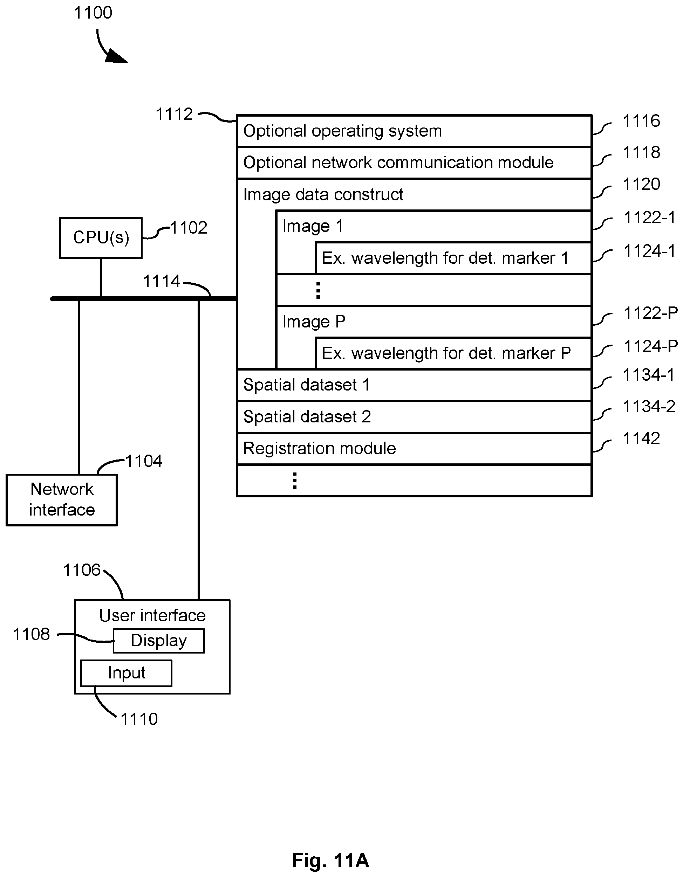

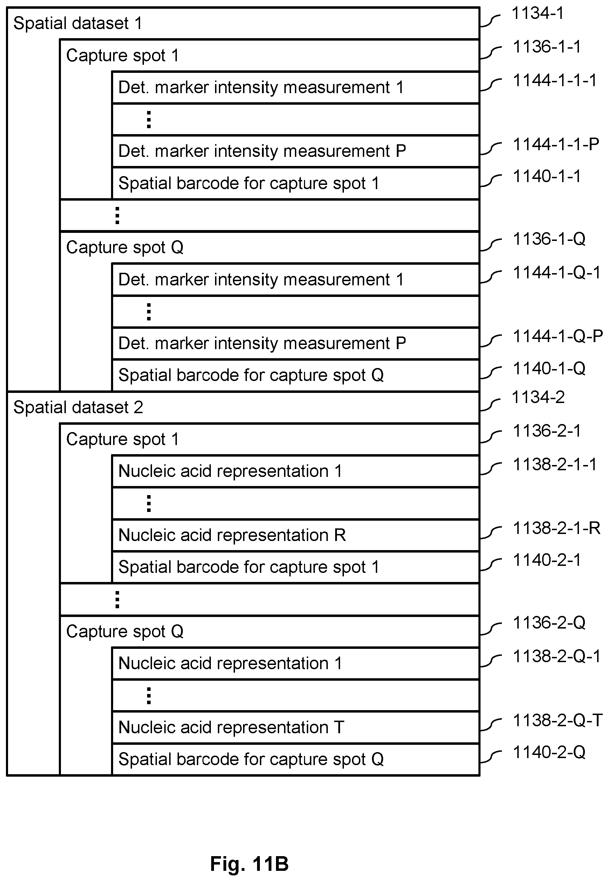

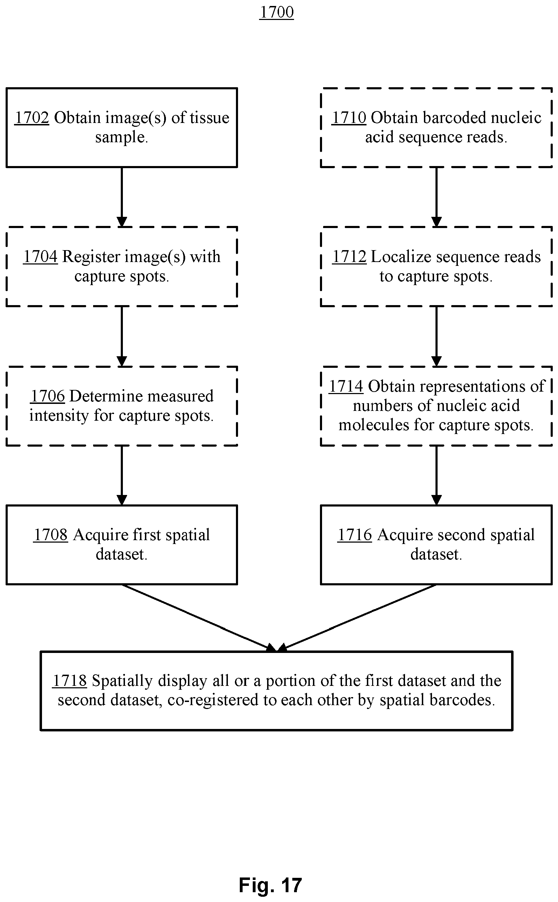

An example overview of a method **1700** in accordance with the present disclosure is provided in **FIG. 17** with further reference to the data structures illustrated in **FIGS. 11A** and **11B****.** The method includes obtaining **1702** a set of images **1122** (*e.g.,* one or more images) of a tissue sample. The tissue sample is overlaid on a substrate in a first orientation, and the substrate includes a plurality of capture spots **1136.** In some implementations, each image in the set of images is an emission image of the tissue sample upon excitation of the tissue sample at a corresponding excitation wavelength of a corresponding detectable marker in a set of detectable markers. For instance, the tissue sample can be prepared for imaging by staining the tissue with one or more detectable markers specific to a corresponding one or more analytes of interest (*e.g.,* proteins). Accordingly, the tissue can be imaged at a corresponding one or more excitation wavelengths such that each image in the set of images is a different "page" (*e.g.,* channel) representing the tissue sample under different imaging conditions. In an example embodiment, the set of images is a set of immunofluorescence images.

Optionally, the method includes inputting the set of images **1122** into an image processing pipeline for visualization, processing, and/or registration. For instance, the method optionally includes registering **1704** the set of images **1122** with the plurality of capture spots **1136.** In some implementations, the registration is fiducial registration (*e.g.,* performed using any of the methods disclosed herein). In some implementations, the registration maps each pixel in a plurality of pixels, for each respective image in the set of images, to a frame of reference for the plurality of capture spots. In some implementations, the registration maps a respective subset of pixels in the plurality of pixels, for each respective image in the set of images, to each corresponding capture spot in the plurality of capture spots.

Optionally, the method further includes determining **1706** a measured intensity **1144** for each respective capture spot **1136** in the plurality of capture spots, for each respective image **1122** in the set of images. In some implementations, the determining the measured intensity further comprises inputting the set of images and a coordinate system representing the registration of each respective image in the set of images to the plurality of capture spots into a data analysis pipeline. In some such implementations, the set of images comprises immunofluorescence images and the data analysis pipeline calculates the immunofluorescence intensity for each respective capture spot in the plurality of capture spots. Each capture spot **1136** is indexed by a spatial barcode **1140** in a plurality of spatial barcodes that represents the respective capture spot. Accordingly, in some embodiments, the method includes assigning a measured intensity (*e.g.,* a measured immunofluorescence intensity), for each respective image in the set of images, to each respective spatial barcode in the plurality of spatial barcodes.

Capture spot intensities can be measured, for each respective image in the set of images, by determining the pixel intensities (*e.g*., pixel values) for each respective pixel in the plurality of pixels of the respective image. For instance, in some embodiments, a respective capture spot intensity is a value obtained by averaging the pixel intensities across the subset of pixels assigned to the corresponding capture spot. As this process is repeated for each image in the set of images, a corresponding spatial dataset of measured intensities assigned to capture spots is generated for each of the images (*e.g.,* "pages" specific to each corresponding excitation wavelength of a corresponding detectable marker in a set of detectable markers and/or specific to different analytes of interest) of the set of images of the tissue sample. A first spatial dataset **1134-1** is thereby acquired **1708,** comprising, for each respective capture spot **1136** in the plurality of capture spots, for each respective detectable marker in the set of detectable markers, a measured intensity **1144** of the respective capture spot, in the corresponding image **1122** in the set of images, indexed by a spatial barcode **1140** in a plurality of spatial barcodes that represents the respective capture spot.

The method further includes acquiring **1716** a second spatial dataset **1134-2,** where the second spatial dataset comprises nucleic acid quantification data. Optionally, the acquiring the second spatial dataset includes obtaining **1710** a plurality of barcoded nucleic acid sequence reads, which can be generated from a sequencing of captured nucleic acid molecules originating from the tissue sample while the tissue sample is overlaid on the substrate in the first orientation. The nucleic acid sequence reads can be localized **1712** to a respective capture spot **1136** using a corresponding spatial barcode **1140,** in the plurality of spatial barcodes, that indicates the respective capture spot at which the nucleic acid molecule was captured. Optionally, the method includes obtaining **1714** representations of the number of molecules of each respective nucleic acid at each respective capture spot. The second spatial dataset therefore comprises, for each respective capture spot **1136** in the plurality of capture spots, for each respective nucleic acid in a plurality of nucleic acids, a corresponding representation **1138** of a number of molecules of the respective nucleic acid originating from the tissue sample while the tissue sample is overlaid on a substrate in the first orientation and localized to the respective capture spot by a corresponding spatial barcode **1140,** in the plurality of spatial barcodes, associated with the respective capture spot.

The method further includes spatially displaying **1718,** on a display, all or a portion of the first spatial dataset **1134-1** and a corresponding all or a portion of the second spatial dataset **1134-2** co-registered to each other by the plurality of spatial barcodes **1140.** In some embodiments, at least the first spatial dataset, second spatial dataset, and, optionally, the set of images are inputted into an image processing pipeline for visualization and analysis. For example, the first spatial dataset **1134-1** and/or the second spatial dataset **1134-2** can be overlaid onto a respective image **1122** in the set of images such that the measured intensity values **1144** and/or the nucleic acid representations **1138** of the plurality of capture spots **1136** are displayed according to their spatial expression patterns within a frame of reference of the respective image. In some embodiments, the measured intensity values and/or the nucleic acid representations are displayed as a heatmap of relative expression and/or abundance values. In some embodiments, the spatial displaying includes displaying a representation (*e.g.,* a clustering and/or a latent representation) of the measured intensity values and/or the nucleic acid representations of the first and/or second spatial dataset.

Advantageously, the systems and methods of the present disclosure improve upon the prior art by allowing for simultaneous, quantitative analysis of spatial analyte data (*e.g.*, obtained from barcoded analyte data) with visual image data of a tissue sample (*e.g.,* immunofluorescence imaging). The presently disclosed systems and methods therefore allow for more robust analysis and reduce the subjectivity and risk of error associated with conventional, qualitative methods that rely on visual inspection and comparison by a user. By obtaining measured intensities (*e.g*., pixel intensities) from images, the presently disclosed systems and methods are not limited to spatial analysis technologies but are applicable for the incorporation and analysis of images obtained using a wide array of imaging technologies. The presently disclosed systems and methods are further amenable to comparison, filtering, dimension reduction analysis, and other analyses using one or both spatial datasets, which can be displayed on linked windows for convenient manipulation and easy interpretation.

Advantageously, the systems and methods disclosed herein also allow for the validation of spatial analyte analysis using additional data derived from imaging. For example, in cases where spatial analyte data for a first analyte of interest is limited or in low abundance (*e.g.,* mRNA expression), imaging data can be used to confirm or validate an associated expression profile and/or a biological condition using a second associated analyte of interest (*e.g.,* protein abundance or cell nuclei).

Imaging data can also be used to isolate regions of interest in a tissue where further spatial analyte analysis should be concentrated. For example, measured intensities for a first analyte of interest can be used to detect populations of interest (*e.g.,* a population of cells in a heterogenous sample characterized by one or more proteins), within which further analysis can be performed using a second analyte of interest (*e.g.,* mRNA expression within the target population of cells).

Thus, the present disclosure advantageously provides systems and methods that allow for improved analysis of tissue samples.

### (A) General Definitions

Specific terminology is used throughout this disclosure to explain various aspects of the apparatus, systems, methods, and compositions that are described. This sub-section includes explanations of certain terms that appear in later sections of the disclosure. To the extent that the descriptions in this section are in apparent conflict with usage in other sections of this disclosure, the definitions in this section will control.

### Analytes

As used herein, the term "analyte" refers to any biological substance, structure, moiety, or component to be analyzed. The term "target" is similarly used herein to refer to an analyte of interest. In some embodiments, the apparatus, systems, methods, and compositions described in this disclosure can be used to detect and analyze a wide variety of different analytes.

Analytes can be broadly classified into one of two groups: nucleic acid analytes, and non-nucleic acid analytes. Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral proteins (*e.g.,* viral capsid, viral envelope, viral coat, viral accessory, viral glycoproteins, viral spike, *etc*.), extracellular and intracellular proteins, antibodies, and antigen binding fragments. In some embodiments, the analyte is an organelle (*e.g.,* nuclei or mitochondria). In some embodiments, the analyte(s) can be localized to subcellular location(s), including, for example, organelles, *e.g.,* mitochondria, Golgi apparatus, endoplasmic reticulum, chloroplasts, endocytic vesicles, exocytic vesicles, vacuoles, lysosomes, *etc.* In some embodiments, analyte(s) can be peptides or proteins, including without limitation antibodies and enzymes. Additional examples of analytes can be found in Section (I)(c) of WO 2020/176788 and/or U.S. Patent Application Publication No. 2020/0277663. In some embodiments, an analyte can be detected indirectly, such as through detection of an intermediate agent, for example, a connected probe (*e.g.,* a ligation product) or an analyte capture agent (*e.g.,* an oligonucleotide-conjugated antibody), such as those described herein. In some embodiments, analytes can include one or more intermediate agents, *e.g.,* connected probes or analyte capture agents that bind to nucleic acid, protein, or peptide analytes in a sample.

Cell surface features corresponding to analytes can include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, an extracellular matrix protein, a posttranslational modification (*e.g.,* phosphorylation, glycosylation, ubiquitination, nitrosylation, methylation, acetylation or lipidation) state of a cell surface protein, a gap junction, and an adherens junction.

Analytes can be derived from a specific type of cell and/or a specific sub-cellular region. For example, analytes can be derived from cytosol, from cell nuclei, from mitochondria, from microsomes, and more generally, from any other compartment, organelle, or portion of a cell. Permeabilizing agents that specifically target certain cell compartments and organelles can be used to selectively release analytes from cells for analysis.

Examples of nucleic acid analytes include DNA analytes such as genomic DNA, methylated DNA, specific methylated DNA sequences, fragmented DNA, mitochondrial DNA, in situ synthesized PCR products, and RNA/DNA hybrids.

Examples of nucleic acid analytes also include RNA analytes such as various types of coding and noncoding RNA. Examples of the different types of RNA analytes include messenger RNA (mRNA), ribosomal RNA (rRNA), transfer RNA (tRNA), microRNA (miRNA), and viral RNA. The RNA can be a transcript (*e.g.,* present in a tissue section). The RNA can be small (*e.g.,* less than 200 nucleic acid bases in length) or large (*e.g.,* RNA greater than 200 nucleic acid bases in length). Small RNAs mainly include 5.8S ribosomal RNA (rRNA), 5S rRNA, transfer RNA (tRNA), microRNA (miRNA), small interfering RNA (siRNA), small nucleolar RNA (snoRNAs), Piwi-interacting RNA (piRNA), tRNA-derived small RNA (tsRNA), and small rDNA-derived RNA (srRNA). The RNA can be double-stranded RNA or single-stranded RNA. The RNA can be circular RNA. The RNA can be a bacterial rRNA (*e.g.,* 16s rRNA or 23s rRNA).

Additional examples of analytes include mRNA and cell surface features (*e.g.,* using the labelling agents described herein), mRNA and intracellular proteins (*e.g.,* transcription factors), mRNA and cell methylation status, mRNA and accessible chromatin (*e.g.,* ATAC-seq, DNase-seq, and/or MNase-seq), mRNA and metabolites (*e.g.,* using the labelling agents described herein), a barcoded labelling agent (*e.g.,* the oligonucleotide tagged antibodies described herein) and a V(D)J sequence of an immune cell receptor (*e.g.,* T-cell receptor), mRNA and a perturbation agent (*e.g.,* a CRISPR crRNA/sgRNA, TALEN, zinc finger nuclease, and/or antisense oligonucleotide as described herein). In some embodiments, a perturbation agent is a small molecule, an antibody, a drug, an aptamer, a miRNA, a physical environmental (*e.g.,* temperature change), or any other known perturbation agents.

Analytes can include a nucleic acid molecule with a nucleic acid sequence encoding at least a portion of a V(D)J sequence of an immune cell receptor (*e.g.,* a TCR or BCR). In some embodiments, the nucleic acid molecule is cDNA first generated from reverse transcription of the corresponding mRNA, using a poly(T) containing primer. The generated cDNA can then be barcoded using a capture probe, featuring a barcode sequence (and optionally, a UMI sequence) that hybridizes with at least a portion of the generated cDNA. In some embodiments, a template switching oligonucleotide hybridizes to a poly(C) tail added to a 3' end of the cDNA by a reverse transcriptase enzyme. The original mRNA template and template switching oligonucleotide can then be denatured from the cDNA and the barcoded capture probe can then hybridize with the cDNA and a complement of the cDNA generated. Additional methods and compositions suitable for barcoding cDNA generated from mRNA transcripts including those encoding V(D)J regions of an immune cell receptor and/or barcoding methods and composition including a template switch oligonucleotide are described in PCT Patent Application No. PCT/US2017/057269, filed October 18, 2017, and U.S. Patent Publication No. US2018-0105808A1. V(D)J analysis can also be completed with the use of one or more labelling agents that bind to particular surface features of immune cells and associated with barcode sequences. The one or more labelling agents can include an MHC or MHC multimer.

As described above, the analyte can include a nucleic acid capable of functioning as a component of a gene editing reaction, such as, for example, clustered regularly interspaced short palindromic repeats (CRISPR)-based gene editing. Accordingly, the capture probe can include a nucleic acid sequence that is complementary to the analyte (*e.g.,* a sequence that can hybridize to the CRISPR RNA (crRNA), single guide RNA (sgRNA), or an adapter sequence engineered into a crRNA or sgRNA).

In certain embodiments, an analyte is extracted from a live cell. Processing conditions can be adjusted to ensure that a biological sample remains live during analysis, and analytes are extracted from (or released from) live cells of the sample. Live cell-derived analytes can be obtained only once from the sample or can be obtained at intervals from a sample that continues to remain in viable condition.

In general, the systems, apparatus, methods, and compositions can be used to analyze any number of analytes. For example, the number of analytes that are analyzed can be at least about 2, at least about 3, at least about 4, at least about 5, at least about 6, at least about 7, at least about 8, at least about 9, at least about 10, at least about 11, at least about 12, at least about 13, at least about 14, at least about 15, at least about 20, at least about 25, at least about 30, at least about 40, at least about 50, at least about 100, at least about 1,000, at least about 10,000, at least about 100,000 or more different analytes present in a region of the sample or within an individual capture spot of the substrate. Methods for performing multiplexed assays to analyze two or more different analytes will be discussed in a subsequent section of this disclosure.

In some embodiments, more than one analyte type (*e.g.,* nucleic acids and proteins) from a biological sample can be detected (*e.g.,* simultaneously or sequentially) using any appropriate multiplexing technique, such as those described in Section (IV) of WO 2020/176788 and/or U.S. Patent Application Publication No. 2020/0277663.

In some embodiments, detection of one or more analytes (*e.g.,* protein analytes) can be performed using one or more analyte capture agents. As used herein, an "analyte capture agent" refers to an agent that interacts with an analyte (*e.g.,* an analyte in a biological sample) and with a capture probe (*e.g.,* a capture probe attached to a substrate or a feature) to identify the analyte. In some embodiments, the analyte capture agent includes: (i) an analyte binding moiety (*e.g.,* that binds to an analyte), for example, an antibody or antigen-binding fragment thereof; (ii) analyte binding moiety barcode; and (iii) a capture handle sequence. As used herein, the term "analyte binding moiety barcode" refers to a barcode that is associated with or otherwise identifies the analyte binding moiety. As used herein, the term "analyte capture sequence" or "capture handle sequence" refers to a region or moiety configured to hybridize to, bind to, couple to, or otherwise interact with a capture domain of a capture probe. In some embodiments, a capture handle sequence is complementary to a capture domain of a capture probe. In some cases, an analyte binding moiety barcode (or portion thereof) may be able to be removed (*e.g.,* cleaved) from the analyte capture agent.

Additional examples of analytes suitable for use in the present disclosure are described in U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue".

### Barcodes

As used herein, the term "barcode" refers to a label, or identifier, that conveys or is capable of conveying information (*e.g*., information about an analyte in a sample, a bead, and/or a capture probe). A barcode can be part of an analyte, or independent of an analyte. A barcode can be attached to an analyte. A particular barcode can be unique relative to other barcodes. Barcodes suitable for use in the present disclosure are further described in U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue".

### Biological samples

As used herein, the term "sample" or "biological sample" refers to any material obtained from a subject for analysis using any of a variety of techniques including, but not limited to, biopsy, surgery, and laser capture microscopy (LCM), and generally includes cells and/or other biological material from the subject. In addition to the subjects described above, a biological sample can also be obtained from non-mammalian organisms (*e.g.,* plants, insects, arachnids, nematodes, fungi, amphibians, and fish. A biological sample can be obtained from a prokaryote such as a bacterium, *e.g., Escherichia coli, Staphylococci* or *Mycoplasma pneumoniae;* archaea; a virus such as Hepatitis C virus or human immunodeficiency virus; or a viroid. A biological sample can also be obtained from a eukaryote, such as a patient derived organoid (PDO) or patient derived xenograft (PDX). The biological sample can include organoids, a miniaturized and simplified version of an organ produced *in vitro* in three dimensions that shows realistic micro-anatomy. Organoids can be generated from one or more cells from a tissue, embryonic stem cells, and/or induced pluripotent stem cells, which can self-organize in three-dimensional culture owing to their self-renewal and differentiation capacities. In some embodiments, an organoid is a cerebral organoid, an intestinal organoid, a stomach organoid, a lingual organoid, a thyroid organoid, a thymic organoid, a testicular organoid, a hepatic organoid, a pancreatic organoid, an epithelial organoid, a lung organoid, a kidney organoid, a gastruloid, a cardiac organoid, or a retinal organoid. Subjects from which biological samples can be obtained can be healthy or asymptomatic individuals, individuals that have or are suspected of having a disease (*e.g.,* cancer) or a predisposition to a disease, and/or individuals that are in need of therapy or suspected of needing therapy.

The biological sample can include any number of macromolecules, for example, cellular macromolecules and organelles (*e.g.,* mitochondria and nuclei). The biological sample can be a nucleic acid sample and/or protein sample. The biological sample can be a nucleic acid sample and/or protein sample. The biological sample can be a carbohydrate sample or a lipid sample. The biological sample can be obtained as a tissue sample, such as a tissue section, biopsy, a core biopsy, needle aspirate, or fine needle aspirate. The sample can be a fluid sample, such as a blood sample, urine sample, or saliva sample. The sample can be a skin sample, a colon sample, a cheek swab, a histology sample, a histopathology sample, a plasma or serum sample, a tumor sample, living cells, cultured cells, a clinical sample such as, for example, whole blood or blood-derived products, blood cells, or cultured tissues or cells, including cell suspensions and/or disaggregated cells.

Cell-free biological samples can include extracellular polynucleotides. Extracellular polynucleotides can be isolated from a bodily sample, *e.g.,* blood, plasma, serum, urine, saliva, mucosal excretions, sputum, stool, and tears.

Biological samples can be derived from a homogeneous culture or population of the subjects or organisms mentioned herein or alternatively from a collection of several different organisms, for example, in a community or ecosystem.

Biological samples can include one or more diseased cells. A diseased cell can have altered metabolic properties, gene expression, protein expression, and/or morphologic features. Examples of diseases include inflammatory disorders, metabolic disorders, nervous system disorders, and cancer. Cancer cells can be derived from solid tumors, hematological malignancies, cell lines, or obtained as circulating tumor cells.

Biological samples can also include fetal cells. For example, a procedure such as amniocentesis can be performed to obtain a fetal cell sample from maternal circulation. Sequencing of fetal cells can be used to identify any of a number of genetic disorders, including, *e.g.,* aneuploidy such as Down's syndrome, Edwards syndrome, and Patau syndrome. Further, cell surface features of fetal cells can be used to identify any of a number of disorders or diseases.

Biological samples can also include immune cells. Sequence analysis of the immune repertoire of such cells, including genomic, proteomic, and cell surface features, can provide a wealth of information to facilitate an understanding the status and function of the immune system. By way of example, determining the status (*e.g.,* negative or positive) of minimal residue disease (MRD) in a multiple myeloma (MM) patient following autologous stem cell transplantation is considered a predictor of MRD in the MM patient (see, *e.g.,* U.S. Patent No. 10,656,144).

Examples of immune cells in a biological sample include, but are not limited to, B cells, T cells (*e.g.,* cytotoxic T cells, natural killer T cells, regulatory T cells, and T helper cells), natural killer cells, cytokine induced killer (CIK) cells, myeloid cells, such as granulocytes (basophil granulocytes, eosinophil granulocytes, neutrophil granulocytes/hyper-segmented neutrophils), monocytes/macrophages, mast cells, thrombocytes/megakaryocytes, and dendritic cells.

As discussed above, a biological sample can include a single analyte of interest, or more than one analyte of interest. Methods for performing multiplexed assays to analyze two or more different analytes in a single biological sample will be discussed in a subsequent section of this disclosure.

A variety of steps can be performed to prepare a biological sample for analysis. Except where indicated otherwise, the preparative steps for biological samples can generally be combined in any manner to appropriately prepare a particular sample for analysis.

For instance, in some embodiments, the biological sample is a tissue section. In some embodiments, the biological sample is prepared using tissue sectioning. A biological sample can be harvested from a subject (*e.g.,* via surgical biopsy, whole subject sectioning, grown *in vitro* on a growth substrate or culture dish as a population of cells, or prepared for analysis as a tissue slice or tissue section). Grown samples may be sufficiently thin for analysis without further processing steps. Alternatively, grown samples, and samples obtained via biopsy or sectioning, can be prepared as thin tissue sections using a mechanical cutting apparatus such as a vibrating blade microtome. As another alternative, in some embodiments, a thin tissue section can be prepared by applying a touch imprint of a biological sample to a suitable substrate material. The thickness of the tissue section can be a fraction of (*e.g.,* less than 0.9, 0.8, 0.7, 0.6, 0.5, 0.4, 0.3, 0.2, or 0.1) the maximum cross-sectional dimension of a cell. However, tissue sections having a thickness that is larger than the maximum cross-section cell dimension can also be used. For example, cryostat sections can be used, which can be, *e.g.,* 10-20 micrometers thick.

More generally, the thickness of a tissue section typically depends on the method used to prepare the section and the physical characteristics of the tissue, and therefore sections having a wide variety of different thicknesses can be prepared and used. For example, the thickness of the tissue section can be at least 0.1, 0.2, 0.3, 0.4, 0.5, 0.7, 1.0, 15, 2, 3, 4, 5, 6, 7, 8, 9, 10, 11, 12, 13, 14, 15, 20, 30, 40, or 50 micrometers. Thicker sections can also be used if desired or convenient, *e.g.,* at least 70, 80, 90, or 100 micrometers or more. Typically, the thickness of a tissue section is between 1-100 micrometers, 1-50 micrometers, 1-30 micrometers, 1-25 micrometers, 1-20 micrometers, 1-15 micrometers, 1-10 micrometers, 2-8 micrometers, 3-7 micrometers, or 4-6 micrometers, but as mentioned above, sections with thicknesses larger or smaller than these ranges can also be analysed.

In some embodiments, a tissue section is a similar size and shape to a substrate (*e.g.,* the first substrate and/or the second substrate). In some embodiments, a tissue section is a different size and shape from a substrate. In some embodiments, a tissue section is on all or a portion of the substrate. For example, **FIG**. **14** illustrates a tissue section with dimensions roughly comparable to the substrate, such that a large proportion of the substrate is in contact with the tissue section. In some embodiments, several biological samples from a subject are concurrently analyzed. For instance, in some embodiments several different sections of a tissue are concurrently analyzed. In some embodiments, 1, 2, 3, 4, 5, 6, 7, 8, 9, 10, 11, 12, 13, 14, 15, 16, 17, 18, 19, or 20 different biological samples from a subject are concurrently analyzed. For example, in some embodiments, 1, 2, 3, 4, 5, 6, 7, 8, 9, 10, 11, 12, 13, 14, 15, 16, 17, 18, 19, or 20 different tissue sections from a single biological sample from a single subject are concurrently analyzed. In some embodiments, one or more images are acquired of each such tissue section.

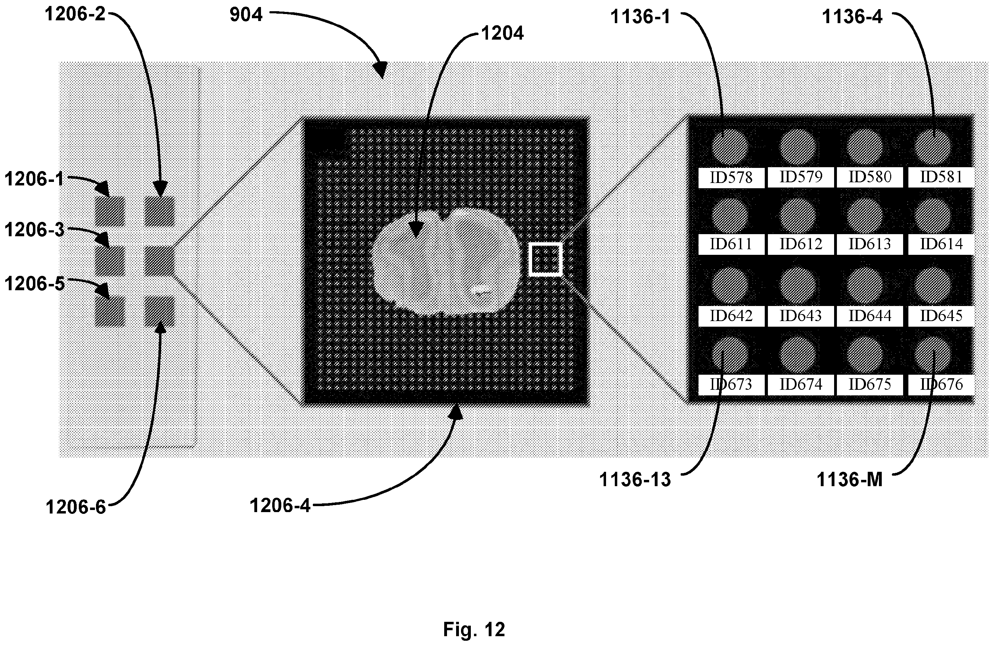

In some embodiments, a tissue section on a substrate is a single uniform section. In some embodiments, multiple tissue sections are on a substrate. In some such embodiments, a single capture area such as capture area **1206** on a substrate, as illustrated in **FIG. 12**, can contain multiple tissue sections **1204,** where each tissue section is obtained from either the same biological sample and/or subject or from different biological samples and/or subjects. In some embodiments, a tissue section is a single tissue section that comprises one or more regions where no cells are present (*e.g.,* holes, tears, or gaps in the tissue). Thus, in some embodiments, such as the above, an image of a tissue section on a substrate can contain regions where tissue is present and regions where tissue is not present.

Additional examples of tissue samples are shown in Table 1 and catalogued, for example, in 10X, 2019, "Visium Spatial Gene Expression Solution," and in U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue".

**Table 1: Examples of tissue samples**

| **Organism** | **Tissue** | **Healthy/Diseased** |
|---|---|---|
| Human | Brain | Cerebrum Glioblastoma Multiforme |
| Human | Breast | Healthy |
| Human | Breast | Invasive Ductal Carcinoma |
| Human | Breast | Invasive Lobular Carcinoma |
| Human | Heart | Healthy |
| Human | Kidney | Healthy |
| Human | Kidney | Nephritis |
| Human | Large Intestine | Colorectal Cancer |
| Human | Lung | Papillary Carcinoma |
| Human | Lymph Node | Healthy |
| Human | Lymph Node | Inflamed |
| Human | Ovaries | Tumor |
| Human | Spleen | Inflamed |
| Mouse | Brain | Healthy |
| Mouse | Eyes | Healthy |
| Mouse | Heart | Healthy |
| Mouse | Kidney | Healthy |
| Mouse | Large Intestine | Healthy |
| Mouse | Liver | Healthy |
| Mouse | Lungs | Healthy |
| Mouse | Ovary | Healthy |
| Mouse | Quadriceps | Healthy |
| Mouse | Small Intestine | Healthy |
| Mouse | Spleen | Healthy |
| Mouse | Stomach | Healthy |
| Mouse | Testes | Healthy |
| Mouse | Thyroid | Healthy |
| Mouse | Tongue | Healthy |
| Rat | Brain | Healthy |
| Rat | Heart | Healthy |
| Rat | Kidney | Healthy |
| Mouse | Tongue | Healthy |
| Rat | Brain | Healthy |
| Rat | Heart | Healthy |
| Rat | Kidney | Healthy |

Multiple sections can also be obtained from a single biological sample. For example, multiple tissue sections can be obtained from a surgical biopsy sample by performing serial sectioning of the biopsy sample using a sectioning blade. Spatial information among the serial sections can be preserved in this manner, and the sections can be analysed successively to obtain three-dimensional information about the biological sample.

In some embodiments, a biological sample is prepared using one or more steps including, but not limited to, freezing, fixation, embedding, formalin fixation and paraffin embedding, hydrogel embedding, biological sample transfer, isometric expansion, cell disaggregation, cell suspension, cell adhesion, permeabilization, lysis, protease digestion, selective permeabilization, selective lysis, selective enrichment, enzyme treatment, library preparation, and/or sequencing pre-processing. Methods for biological sample preparation that are contemplated in the present disclosure are described in further detail in U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue".

In some embodiments, a biological sample is prepared by staining. To facilitate visualization, biological samples can be stained using a wide variety of stains and staining techniques. In some embodiments, for example, a sample can be stained using any number of biological stains, including but not limited to, acridine orange, Bismarck brown, carmine, Coomassie blue, cresyl violet, DAPI, eosin, ethidium bromide, acid fuchsine, hematoxylin, Hoechst stains, iodine, methyl green, methylene blue, neutral red, Nile blue, Nile red, osmium tetroxide, propidium iodide, rhodamine, safranin, or a combination thereof.

The sample can be stained using known staining techniques, including Can-Grunwald, Giemsa, hematoxylin and eosin (H&E), Jenner's, Leishman, Masson's trichrome, Papanicolaou, Romanowsky, silver, Sudan, Wright's, and/or Periodic Acid Schiff (PAS) staining techniques. PAS staining is typically performed after formalin or acetone fixation.

In some embodiments, the sample is stained using a detectable label (*e.g.,* radioisotopes, fluorophores, chemiluminescent compounds, bioluminescent compounds, and dyes). In some embodiments, a biological sample is stained using only one type of stain or one technique. In some embodiments, staining includes biological staining techniques such as H&E staining. In some embodiments, staining includes identifying analytes using fluorescently-labeled antibodies. In some embodiments, a biological sample is stained using two or more different types of stains, or two or more different staining techniques. For example, a biological sample can be prepared by staining and imaging using one technique (*e.g.,* H&E staining and bright-field imaging), followed by staining and imaging using another technique (*e.g.,* IHC/IF staining and fluorescence microscopy) for the same biological sample.

In some embodiments, biological samples can be destained. Methods of destaining or discoloring a biological sample are known in the art, and generally depend on the nature of the stain(s) applied to the sample. For example, H&E staining can be destained by washing the sample in HCl, or any other low pH acid (*e.g.*, selenic acid, sulfuric acid, hydroiodic acid, benzoic acid, carbonic acid, malic acid, phosphoric acid, oxalic acid, succinic acid, salicylic acid, tartaric acid, sulfurous acid, trichloroacetic acid, hydrobromic acid, hydrochloric acid, nitric acid, orthophosphoric acid, arsenic acid, selenous acid, chromic acid, citric acid, hydrofluoric acid, nitrous acid, isocyanic acid, formic acid, hydrogen selenide, molybdic acid, lactic acid, acetic acid, carbonic acid, hydrogen sulfide, or combinations thereof). In some embodiments, destaining can include 1, 2, 3, 4, 5, or more washes in a low pH acid (*e.g.*, HCl). In some embodiments, destaining can include adding HCl to a downstream solution (*e.g.*, permeabilization solution). In some embodiments, destaining can include dissolving an enzyme used in the disclosed methods (*e.g.*, pepsin) in a low pH acid (*e.g.*, HCl) solution. In some embodiments, after destaining hematoxylin with a low pH acid, other reagents can be added to the destaining solution to raise the pH for use in other applications. For example, SDS can be added to a low pH acid destaining solution in order to raise the pH as compared to the low pH acid destaining solution alone. As another example, in some embodiments, one or more immunofluorescence stains are applied to the sample via antibody coupling. Such stains can be removed using techniques such as cleavage of disulfide linkages via treatment with a reducing agent and detergent washing, chaotropic salt treatment, treatment with antigen retrieval solution, and treatment with an acidic glycine buffer. Methods for multiplexed staining and destaining are described, for example, in Bolognesi et al., 2017, J. Histochem. Cytochem. 65(8): 431-444, Lin et al., 2015, Nat Commun. 6:8390, Pirici et al., 2009, J. Histochem. Cytochem. 57:567-75, and Glass et al., 2009, J. Histochem. Cytochem. 57:899-905.

In some embodiments, the biological sample can be attached to a substrate (*e.g.,* a slide and/or a chip). Examples of substrates suitable for this purpose are described in detail elsewhere herein (*see,* for example, "(A) General Definitions: Substrates," below). Attachment of the biological sample can be irreversible or reversible, depending upon the nature of the sample and subsequent steps in the analytical method.

In certain embodiments, the sample can be attached to the substrate reversibly by applying a suitable polymer coating to the substrate and contacting the sample to the polymer coating. The sample can then be detached from the substrate using an organic solvent that at least partially dissolves the polymer coating. Hydrogels are examples of polymers that are suitable for this purpose. More generally, in some embodiments, the substrate can be coated or functionalized with one or more substances to facilitate attachment of the sample to the substrate. Suitable substances that can be used to coat or functionalize the substrate include, but are not limited to, lectins, poly-lysine, antibodies, and polysaccharides.

Biological samples contemplated for use in the present disclosure are further described in U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue".

### Capture probes

A "capture probe," also interchangeably referred to herein as a "probe," refers to any molecule capable of capturing (directly or indirectly) and/or labelling an analyte (*e.g.,* an analyte of interest) in a biological sample. In some embodiments, the capture probe is a nucleic acid or a polypeptide. In some embodiments, the capture probe is a conjugate (*e.g.,* an oligonucleotide-antibody conjugate). In some embodiments, the capture probe includes a barcode (*e.g.,* a spatial barcode and/or a unique molecular identifier (UMI)) and a capture domain.

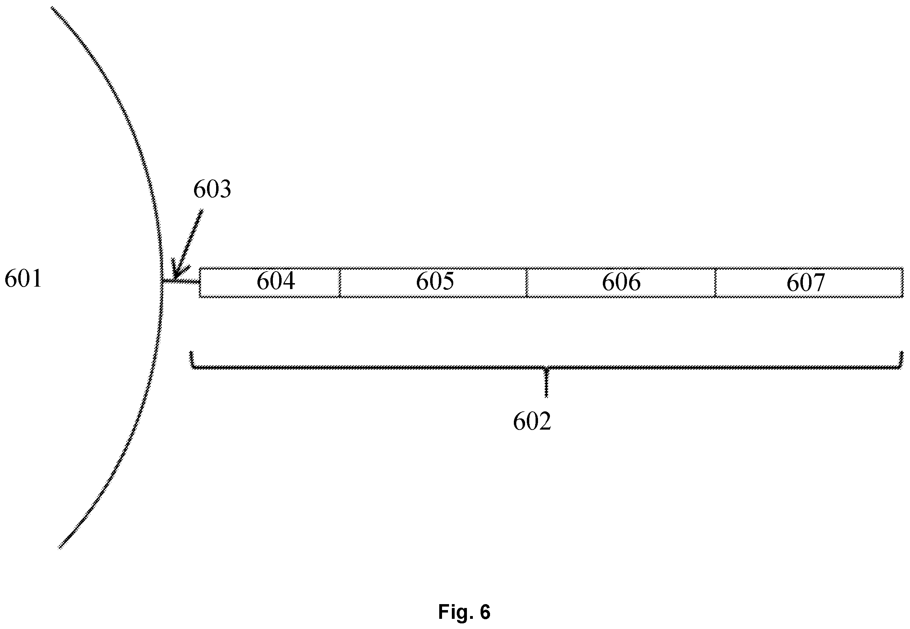

**FIG.** 6 is a schematic diagram showing an example of a capture probe, as described herein. As shown, the capture probe **602** is optionally coupled to a capture spot **601** (*e.g.,* a barcoded capture spot **1136)** by a cleavage domain **603,** such as a disulfide linker.

The capture probe **602** can include functional sequences that are useful for subsequent processing, such as functional sequence **604,** which can include a sequencer specific flow cell attachment sequence, *e.g.,* a P5 sequence, as well as functional sequence **606,** which can include sequencing primer sequences, *e.g.,* an R1 primer binding site, an R2 primer binding site. In some embodiments, sequence **604** is a P7 sequence and sequence **606** is a R2 primer binding site.

A spatial barcode **605** can be included within the capture probe for use in barcoding the target analyte. The functional sequences can be selected for compatibility with a variety of different sequencing systems, *e.g.,* 454 Sequencing, Ion Torrent Proton or PGM, Illumina sequencing instruments, PacBio, Oxford Nanopore, etc., and the requirements thereof. In some embodiments, functional sequences can be selected for compatibility with non-commercialized sequencing systems. Examples of such sequencing systems and techniques, for which suitable functional sequences can be used, include (but are not limited to) Ion Torrent Proton or PGM sequencing, Illumina sequencing, PacBio SMRT sequencing, and Oxford Nanopore sequencing. Further, in some embodiments, functional sequences can be selected for compatibility with other sequencing systems, including non-commercialized sequencing systems.

In some embodiments, the spatial barcode **605,** functional sequences **604** (*e.g.,* flow cell attachment sequence) and **606** (*e.g.,* sequencing primer sequences) can be common to all of the probes attached to a given capture spot. The spatial barcode can also include a capture domain **607** to facilitate capture of a target analyte.

Other aspects of capture probes contemplated for use in the present disclosure are known in the art. For instance, example suitable cleavage domains are described in further detail in PCT publication 202020176788A1, entitled "Profiling of biological analytes with spatially barcoded oligonucleotide arrays". Example suitable functional domains are described in further detail in U.S. Patent Publication No. US2021-0052272A1, entitled "Systems and Methods for Using the Spatial Distribution of Haplotypes to Determine a Biological Condition," as well as PCT publication 202020176788A1, entitled "Profiling of biological analytes with spatially barcoded oligonucleotide arrays". Example suitable spatial barcodes and unique molecular identifiers are described in further detail in U.S. Patent Publication No. US2021-0062272A1, entitled "Systems and Methods for Using the Spatial Distribution of Haplotypes to Determine a Biological Condition," and PCT publication 202020176788A1, entitled "Profiling of biological analytes with spatially barcoded oligonucleotide arrays".

Capture probes contemplated for use in the present disclosure are further described in U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue".

### Capture spots

As used interchangeably herein, the terms "capture spot," "feature," or "capture probe plurality" refer to an entity that acts as a support or repository for various molecular entities used in sample analysis. Examples of capture spots include, but are not limited to, a bead, a spot of any two- or three-dimensional geometry (*e.g.,* an inkjet spot, a masked spot, a square on a grid), a well, and a hydrogel pad. In some embodiments, a capture spot is an area on a substrate at which capture probes labelled with spatial barcodes are clustered. Specific non-limiting embodiments of capture spots and substrates are further described below in the present disclosure.

In some embodiments, capture spots are directly or indirectly attached or fixed to a substrate (*e.g.,* of a chip or a slide). In some embodiments, the capture spots are not directly or indirectly attached or fixed to a substrate, but instead, for example, are disposed within an enclosed or partially enclosed three dimensional space (*e.g.,* wells or divots). In some embodiments, some or all capture spots in an array include a capture probe.

In some embodiments, a capture spot includes different types of capture probes attached to the capture spot. For example, the capture spot can include a first type of capture probe with a capture domain designed to bind to one type of analyte, and a second type of capture probe with a capture domain designed to bind to a second type of analyte. In general, capture spots can include one or more (*e.g.,* two or more, three or more, four or more, five or more, six or more, eight or more, ten or more, 12 or more, 15 or more, 20 or more, 30 or more, 50 or more) different types of capture probes attached to a single capture spot.

In some embodiments, a capture spot on the array includes a bead. In some embodiments, two or more beads are dispersed onto a substrate to create an array, where each bead is a capture spot on the array. Beads can optionally be dispersed into wells on a substrate, *e.g.,* such that only a single bead is accommodated per well.

Further details and non-limiting embodiments relating to capture spots, including but not limited to beads, bead arrays, bead properties (*e.g.,* structure, materials, construction, cross-linking, degradation, reagents, and/or optical properties), and for covalently and non-covalently bonding beads to substrates are described in U.S. Patent Publication No. US2021-0062272A1, U.S. Patent Publication No. 20110059865A1, U.S. Provisional Application No. 62/839,346, U.S. Patent No. 9,012,022, and PCT publication 202020176788A1, entitled "Profiling of biological analytes with spatially barcoded oligonucleotide arrays"; U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue".

### Capture spot arrays

In some embodiments, capture spots are collectively positioned on a substrate. As used herein, the term "capture spot array" or "array" refers to a specific arrangement of a plurality of capture spots (also termed "features") that is either irregular or forms a regular pattern. Individual capture spots in the array differ from one another based on their relative spatial locations. In general, at least two of the plurality of capture spots in the array include a distinct capture probe (*e.g.,* any of the examples of capture probes described herein).

Arrays can be used to measure large numbers of analytes simultaneously. In some embodiments, oligonucleotides are used, at least in part, to create an array. For example, one or more copies of a single species of oligonucleotide (*e.g.,* capture probe) can correspond to or be directly or indirectly attached to a given capture spot in the array. In some embodiments, a given capture spot in the array includes two or more species of oligonucleotides (*e.g.,* capture probes). In some embodiments, the two or more species of oligonucleotides (*e.g.,* capture probes) attached directly or indirectly to a given capture spot on the array include a common (*e.g.,* identical) spatial barcode.

**FIG. 12** depicts an exemplary arrangement of barcoded capture spots within an array. From left to right, **FIG. 12** shows (L) a slide including six capture areas **1206** including spatially-barcoded capture spot arrays **904,** (C) An enlarged schematic of one of the six spatially-barcoded arrays, showing a grid of barcoded capture spots **1136** in relation to a biological sample **1204,** and (R) an enlarged schematic of one section of an array, showing the specific identification of multiple capture spots **1136** within the array (labelled as ID578, ID579, ID580, etc.).

In some embodiments, a substrate and/or an array (*e.g.,* two-dimensional array) comprises a plurality of capture spots. In some embodiments, a substrate and/or an array includes between 4000 and 10,000 capture spots, or any range within 4000 to 6000 capture spots. For example, a substrate and/or an array includes between 4,000 to 4,400 capture spots, 4,000 to 4,800 capture spots, 4,000 to 5,200 capture spots, 4,000 to 5,600 capture spots, 5,600 to 6,000 capture spots, 5,200 to 6,000 capture spots, 4,800 to 6,000 capture spots, or 4,400 to 6,000 capture spots. In some embodiments, the substrate and/or array includes between 4,100 and 5,900 capture spots, between 4,200 and 5,800 capture spots, between 4,300 and 5,700 capture spots, between 4,400 and 5,600 capture spots, between 4,500 and 5,500 capture spots, between 4,600 and 5,400 capture spots, between 4,700 and 5,300 capture spots, between 4,800 and 5,200 capture spots, between 4,900 and 5,100 capture spots, or any range within the disclosed sub-ranges. For example, the substrate and/or array can include about 4,000 capture spots, about 4,200 capture spots, about 4,400 capture spots, about 4,800 capture spots, about 5,000 capture spots, about 5,200 capture spots, about 5,400 capture spots, about 5,600 capture spots, or about 6,000 capture spots. In some embodiments, the substrate and/or array comprises at least 4,000 capture spots. In some embodiments, the substrate and/or array includes approximately 5,000 capture spots.

In some embodiments, the plurality of capture spots comprises at least 50, at least 100, at least 200, at least 500, at least 1000, at least 2000, at least 5000, at least 10,000, at least 100,000, at least 500,000, or at least 1 million capture spots. In some embodiments, the plurality of capture spots comprises no more than 5 million, no more than 1 million, no more than 100,000, no more than 10,000, no more than 1000, or no more than 500 capture spots. In some embodiments, the plurality of capture spots comprises from 100 to 10,000, from 300 to 5000, from 2000 to 100,000, or from 50,000 to 500,000 capture spots. In some embodiments, the plurality of capture spots includes another range starting no lower than 50 capture spots and ending no higher than 5 million capture spots.

Arrays suitable for use in the present disclosure are further described in PCT publication 202020176788A1, entitled "Profiling of biological analytes with spatially barcoded oligonucleotide arrays"; U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue".

### Contact

As used herein, the terms "contact," "contacted," and/ or "contacting" of a biological sample with a substrate comprising capture spots refers to any contact (e.g., direct or indirect) such that capture probes can interact (e.g., capture) with analytes from the biological sample. For example, the substrate may be near or adjacent to the biological sample without direct physical contact, yet capable of capturing analytes from the biological sample. In some embodiments the biological sample is in direct physical contact with the substrate. In some embodiments, the biological sample is in indirect physical contact with the substrate. For example, a liquid layer may be between the biological sample and the substrate. In some embodiments, the analytes diffuse through the liquid layer. In some embodiments the capture probes diffuse through the liquid layer. In some embodiments, reagents may be delivered via the liquid layer between the biological sample and the substrate. In some embodiments, indirect physical contact may be the presence of a second substrate (*e.g.,* a hydrogel, a film, a porous membrane) between the biological sample and the first substrate comprising capture spots with capture probes. In some embodiments, reagents are delivered by the second substrate to the biological sample.

In some embodiments, a cell immobilization agent can be used to contact a biological sample with a substrate (*e.g.,* by immobilizing non-aggregated or disaggregated sample on a spatially-barcoded array prior to analyte capture). A "cell immobilization agent" as used herein can refer to an agent (*e.g.,* an antibody), attached to a substrate, which can bind to a cell surface marker. Non-limiting examples of a cell surface marker include CD45, CD3, CD4, CD8, CD56, CD19, CD20, CD11c, CD14, CD33, CD66b, CD34, CD41, CD61, CD235a, CD146, and epithelial cellular adhesion molecule (EpCAM). A cell immobilization agent can include any probe or component that can bind to (*e.g.,* immobilize) a cell or tissue when on a substrate. A cell immobilization agent attached to the surface of a substrate can be used to bind a cell that has a cell surface maker. The cell surface marker can be a ubiquitous cell surface marker, wherein the purpose of the cell immobilization agent is to capture a high percentage of cells within the sample. The cell surface marker can be a specific, or more rarely expressed, cell surface marker, wherein the purpose of the cell immobilization agent is to capture a specific cell population expressing the target cell surface marker. Accordingly, a cell immobilization agent can be used to selectively capture a cell expressing the target cell surface marker from a population of cells that do not have the same cell surface marker.

Generally, analytes can be captured when contacting a biological sample with, *e.g.,* a substrate comprising capture probes (e.g., substrate with capture probes embedded, spotted, printed on the substrate or a substrate with capture spots (*e.g.,* beads, wells) comprising capture probes). Capture can be performed using passive capture methods and/or active capture methods.

In some embodiments, capture of analytes is facilitated by treating the biological sample with permeabilization reagents. If a biological sample is not permeabilized sufficiently, the amount of analyte captured on the substrate can be too low to enable adequate analysis. Conversely, if the biological sample is too permeable, the analyte can diffuse away from its origin in the biological sample, such that the relative spatial relationship of the analytes within the biological sample is lost. Hence, a balance between permeabilizing the biological sample enough to obtain good signal intensity while still maintaining the spatial resolution of the analyte distribution in the biological sample is desired. Methods of preparing biological samples to facilitate capture are known in the art and can be modified depending on the biological sample and how the biological sample is prepared (*e.g.,* fresh frozen, FFPE, *etc*.). Examples of analyte capture suitable for use in the present disclosure are further described in U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue".

### Spatial fiducials

As used interchangeably herein, the terms "fiducial," "spatial fiducial," "fiducial marker," and "fiducial spot" generally refers to a point of reference or measurement scale. In some embodiments, imaging is performed using one or more fiducial markers, i.e., objects placed in the field of view of an imaging system that appear in the image produced. Fiducial markers can include, but are not limited to, detectable labels such as fluorescent, radioactive, chemiluminescent, calorimetric, and colorimetric labels. The use of fiducial markers to stabilize and orient biological samples is described, for example, in Carter et al., Applied Optics 46:421-427, 2007).

In some embodiments, a fiducial marker can be present on a substrate to provide orientation of the biological sample. In some embodiments, a microsphere can be coupled to a substrate to aid in orientation of the biological sample. In some examples, a microsphere coupled to a substrate can produce an optical signal (*e.g.,* fluorescence). In another example, a microsphere can be attached to a portion (*e.g.,* corner) of an array in a specific pattern or design (*e.g.,* hexagonal design) to aid in orientation of a biological sample on an array of capture spots on the substrate. In some embodiments, a fiducial marker can be an immobilized molecule with which a detectable signal molecule can interact to generate a signal. For example, a marker nucleic acid can be linked or coupled to a chemical moiety capable of fluorescing when subjected to light of a specific wavelength (or range of wavelengths). Such a marker nucleic acid molecule can be contacted with an array before, contemporaneously with, or after the tissue sample is stained to visualize or image the tissue section. In some embodiments, it can be advantageous to use a marker that can be detected using the same conditions (*e.g.,* imaging conditions) used to detect an analyte of interest.

In some embodiments, fiducial markers are included to facilitate the orientation of a tissue sample or an image thereof in relation to an immobilized capture probes on a substrate. Any number of methods for marking an array can be used such that a marker is detectable only when a tissue section is imaged. For instance, a molecule, *e.g.,* a fluorescent molecule that generates a signal, can be immobilized directly or indirectly on the surface of a substrate. Markers can be provided on a substrate in a pattern (*e.g.,* an edge, one or more rows, one or more lines, *etc*.).

In some embodiments, a fiducial marker can be stamped, attached, or synthesized on the substrate and contacted with a biological sample. Typically, an image of the sample and the fiducial marker is taken, and the position of the fiducial marker on the substrate can be confirmed by viewing the image.

In some examples, fiducial markers can surround the array. In some embodiments the fiducial markers allow for detection of, *e.g.,* mirroring. In some embodiments, the fiducial markers may completely surround the array. In some embodiments, the fiducial markers may not completely surround the array. In some embodiments, the fiducial markers identify the corners of the array. In some embodiments, one or more fiducial markers identify the center of the array.

Example spatial fiducials suitable for use in the present disclosure are further described in U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue".

### Imaging

As used herein, the term "imaging" refers to any method of obtaining an image, *e.g.,* a microscope image. For example, images include bright-field images, which are transmission microscopy images where broad-spectrum, white light is placed on one side of the sample mounted on a substrate and the camera objective is placed on the other side and the sample itself filters the light in order to generate colors or grayscale intensity images.

In some embodiments, in addition to or instead of bright-field imaging, emission imaging, such as fluorescence imaging is used. In emission imaging approaches, the sample on the substrate is exposed to light of a specific narrow band (first wavelength band) of light and the light that is re-emitted from the sample at a slightly different wavelength (second wavelength band) is measured. This absorption and re-emission is due to the presence of a fluorophore that is sensitive to the excitation used and can be either a natural property of the sample or an agent the sample has been exposed to in preparation for the imaging. As an example, in an immunofluorescence experiment, an antibody that binds to a certain protein or class of proteins, and that is labeled with a certain fluorophore, is added to the sample. The locations on the sample that include the protein or class of proteins will then emit the second wavelength band. In some implementations, multiple antibodies with multiple fluorophores can be used to label multiple proteins in the sample. Each such fluorophore undergoes excitation with a different wavelength of light and further emits a different unique wavelength of light. In order to spatially resolve each of the different emitted wavelengths of light, the sample is subjected to the different wavelengths of light that will excite the multiple fluorophores on a serial basis and images for each of these light exposures is saved as an image thus generating a plurality of images. For instance, the image is subjected to a first wavelength that excites a first fluorophore to emit at a second wavelength and a first image of the sample is taken while the sample is being exposed to the first wavelength. The exposure of the sample to the first wavelength is discontinued and the sample is exposed to a third wavelength (different from the first wavelength) that excites a second fluorophore at a fourth wavelength (different from the second wavelength) and a second image of the sample is taken while the sample is being exposed to the third wavelength. Such a process is repeated for each different fluorophore in the multiple fluorophores (*e.g.,* two or more fluorophores, three or more fluorophores, four or more fluorophores, five or more fluorophores). In this way, a series of images of the tissue, each depicting the spatial arrangement of some different parameter such as a particular protein or protein class, is obtained. In some embodiments, more than one fluorophore is imaged at the same time. In such an approach a combination of excitation wavelengths is used, each for one of the more than one fluorophores, and a single image is collected.

In some embodiments, each of the images collected through emission imaging is a grayscale image. To differentiate such grayscaled images, in some embodiments each of the images are assigned a color (shades of red, shades of blue, *etc*.). In some implementations, each image is then combined into one composite color image for viewing. This allows for the spatial analysis of analytes (*e.g.,* spatial proteomics, spatial transcriptomics, *etc*.) in the sample. In some embodiments, spatial analysis of one type of analyte is performed independently of any other analysis. In some embodiments, spatial analysis is performed together for a plurality of types of analytes.

### Nucleic acid and Nucleotide

As used herein, the terms "nucleic acid" and "nucleotide" are intended to be consistent with their use in the art and to include naturally-occurring species or functional analogs thereof. Particularly useful functional analogs of nucleic acids are capable of hybridizing to a nucleic acid in a sequence-specific fashion (*e.g.,* capable of hybridizing to two nucleic acids such that ligation can occur between the two hybridized nucleic acids) or are capable of being used as a template for replication of a particular nucleotide sequence. Naturally-occurring nucleic acids generally have a backbone containing phosphodiester bonds. An analog structure can have an alternate backbone linkage including any of a variety of those known in the art. Naturally-occurring nucleic acids generally have a deoxyribose sugar (*e.g.,* found in deoxyribonucleic acid (DNA)) or a ribose sugar (*e.g.,* found in ribonucleic acid (RNA)).

A nucleic acid can contain nucleotides having any of a variety of analogs of these sugar moieties that are known in the art. A nucleic acid can include native or non-native nucleotides. In this regard, a native deoxyribonucleic acid can have one or more bases selected from the group consisting of adenine (A), thymine (T), cytosine (C), or guanine (G), and a ribonucleic acid can have one or more bases selected from the group consisting of uracil (U), adenine (A), cytosine (C), or guanine (G). Useful non-native bases that can be included in a nucleic acid or nucleotide are known in the art.

### Region of interest

As used herein, the term "region of interest" generally refers to a region or area within a biological sample that is selected for specific analysis (*e.g.,* a region in a biological sample that has morphological features of interest). A biological sample can have regions that show morphological feature(s) that may indicate the presence of disease or the development of a disease phenotype. For example, morphological features at a specific site within a tumor biopsy sample can indicate the aggressiveness, therapeutic resistance, metastatic potential, migration, stage, diagnosis, and/or prognosis of cancer in a subject. A change in the morphological features at a specific site within a tumor biopsy sample often correlate with a change in the level or expression of an analyte in a cell within the specific site, which can, in turn, be used to provide information regarding the aggressiveness, therapeutic resistance, metastatic potential, migration, stage, diagnosis, and/or prognosis of cancer in a subject. A region of interest in a biological sample can be used to analyze a specific area of interest within a biological sample, and thereby, focus experimentation and data gathering to a specific region of a biological sample (rather than an entire biological sample). This results in increased time efficiency of the analysis of a biological sample.

A region of interest can be identified in a biological sample using a variety of different techniques, *e.g.,* expansion microscopy, bright field microscopy, dark field microscopy, phase contrast microscopy, electron microscopy, fluorescence microscopy, reflection microscopy, interference microscopy, and confocal microscopy, and combinations thereof. For example, the staining and imaging of a biological sample can be performed to identify a region of interest. In some examples, the region of interest can correspond to a specific structure of cytoarchitecture. In some embodiments, a biological sample can be stained prior to visualization to provide contrast between the different regions of the biological sample. The type of stain can be chosen depending on the type of biological sample and the region of the cells to be stained. In some embodiments, more than one stain can be used to visualize different aspects of the biological sample, *e.g.,* different regions of the sample, specific cell structures (*e.g.,* organelles), or different cell types. In other embodiments, the biological sample can be visualized or imaged without staining the biological sample.

In some examples, a region of interest can be removed from a biological sample and then the region of interest can be contacted to the substrate and/or array (*e.g.,* as described herein). A region of interest can be removed from a biological sample using microsurgery, laser capture microdissection, chunking, a microtome, dicing, trypsinization, labelling, and/or fluorescence-assisted cell sorting.

### Subject

As used herein, the term "subject" refers to an animal, such as a mammal (*e.g.,* human or a non-human simian), avian (*e.g.,* bird), or other organism, such as a plant. Examples of subjects include, but are not limited to, a mammal such as a rodent, mouse, rat, rabbit, guinea pig, ungulate, horse, sheep, pig, goat, cow, cat, dog, primate (*e.g.,* human or non-human primate); a plant such as *Arabidopsis thaliana,* corn, sorghum, oat, wheat, rice, canola, or soybean; an algae such as *Chlamydomonas reinhardtii;* a nematode such as *Caenorhabditis elegans;* an insect such as *Drosophila melanogaster,* mosquito, fruit fly, honey bee or spider; a fish such as zebrafish; a reptile; an amphibian such as a frog or *Xenopus laevis; a Dictyostelium discoideum;* a fungi such as *Pneumocystis carinii, Takifugu rubripes,* yeast, *Saccharamoyces cerevisiae* or *Schizosaccharomyces pombe;* or a *Plasmodium falciparum.*

### Substrates

As used herein, a "substrate" refers to a support that is insoluble in aqueous liquid and that allows for positioning of biological samples, analytes, capture spots, and/or capture probes on the substrate. For instance, a substrate can be any surface onto which a sample and/or capture probes can be affixed (*e.g.,* a chip, solid array, a bead, a slide, a coverslip, *etc*.). For the spatial analytical methods described in this section, a substrate is used to provide support to a biological sample, particularly, for example, a thin tissue section. In addition, in some embodiments, a substrate (*e.g.,* the same substrate or a different substrate) functions as a support for direct or indirect attachment of capture probes to capture spots of the array.

A wide variety of different substrates can be used for the foregoing purposes. In general, a substrate can be any suitable support material. Exemplary substrates include, but are not limited to, glass, modified and/or functionalized glass, hydrogels, films, membranes, plastics (including *e.g.,* acrylics, polystyrene, copolymers of styrene and other materials, polypropylene, polyethylene, polybutylene, polyurethanes, Teflon^{™}, cyclic olefins, polyimides, *etc*.), nylon, ceramics, resins, Zeonor, silica or silica-based materials including silicon and modified silicon, carbon, metals, inorganic glasses, optical fiber bundles, and polymers, such as polystyrene, cyclic olefin copolymers (COCs), cyclic olefin polymers (COPs), polypropylene, polyethylene and polycarbonate.

The substrate can also correspond to a flow cell. Flow cells can be formed of any of the foregoing materials, and can include channels that permit reagents, solvents, capture spots, and molecules to pass through the flow cell.

The substrate can generally have any suitable form or format. For example, the substrate can be flat, curved, *e.g.,* convexly or concavely curved towards the area where the interaction between a biological sample, *e.g.,* tissue sample, and the substrate takes place. In some embodiments, the substrate is a flat, *e.g.,* planar, chip or slide. The substrate can contain one or more patterned surfaces within the substrate (e*.g.,* channels, wells, projections, ridges, divots, *etc*.). A substrate can be of any desired shape. For example, a substrate can be typically a thin, flat shape (*e.g.,* a square or a rectangle). In some embodiments, a substrate structure has rounded corners (*e.g.,* for increased safety or robustness). In some embodiments, a substrate structure has one or more cut-off corners (*e.g.,* for use with a slide clamp or cross-table). In some embodiments, where a substrate structure is flat, the substrate structure can be any appropriate type of support having a flat surface (*e.g.,* a chip or a slide such as a microscope slide).

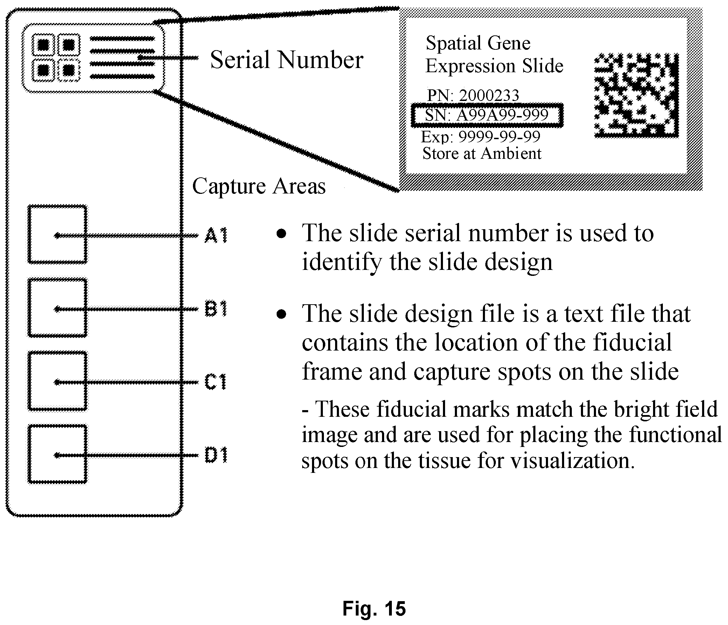

In some embodiments, a substrate includes one or more markings on a surface of the substrate, *e.g.,* to provide guidance for correlating spatial information with the characterization of the analyte of interest. For example, a substrate can be marked with a grid of lines (*e.g.,* to allow the size of objects seen under magnification to be easily estimated and/or to provide reference areas for counting objects). In some embodiments, fiducials (*e.g.,* fiducial markers, fiducial spots, or fiducial patterns) can be included on the substrate. Fiducials can be made using techniques including, but not limited to, printing, sand-blasting, and depositing on the surface. In some embodiments, the substrate (*e.g.,* or a bead or a capture spot on an array) includes a plurality of oligonucleotide molecules (*e.g.,* capture probes). In some embodiments, the substrate includes tens to hundreds of thousands or millions of individual oligonucleotide molecules (*e.g.,* at least about 10,000, 50,000, 100,000, 500,000, 1,000,000, 10,000,000, 100,000,000, 1,000,000,000 or 10,000,000,000 oligonucleotide molecules). In some embodiments, as illustrated in **FIG. 15**, a substrate can include a substrate identifier, such as a serial number.

Further examples of substrates, including for example fiducial markers on such substrates, are disclosed in PCT publication 202020176788A1, entitled "Profiling of biological analytes with spatially barcoded oligonucleotide arrays"; U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue".

***Spatial Analyte Data.*** As used herein, "spatial analyte data" refers to any data measured, either directly, from the capture of analytes on capture probes, or indirectly, through intermediate agents disclosed herein that bind to analytes in a sample, *e.g.,* connected probes disclosed herein, analyte capture agents or portions thereof (such as, *e.g.,* analyte binding moieties and their associated analyte binding moiety barcodes). Spatial analyte data thus may, in some aspects, include two different labels from two different classes of barcodes. One class of barcode identifies the analyte, while the other class of barcodes identifies the specific capture probe in which an analyte was detected.

### (B) Methods for Spatial Analysis of Analytes

Array-based spatial analysis methods involve the transfer of one or more analytes from a biological sample to an array of capture spots on a substrate, each of which is associated with a unique spatial location on the array. Subsequent analysis of the transferred analytes includes determining the identity of the analytes and the spatial location of each analyte within the sample. The spatial location of each analyte within the sample is determined based on the capture spot to which each analyte is bound in the array, and the capture spot's relative spatial location within the array.

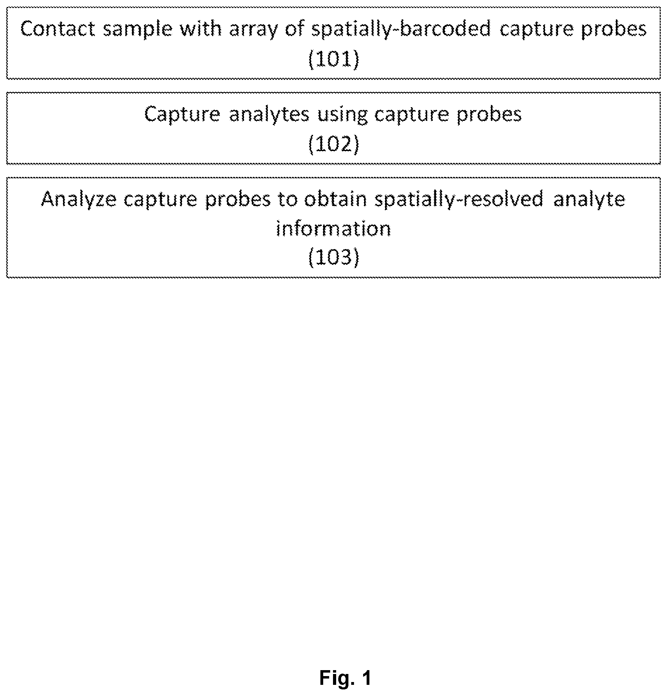

There are at least several general methods to associate a spatial barcode with one or more neighboring cells, such that the spatial barcode identifies the one or more cells, and/or contents of the one or more cells, as associated with a particular spatial location. One general method is to promote analytes out of a cell and towards the spatially-barcoded array. **FIG. 1** depicts an exemplary embodiment of this general method. In **FIG. 1****,** the spatially-barcoded array populated with capture probes (as described further herein) is contacted with a sample **101,** and the sample is permeabilized **102,** allowing the target analyte to migrate away from the sample and toward the array **102.** The target analyte interacts with a capture probe on the spatially-barcoded array. Once the target analyte hybridizes/is bound to the capture probe, the sample is optionally removed from the array and the capture probes are analyzed in order to obtain spatially-resolved analyte information **103.**

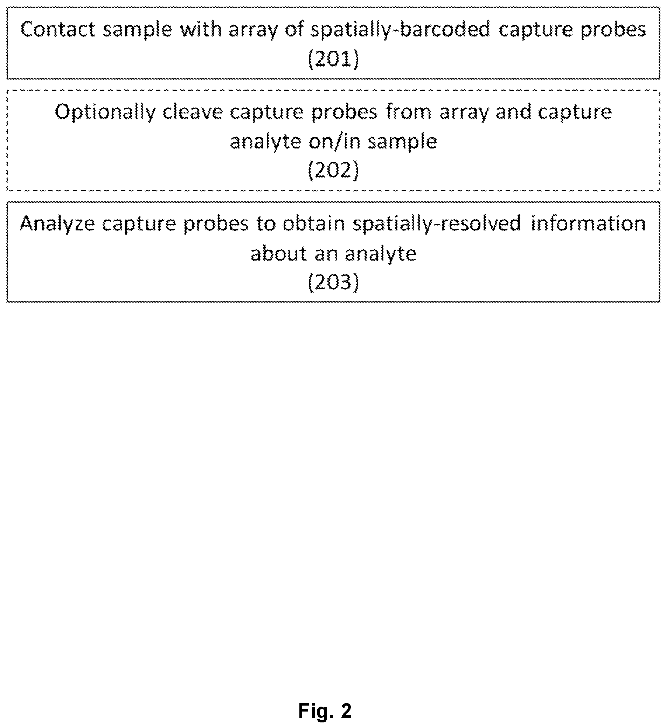

Another general method is to cleave the spatially-barcoded capture probes from an array and promote the spatially-barcoded capture probes towards and/or into or onto the sample. **FIG. 2** depicts an exemplary embodiment of this general method, the spatially-barcoded array populated with capture probes (as described further herein) can be contacted with a sample **201.** The spatially-barcoded capture probes are cleaved and then interact with cells within the provided sample **202.** The interaction can be a covalent or non-covalent cell-surface interaction. The interaction can be an intracellular interaction facilitated by a delivery system or a cell penetration peptide. Once the spatially-barcoded capture probe is associated with a particular cell, the sample can be optionally removed for analysis. The sample can be optionally dissociated before analysis. Once the tagged cell is associated with the spatially-barcoded capture probe, the capture probes can be analyzed to obtain spatially-resolved information about the tagged cell **203.**

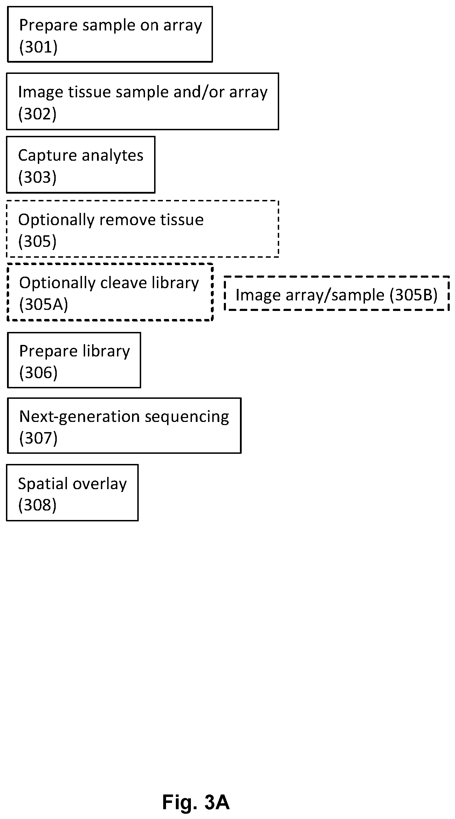

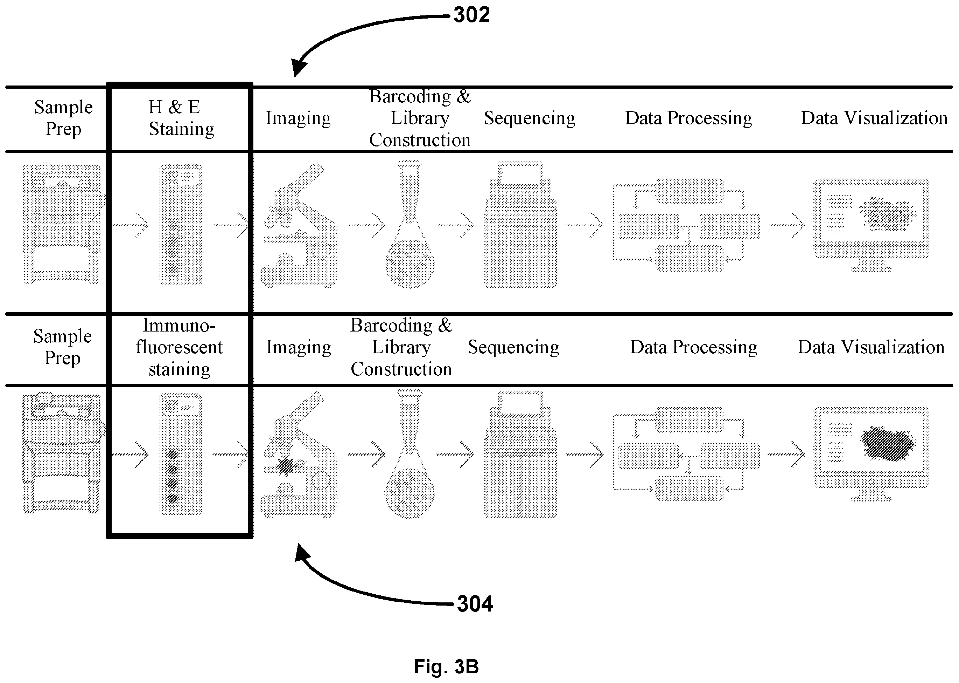

**FIGS. 3A** and **3B** show exemplary workflows that include preparing a sample on a spatially-barcoded array **301.** Sample preparation may include placing the sample on a substrate (*e.g.,* chip, slide, *etc*.), fixing the sample, and/or staining the sample for imaging. The sample (stained or not stained) is then imaged on the array **302** using bright-field (to image the sample, *e.g.,* using a hematoxylin and eosin stain) or fluorescence (to image capture spots) as illustrated in the upper panel **302** of **FIG. 3B****)** and/or emission imaging modalities (as illustrated in the lower panel **304** of **FIG. 3B****).**

In some embodiments where the sample is analyzed with transcriptomics, along with the bright-field and/or emission imaging (*e.g.,* fluorescence imaging), target analytes are released from the sample and capture probes forming a spatially-barcoded array hybridize or bind the released target analytes **303**. The sample can be optionally removed from the array **305** and the capture probes can be optionally cleaved from the array **305A.** The sample and array are then optionally imaged a second time in both modalities **305B** while the analytes are reverse transcribed into cDNA, and an amplicon library is prepared **306** and sequenced **307.** The images are then spatially-overlaid in order to correlate spatially-identified sample information **308.** When the sample and array are not imaged a second time, **305B,** a spot coordinate file is supplied instead. The spot coordinate file replaces the second imaging step **305B.** Further, amplicon library preparation **306** can be performed with a unique PCR adapter and sequenced **307.**

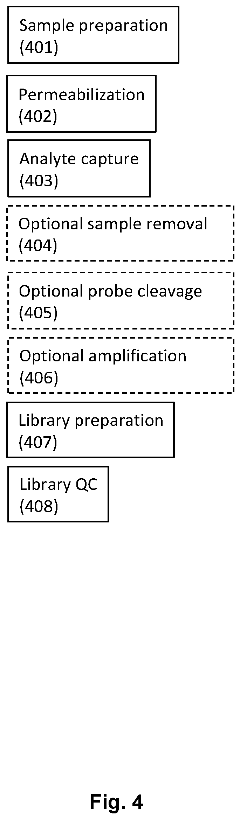

**FIG. 4** shows another exemplary workflow that utilizes a spatially-barcoded array on a substrate (*e.g*., chip), where spatially-barcoded capture probes are clustered at areas called capture spots. The spatially-labelled capture probes can include a cleavage domain, one or more functional sequences, a spatial barcode, a unique molecular identifier, and a capture domain. The spatially-labelled capture probes can also include a 5' end modification for reversible attachment to the substrate. The spatially-barcoded array is contacted with a sample **401,** and the sample is permeabilized through application of permeabilization reagents **402.** Permeabilization reagents may be administered by placing the array/sample assembly within a bulk solution. Alternatively, permeabilization reagents may be administered to the sample via a diffusion-resistant medium and/or a physical barrier such as a lid, where the sample is sandwiched between the diffusion-resistant medium and/or barrier and the array-containing substrate. The analytes are migrated toward the spatially-barcoded capture array using any number of techniques disclosed herein. For example, analyte migration can occur using a diffusion-resistant medium lid and passive migration. As another example, analyte migration can be active migration, using an electrophoretic transfer system, for example. Once the analytes are in close proximity to the spatially-barcoded capture probes, the capture probes can hybridize or otherwise bind a target analyte **403.** The sample can be optionally removed from the array **404.**

The capture probes can be optionally cleaved from the array **405,** and the captured analytes can be spatially-barcoded by performing a reverse transcriptase first strand cDNA reaction. A first strand cDNA reaction can be optionally performed using template switching oligonucleotides. For example, a template switching oligonucleotide can hybridize to a poly(C) tail added to a 3' end of the cDNA by a reverse transcriptase enzyme. Template switching is described, for example, in in U.S. Patent No. 11,501,440; U.S. Patent Publication No. US20210150707A1, entitled "SYSTEMS AND METHODS FOR TISSUE CLASSIFICATION"; U.S. Patent No. 11,514,575; and U.S. Patent Publication No. US2021-0155982A1, entitled "Spatial Analysis of Analytes".

The original mRNA template and template switching oligonucleotide can then be denatured from the cDNA and the spatially-barcoded capture probe can then hybridize with the cDNA and a complement of the cDNA can be generated. The first strand cDNA can then be purified and collected for downstream amplification steps. The first strand cDNA can be optionally amplified using PCR **406,** where the forward and reverse primers flank the spatial barcode and target analyte regions of interest, generating a library associated with a particular spatial barcode **407.** In some embodiments, the library preparation can be quantified and/or subjected to quality control to verify the success of the library preparation steps 408. In some embodiments, the cDNA comprises a sequencing by synthesis (SBS) primer sequence. The library amplicons are sequenced and analyzed to decode spatial information **407,** with an additional library quality control (QC) step **408.**

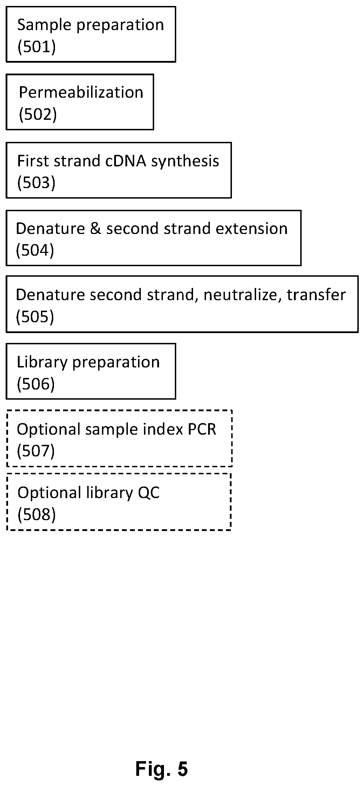

**FIG. 5** depicts an exemplary workflow where the sample is removed from the spatially-barcoded array and the spatially-barcoded capture probes are removed from the array for barcoded analyte amplification and library preparation. Another embodiment includes performing first strand synthesis using template switching oligonucleotides on the spatially-barcoded array without cleaving the capture probes. In this embodiment, sample preparation **501** and permeabilization **502** are performed as described elsewhere herein. Once the capture probes capture the target analyte(s), first strand cDNA created by template switching and reverse transcriptase **503** is then denatured and the second strand is then extended **504.** The second strand cDNA is then denatured from the first strand cDNA, neutralized, and transferred to a tube **505.** cDNA quantification and amplification can be performed using standard techniques discussed herein. The cDNA can then be subjected to library preparation **506** and indexing **507,** including fragmentation, end-repair, and a-tailing, and indexing PCR steps. The library can also be optionally tested for quality control (QC) **508.**

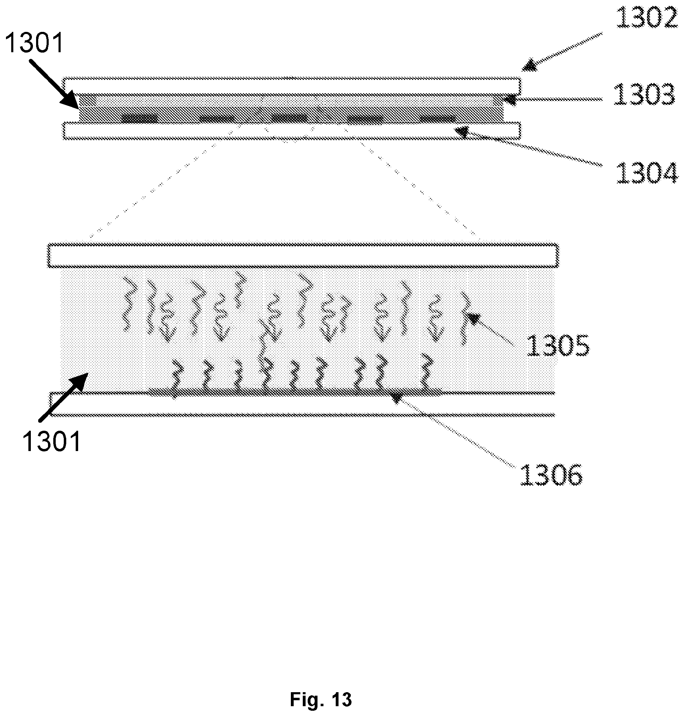

In some embodiments, a spatial dataset is obtained for a sample on a first substrate overlayed on a second substrate (*e.g.,* in a sandwich configuration). An example workflow for obtaining spatial analyte data from a biological sample on a first substrate overlayed on a second substate in a "sandwich configuration" is described with reference to **FIG. 13****.**

**FIG. 13** is an illustration of an exemplary sandwich configuration. A first substrate **1302** can be contacted with (*e.g.,* attached to) a sample **1303.** In **FIG. 13****,** a second substrate **1304** is populated with a plurality of capture probes **1306** at each capture spot **1136** in a plurality of capture spots, and the sample **1303,** including analytes **1305,** is contacted with the plurality of capture probes **1306** on the second substrate **1304.** In some embodiments, the second substrate comprises a spatially barcoded array of capture probes **1306.** In some embodiments, a fiducial frame surrounds the array. Accordingly, the sample **1303** is sandwiched between the first substrate **1302** and the second substrate **1304.** When a permeabilization solution **1301** is applied to the sample, analytes **1305** migrate toward the capture probes **1306.**

Further details and non-limiting embodiments relating to methods for spatial analysis of analytes in biological samples, including removal of sample from the array, release and amplification of analytes, analysis of captured analytes (*e.g.,* by sequencing and/or multiplexing), and spatial resolution of analyte information (*e.g.,* using lookup tables) are described in U.S. Patent Pubication No. US2021-0062272A1 entitled "Systems and Methods for Using the Spatial Distribution of Haplotypes to Determine a Biological Condition," in U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue"; and U.S. Patent Publication No. US2023-0167495A1, entitled "Systems and Methods for Identifying Regions of Aneuploidy in a Tissue".

### II. Exemplary System Embodiments

Now that a general summary of the methods and terminology has been presented, detailed descriptions of various implementations of the present disclosure will now be described in conjunction with the figures.

**FIGS. 11A** **and** **11B** collectively illustrate a block diagram illustrating an exemplary, non-limiting system **1100** for analyzing a tissue sample in accordance with some implementations. The system **1100** in some implementations includes one or more processing units CPU(s) **1102** (also referred to as processors), one or more network interfaces **1104,** a user interface **1106,** a memory **1112,** and one or more communication buses **1114** for interconnecting these components. The communication buses **1114** optionally include circuitry (sometimes called a chipset) that interconnects and controls communications between system components. The memory **1112** typically includes high-speed random access memory, such as DRAM, SRAM, DDR RAM, ROM, EEPROM, flash memory, CD-ROM, digital versatile disks (DVD) or other optical storage, magnetic cassettes, magnetic tape, magnetic disk storage or other magnetic storage devices, other random access solid state memory devices, or any other medium which can be used to store desired information; and optionally includes non-volatile memory, such as one or more magnetic disk storage devices, optical disk storage devices, flash memory devices, or other non-volatile solid state storage devices. The memory **1112** optionally includes one or more storage devices remotely located from the CPU(s) **1102.** The memory **1112,** or alternatively the non-volatile memory device(s) within the memory **1112,** comprises a non-transitory computer readable storage medium. It will be appreciated that this memory **1112** can be distributed across one or more computers. In some implementations, the memory **1112** or alternatively the non-transitory computer readable storage medium stores the following programs, modules and data structures, or a subset thereof:
- an optional operating system **1116,** which includes procedures for handling various basic system services and for performing hardware dependent tasks;
- an optional network communication module (or instructions) **1118** for connecting the device **1100** with other devices, or a communication network;
- an image data construct **1120** comprising a set of images **1122** (*e.g.,* **1122-1, ...1122-P)** of the tissue sample obtained while the tissue sample is overlaid on a substrate in a first orientation, where the substrate comprises a plurality of capture spots **1140,** and each respective image **1122** in the set of images is an emission image of the tissue sample upon excitation of the tissue sample at a corresponding excitation wavelength **1124** (*e.g.,* **1124-1, ...1124-P)** of a corresponding detectable marker, in a set of one or more detectable markers, associated with the respective image;
- a first spatial dataset **1134-1** comprising, for each respective capture spot **1136** in the plurality of capture spots (*e.g.,* **1136-1-1,...1136-1-Q),** for each respective detectable marker in the set of detectable markers, a measured intensity of the respective capture spot **1138** (*e.g.,* **1144-1-1-1, 1144-1-1-P, 1144-1-Q-1, 1144-1-Q-P)** in the corresponding image in the set of images, indexed by a spatial barcode **1140** in a plurality of spatial barcodes that represents the respective capture spot (*e.g.,* **1140-1-1, 1140-1-Q);**
- a second spatial dataset **1134-2** comprising, for each respective capture spot **1136** in the plurality of capture spots (*e.g.,* **1136-2-1,.. 1136-2-Q),** for each respective nucleic acid in a plurality of nucleic acids, a corresponding representation **1138** (e.g., **1138-2-1-1, 1138-2-1-R, 1138-2-Q-1, 1138-2-Q-T)** of a number of molecules of the respective nucleic acid originating from the tissue sample while the tissue sample is overlaid on a substrate in the first orientation and localized to the respective capture spot by a corresponding spatial barcode **1140,** in the plurality of spatial barcodes (*e.g.,* **1140-2-1, 1140-2-Q),** associated with the respective capture spot; and
- a registration module **1142** for determining a registration for all or a portion of the first spatial dataset **1134-1** and a corresponding all or a portion of the second spatial dataset **1134-2,** using the plurality of spatial barcodes **1140.**

In some implementations, the user interface **1106** includes an input device (*e.g.,* a keyboard, a mouse, a touchpad, a track pad, and/or a touch screen) **1110** for a user to interact with the system **1100** and a display **1108.**

In some implementations, one or more of the above identified elements are stored in one or more of the previously mentioned memory devices and correspond to a set of instructions for performing a function described above. The above identified modules or programs (*e.g.,* sets of instructions) need not be implemented as separate software programs, procedures or modules, and thus various subsets of these modules may be combined or otherwise re-arranged in various implementations. In some implementations, the memory **1112** optionally stores a subset of the modules and data structures identified above. Furthermore, in some embodiments, the memory stores additional modules and data structures not described above. In some embodiments, one or more of the above identified elements is stored in a computer system, other than that of system **1100,** that is addressable by system **1100** so that system **1100** may retrieve all or a portion of such data when needed.

Although **FIG. 11** shows an exemplary system **1100,** the figure is intended more as functional description of the various features that may be present in computer systems than as a structural schematic of the implementations described herein. In practice, and as recognized by those of ordinary skill in the art, items shown separately could be combined and some items could be separated.

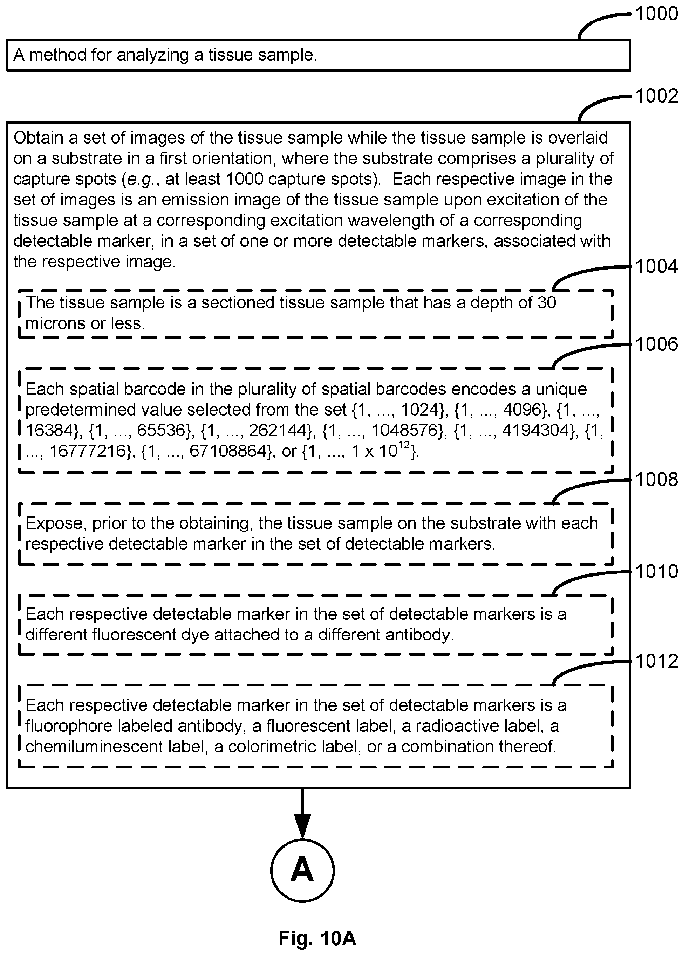

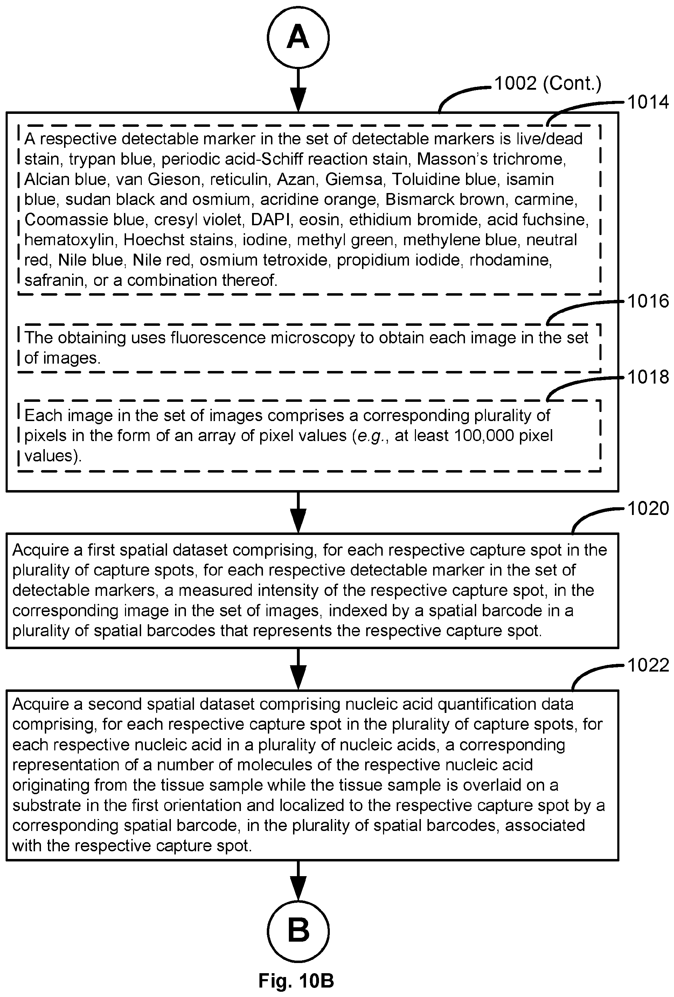

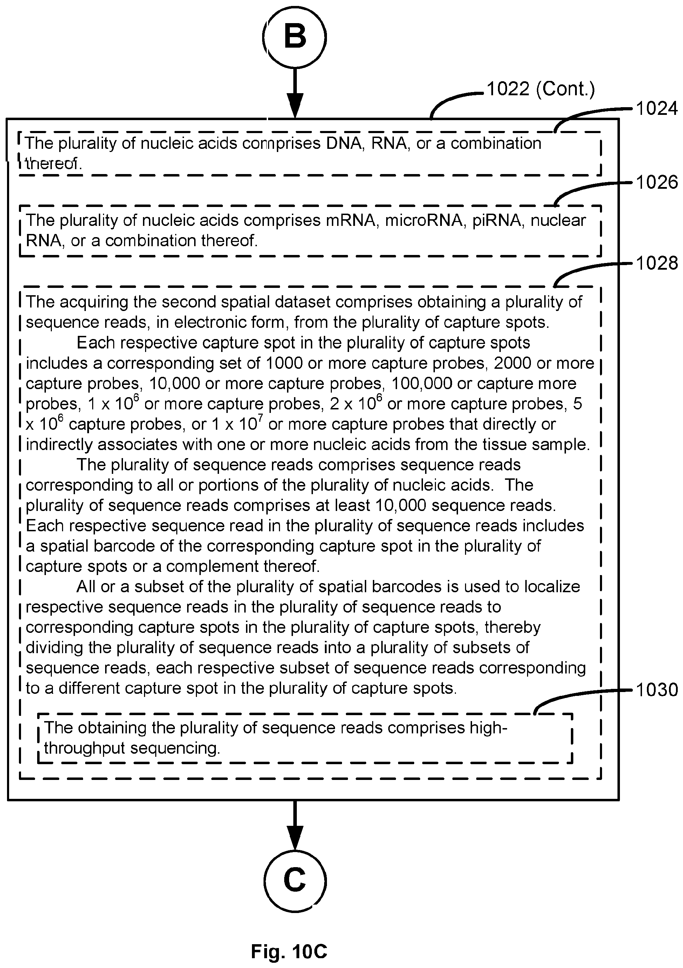

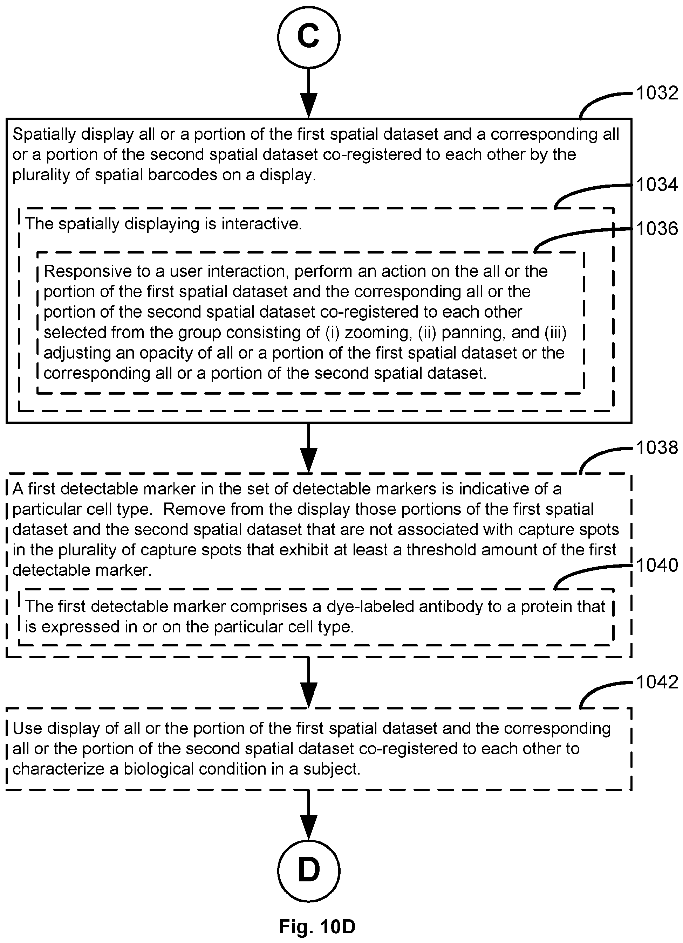

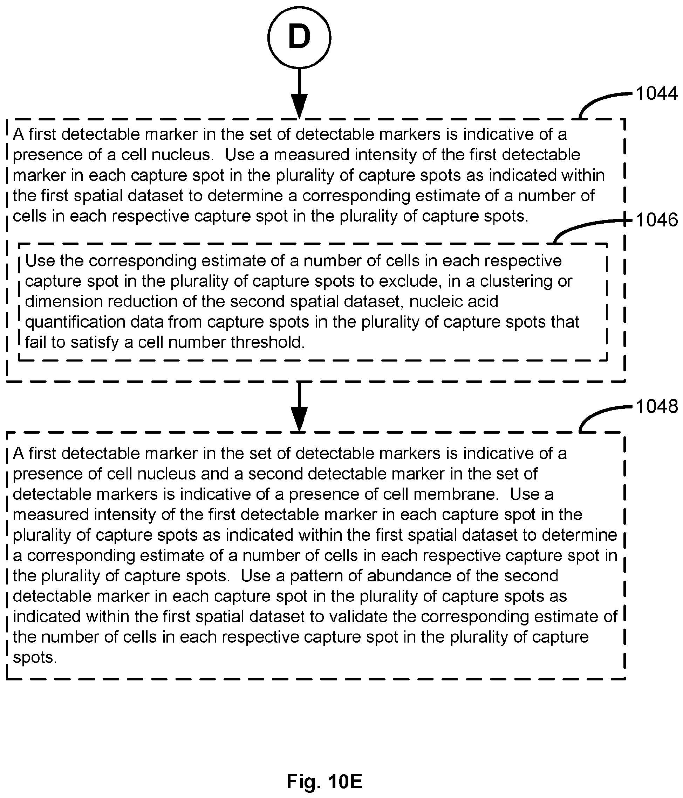

While a system in accordance with the present disclosure has been disclosed with reference to **FIG. 11****,** methods in accordance with the present disclosure are now detailed with reference to **FIGS. 10A****,** **10B****,** **10C****,** **10D****,** and **10E****.**

### III. Specific Embodiments

This disclosure also provides methods and systems for analyzing a tissue sample. Provided below are detailed descriptions and explanations of various embodiments of the present disclosure. These embodiments are non-limiting and do not preclude any alternatives, variations, changes, and substitutions that can occur to those skilled in the art from the scope of this disclosure.

One aspect of the present disclosure provides a method for analyzing a tissue sample, using a computer system comprising one or more processing cores and a memory. The method includes obtaining a set of images of the tissue sample while the tissue sample is overlaid on a substrate in a first orientation, where the substrate comprises a plurality of capture spots (*e.g.,* at least 1000 capture spots). Each respective image in the set of images is an emission image of the tissue sample upon excitation of the tissue sample at a corresponding excitation wavelength of a corresponding detectable marker, in a set of one or more detectable markers, associated with the respective image (*e.g.,* an immunofluorescence image).

A first spatial dataset is acquired, comprising, for each respective capture spot in the plurality of capture spots, for each respective detectable marker in the set of detectable markers, a measured intensity of the respective capture spot, in the corresponding image in the set of images. For example, in some embodiments, the measured intensity is a fluorescence intensity, determined by analyzing a plurality of pixels in the corresponding emission image. In some embodiments, the measured intensity is associated with the respective capture spot by overlaying the corresponding emission image with a frame of reference for the plurality of capture spots. In some such embodiments, the frame of reference localizes a corresponding position, in the respective emission image, of each capture spot in the plurality of capture spots. In some embodiments, the overlaying the corresponding emission image with a frame of reference for the plurality of capture spots is performed using fiducial registration. The measured intensity (*e.g.,* the fluorescence intensity) is indexed by a spatial barcode in a plurality of spatial barcodes that represents the respective capture spot.

A second spatial dataset is acquired, comprising nucleic acid quantification data including, for each respective capture spot in the plurality of capture spots, for each respective nucleic acid in a plurality of nucleic acids, a corresponding representation of a number of molecules of the respective nucleic acid originating from the tissue sample while the tissue sample is overlaid on a substrate in the first orientation and localized (*e.g.,* spatial nucleic acid quantification data) to the respective capture spot by a corresponding spatial barcode, in the plurality of spatial barcodes, associated with the respective capture spot. All or a portion of the first spatial dataset and a corresponding all or a portion of the second spatial dataset co-registered to each other by the plurality of spatial barcodes are spatially displayed on a display.

An overview of an example workflow for a method **1700** is described above with reference to **FIG. 17****.** Details of a method **1000** for analyzing a tissue sample will now be provided with reference to **FIGS. 10A****,** **10B****,** **10C****,** **10D****,** and **10E****,** in accordance with some embodiments of the present disclosure. In some embodiments, the method is performed at a computer system comprising one or more processing cores and a memory.

Referring to Block 1002, the method includes obtaining a set of images 1120 of the tissue sample while the tissue sample is overlaid on a substrate in a first orientation, where the substrate comprises a plurality of capture spots 1136 (*e.g.,* at least 1000 capture spots) and each respective image 1122 in the set of images is an emission image of the tissue sample upon excitation of the tissue sample at a corresponding excitation wavelength 1124 of a corresponding detectable marker, in a set of one or more detectable markers, associated with the respective image. In some embodiments the set of detectable markers includes a first detectable marker having a first excitation wavelength and a second detectable marker having a second excitation wavelength, where the first excitation wavelength is other than the second excitation wavelength. In some embodiments the first excitation wavelength is a first subset of wavelengths in the visible spectrum and the second excitation wavelength is a second subset of wavelengths in the visible spectrum that does not overlap with the first excitation wavelength.

### Samples and substrates.

In some embodiments, the tissue sample comprises a plurality of cells. In some embodiments, the tissue sample is a plurality of cells. In some embodiments, for instance, the tissue sample is a plurality of spatially arrayed cells. Examples of suitable tissue samples contemplated for use in the present disclosure are described in further detail herein (*see,* "(A) General Definitions: Biological Samples," above).

In some embodiments, the tissue sample comprises a cell type (*e.g.,* cancer cell, normal cell, healthy cell, diseased cell, *etc*.) and/or a disease condition (*e.g.,* a cancer stage, cancer type, tissue of origin, *etc*.)*.* In some embodiments, the tissue sample comprises a plurality of cell types (*e.g.,* cancer cell, normal cell, healthy cell, diseased cell, *etc*.) and/or a plurality of disease conditions (*e.g.,* a cancer stage, cancer type, tissue of origin, *etc*.).

In some embodiments, the tissue sample is a sample that is undergoing a particular treatment and/or biological process. For instance, in some embodiments, the tissue sample is a sample obtained from a subject that is undergoing a treatment and is being monitored for changes in analyte expression or physiology. Suitable treatments include, but are not limited to, chemical treatments, radiological treatment, and/or physiological treatments (*e.g.,* infusion of a tumor sample with lymphocytes). Biological processes include, but are not limited to, progression of cell cycle, activation of functional pathways, signaling pathways, differentiation, tumorization, metastasis, and/or cell death.

In some embodiments, the tissue sample is characterized by the presence or absence of one or more known analytes of interest (*e.g.,* expression, translation, silencing, and/or deactivation of one or more biomarkers). In some embodiments, the tissue sample is characterized by a relative abundance of one or more known analytes of interest (*e.g.,* upregulation and/or downregulation of one or more biomarkers). In some embodiments, the tissue sample is characterized by the presence, absence, and/or relative abundance of a plurality of analytes of interest (*e.g.,* transcriptome, proteome, metabolome, and/or lipidome). In some embodiments, the tissue sample has no available or previously determined expression or abundance profiles.

In some embodiments, the tissue sample is a tissue section (*e.g.,* a sectioned tissue sample). For instance, referring to Block 1004, in some embodiments, the tissue sample is a sectioned tissue sample that has a depth of 30 microns or less. In some embodiments, the tissue sample is a sectioned tissue sample that has a depth of 10 microns or less. In some embodiments, the tissue sample is a sectioned tissue sample that has a depth of between 4 microns and 30 microns.

In some embodiments, the tissue sample is a sectioned tissue sample having a depth of 500 microns or less. In some embodiments, the tissue sample is a sectioned tissue sample having a depth of 100 microns or less. In some embodiments, the sectioned tissue sample has a depth of 80 microns or less, 70 microns or less, 60 microns or less, 50 microns or less, 40 microns or less, 25 microns or less, 20 microns or less, 15 microns or less, 10 microns or less, 5 microns or less, 2 microns or less, or 1 micron or less. In some embodiments, the tissue sample is a sectioned tissue sample having a depth of at least 0.1 microns, at least 1 micron, at least 5 microns, at least 10 microns, at least 15 microns, at least 20 microns, at least 30 microns, at least 50 microns, or at least 80 microns. In some embodiments, the sectioned tissue sample has a depth of between 10 microns and 20 microns, between 1 and 10 microns, between 0.1 and 5 microns, between 20 and 100 microns, between 1 and 50 microns, or between 0.5 and 10 microns. In some embodiments, the sectioned tissue sample falls within another range starting no lower than 0.1 microns and ending no higher than 500 microns. Further embodiments of tissue sections are provided herein (*see,* "(A) General Definitions: Biological Samples," above).

In some embodiments, the tissue sample comprises a plurality of analytes. In some embodiments, the plurality of analytes of the tissue sample comprises five or more analytes, ten or more analytes, fifty or more analytes, one hundred or more analytes, five hundred or more analytes, 1000 or more analytes, 2000 or more analytes, or between 2000 and 100,000 analytes.

In some embodiments, the plurality of analytes comprises at least 5, at least 10, at least 20, at least 50, at least 100, at least 200, at least 300, at least 400, at least 500, at least 600, at least 700, at least 800, at least 900, at least 1000, at least 2000, at least 3000, at least 5000, at least 6000, at least 7000, at least 8000, at least 9000, at least 10,000, at least 20,000, at least 30,000, at least 40,000, at least 50,000, at least 100,000, at least 200,000, or at least 300,000 analytes. In some embodiments, the plurality of analytes comprises no more than 500,000, no more than 200,000, no more than 100,000, no more than 80,000, no more than 50,000, no more than 30,000, no more than 20,000, no more than 10,000, no more than 5000, no more than 3000, no more than 2000, no more than 1000, no more than 500, no more than 100, or no more than 50 analytes. In some embodiments, the plurality of analytes comprises between 5 and 2000, between 1000 and 100,000, between 2000 and 10,000, between 5000 and 50,000, between 50 and 5000, or between 100 and 10,000 analytes. In some embodiments, the plurality of analytes falls within another range starting no lower than 5 analytes and ending no higher than 500,000 analytes.

In some embodiments, the plurality of analytes comprises DNA, RNA, proteins, or a combination thereof. For instance, in some embodiments, each respective analyte in the plurality of analytes is the same type of analyte. In some embodiments, the plurality of analytes includes at least an analyte of a first type (*e.g.,* RNA molecule) and an analyte of a second type (*e.g*., protein). In some embodiments, the plurality of analytes comprises a plurality of analyte types (*e.g.,* RNA and protein, RNA and DNA, DNA and protein, or a combination of RNA, DNA, and protein). Examples of suitable analytes contemplated for use in the present disclosure are described in further detail herein (*see,* "(A) General Definitions: Analytes," above).

In some embodiments, the tissue sample is attached (*e.g.,* mounted) onto a substrate. In some embodiments, the tissue sample is attached onto a substrate in a first orientation, where the first orientation is a position, relative to the dimensions of the substrate, in which the tissue sample is attached to the substrate. In some embodiments, the first orientation is a position, relative to the one or more fiducial markers on the substrate, in which the tissue sample is attached to the substrate. In some implementations, the tissue sample can be placed on the substrate in any number of orientations, including a first, second, third, fourth, and/or fifth orientation. In some implementations, the tissue sample is placed on the substrate in more than five orientations. In some embodiments, the method includes obtaining additional images of the tissue sample on the substrate at different orientations. For instance, in some embodiments, the method includes, for each respective orientation in a plurality of orientations, obtaining a respective set of images of the tissue sample while the tissue sample is overlaid on a substrate in the respective orientation.

In some embodiments, the substrate comprises a sample area into which the tissue sample is to be placed. In some embodiments, the substrate further includes a sample area indicator identifying the sample area. In some embodiments, the substrate includes one or more spatial fiducials **1130.**

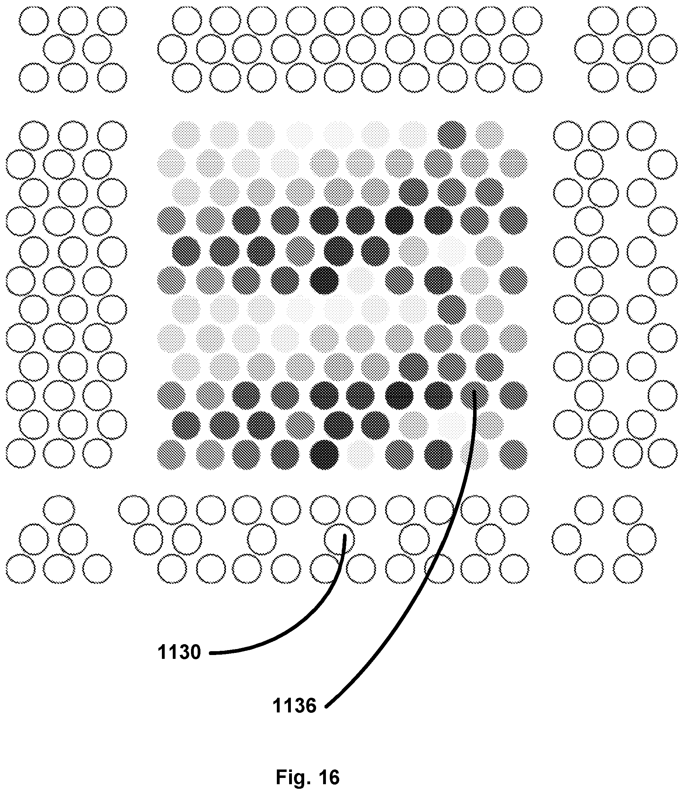

For instance, **FIG. 16** illustrates a substrate (*e.g.,* a chip) that has a plurality of spatial fiducials **1130** and a plurality of capture spots **1136,** in accordance with an embodiment of the present disclosure.

In some embodiments, the tissue sample on a first substrate is overlayed on a second substrate (*e.g.,* in a sandwich configuration). Additional suitable embodiments for substrates that are contemplated for use in the present disclosure include any of the embodiments described herein, such as those disclosed above (*see*, "(A) General Definitions: Substrates") and in PCT publication 202020176788A1, entitled "Profiling of biological analytes with spatially barcoded oligonucleotide arrays"; U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue".

Alignment of substrates, including sandwich configurations, are further described in U.S. Patent Application No. 63/080547, entitled "Sample Handling Apparatus and Image Registration Methods," filed September 18, 2020; U.S. Patent Application No. 63/080,514, entitled "Sample Handling Apparatus and Fluid Delivery Methods," filed September 18, 2020; U.S. Patent Application No. 63/155,173, entitled "Sample Handling Apparatus and Image Registration Methods," filed March 1, 2021; PCT Publication No. WO2020123320A2, entitled "Imaging system hardware"; and U.S. Provisional Patent Application No. 63/245,453, entitled "Systems and Methods for Image Registration or Alignment," filed September 17, 2021.

In some embodiments, each capture spot in the plurality of capture spots 1136 is attached directly or attached indirectly to the substrate.

In some embodiments, the plurality of capture spots comprises at least 50, at least 100, at least 200, at least 300, at least 400, at least 500, at least 1000, at least 2000, at least 3000, at least 4000, at least 5000, at least 10,000, at least 15,000, at least 20,000, or at least 40,000 capture spots. In some embodiments, the plurality of capture spots comprises no more than 100,000, no more than 50,000, no more than 20,000, no more than 10,000, no more than 5000, no more than 1000, no more than 500, or no more than 100 capture spots. In some embodiments, the plurality of capture spots comprises from 100 to 500, between 500 and 1000, from 1000 to 5000, from 5000 to 10,000, from 10,000 to 15,000, or from 15,000 to 20,000 capture spots. In some embodiments, the plurality of capture spots falls within another range starting no lower than 50 capture spots and ending no higher than 100,000 capture spots.

In some embodiments, the plurality of capture spots comprises at least 50, at least 100, at least 200, at least 500, at least 1000, at least 2000, at least 5000, at least 10,000, at least 100,000, at least 500,000, or at least 1 million capture spots. In some embodiments, the plurality of capture spots comprises no more than 5 million, no more than 1 million, no more than 100,000, no more than 10,000, no more than 1000, or no more than 500 capture spots. In some embodiments, the plurality of capture spots comprises from 100 to 10,000, from 300 to 5000, from 2000 to 100,000, or from 50,000 to 500,000 capture spots. In some embodiments, the plurality of capture spots falls within another range starting no lower than 50 capture spots and ending no higher than 5 million capture spots.

In some embodiments, each respective capture spot in the plurality of capture spots includes a plurality of capture probes. In some embodiments, the plurality of capture probes includes 500 or more, 1000 or more, 2000 or more, 3000 or more, 5000 or more, 10,000 or more, 20,000 or more, 30,000 or more, 50,000 or more, 100,000 or more, 500,000 or more, 1 x 10⁶ or more, 2 x 10⁶ or more, or 5 x 10⁶ or more capture probes. In some embodiments, the plurality of capture probes includes no more than 1 x 10⁷, no more than 1 x 10⁶, no more than 100,000, no more than 50,000, no more than 10,000, no more than 5000, no more than 2000, or no more than 1000 capture probes. In some embodiments, the plurality of capture probes is from 500 to 10,000, from 5000 to 100,000, from 1000 to 1 x 10⁶, from 10,000 to 500,000, or from 1 x 10⁶ to 1 x 10⁷ capture probes. In some embodiments, the plurality of capture probes falls within another range starting no lower than 500 capture probes and ending no higher than 1 x 10⁷ capture probes.

In some embodiments, a respective capture spot comprises any area of any two- or three-dimensional geometry (*e.g.,* of any shape). For example, in some embodiments, a shape of each capture spot in the plurality of capture spots on the substrate is a closed-form shape. In some embodiments, a respective capture spot is elliptic or circular. In some embodiments, a respective capture spot is not circular. In some embodiments, the closed-form shape is square or rectangular.

In some embodiments, the closed-form shape is elliptic or circular and each capture spot in the plurality of capture spots has a diameter of between 3 microns and 90 microns. In some embodiments, the closed-form shape is elliptic or circular and each capture spot in the plurality of capture spots has a diameter of between 3 microns and 20 microns. In some embodiments, the closed-form shape is elliptic or circular and each capture spot in the plurality of capture spots has a diameter of between 2 microns and 90 microns. In some embodiments, the closed-form shape is elliptic or circular and each capture spot in the plurality of capture spots has a diameter of between 2 microns and 20 microns. In some embodiments, the closed-form shape is square or rectangular and, for each capture spot in the plurality of capture spots, a dimension (*e.g.,* a length and/or a width) of the respective capture spot is between 2 microns and 90 microns. In some embodiments, the closed-form shape is square or rectangular and, for each capture spot in the plurality of capture spots, a dimension (*e.g.,* a length and/or a width) of the respective capture spot is between 2 microns and 20 microns. In some embodiments, each capture spot in the plurality of capture spots has a diameter and/or a dimension (*e.g.,* a length and/or a width) of at least 1, at least 2, at least 3, at least 4, at least 5, at least 10, at least 15, at least 20, at least 30, at least 40, at least 50, at least 60, at least 70, at least 80, at least 90, or at least 100 microns. In some embodiments, each capture spot in the plurality of capture spots has a diameter and/or a dimension (*e.g.,* a length and/or a width) of no more than 200, no more than 100, no more than 80, no more than 60, no more than 40, no more than 30, no more than 20, or no more than 10 microns. In some embodiments, each capture spot in the plurality of capture spots has a diameter and/or a dimension (*e.g.,* a length and/or a width) of from 2 to 10 microns, from 3 to 8 microns, from 10 to 30 microns, from 20 to 80 microns, from 30 to 60 microns, from 40 to 150 microns, or from 80 to 200 microns. In some embodiments, each capture spot in the plurality of capture spots has a diameter and/or a dimension (*e.g.,* a length and/or a width) that falls within another range starting no lower than 1 micron and ending no higher than 200 microns. In some embodiments, a first dimension of the closed-form shape (*e.g.,* a length and/or a width) is the same or different as a second dimension of the closed-form shape (*e.g.,* a length and/or a width).

In some embodiments, each respective capture spot in the plurality of capture spots is contained within a 10 micron by 10 micron square on the substrate. In some embodiments, each respective capture spot in the plurality of capture spots is contained within a 2 micron by 2 micron square on the substrate. In some embodiments, each respective capture spot in the plurality of capture spots is contained within a square having dimensions of at least 1 micron by 1 micron, at least 2 microns by 2 microns, at least 5 microns by 5 microns, at least 10 microns by 10 microns, at least 20 microns by 20 microns, at least 30 microns by 30 microns, at least 50 microns by 50 microns, or at least 100 microns by 100 microns. In some embodiments, each respective capture spot in the plurality of capture spots is contained within a square having dimensions of no more than 200 microns by 200 microns, no more than 100 microns by 100 microns, no more than 50 microns by 50 microns, no more than 20 microns by 20 microns, no more than 10 microns by 10 microns, or no more than 5 microns by 5 microns.

In some embodiments, the plurality of capture spots is positioned on a respective substrate in a specific arrangement. In some such embodiments, the plurality of capture spots is provided as a capture spot array. In some embodiments, each respective capture spot in the plurality of capture spots is at a different position in a two-dimensional array on the substrate. In some embodiments, a distance between a center of each respective capture spot to a neighboring capture spot in the plurality of capture spots on the substrate is between 2 microns and 8 microns. In some embodiments, a distance between a center of each respective capture spot to a neighboring capture spot in the plurality of capture spots on the substrate is between 4 microns and 8 microns. In some embodiments, a distance between a center of each respective capture spot to a neighboring capture spot in the plurality of capture spots on the substrate is at least 1, at least 2, at least 3, at least 4, at least 5, at least 10, at least 20, at least 30, at least 50, at least 80, at least 100, or at least 200 microns. In some embodiments, a distance between a center of each respective capture spot to a neighboring capture spot in the plurality of capture spots on the substrate is no more than 500, no more than 200, no more than 100, no more than 50, no more than 30, no more than 20, no more than 10 microns, or no more than 5 microns. In some embodiments, a distance between a center of each respective capture spot to a neighboring capture spot in the plurality of capture spots on the substrate is from 3 to 10, from 5 to 20, from 10 to 50, from 30 to 150, from 80 to 120, from 100 to 200, or from 200 to 500 microns. In some embodiments, a distance between a center of each respective capture spot to a neighboring capture spot in the plurality of capture spots on the substrate falls within another range starting no lower than 2 microns and ending no higher than 500 microns.

In some embodiments, each capture spot corresponds to a plurality of cells in the tissue sample. In some such embodiments, each respective capture spot in the plurality of capture spots corresponds to a different plurality of cells in the tissue sample.

In some embodiments, each capture spot corresponds to a single cell in the tissue sample. In some such embodiments, each respective capture spot in the plurality of capture spots corresponds to a different respective cell in the tissue sample.

In some embodiments, each cell in the tissue sample corresponds to one or more capture spots. In some such embodiments, each respective cell in the tissue sample corresponds to a different set of capture spots in the plurality of capture spots. In some embodiments, each cell in the tissue sample corresponds to a single capture spot in the plurality of capture spots. In some embodiments, each cell in the tissue sample corresponds to a different respective capture spot.

In some embodiments, as described above (see, *e.g.,* "(A) General Definitions: Capture spots," and "(B) Methods for Spatial Analysis of Analytes," above), each respective capture spot in the plurality of capture spots is represented by a respective spatial barcode in a plurality of spatial barcodes. Accordingly, referring to Block 1006, in some embodiments, each spatial barcode in the plurality of spatial barcodes encodes a unique predetermined value selected from the set {1, ..., 1024}, {1, ..., 4096}, {1, ..., 16384}, {1, ..., 65536}, {1, ..., 262144}, {1, ..., 1048576}, {1, ..., 4194304}, {1, ..., 16777216}, {1, ..., 67108864}, or {1, ..., 1 x 10¹²}.

Numerous additional embodiments of capture spots, including capture spot sizes, capture spot arrays, capture probes, spatial barcodes, analytes, capture domain types, and/or other features of capture spots including but not limited to dimensions, designs, and modifications, and any substitutions and/or combinations thereof, are discussed in detail above (*e.g.,* in "(A) General Definitions: Capture Probes," "(A) General Definitions: Capture spots," "(A) General Definitions: Capture spot arrays," and "(B) Methods for Spatial Analysis of Analytes," above).

### Obtaining images.

Referring again to Block 1002, in some embodiments, the obtaining the set of images are performed using any suitable imaging technique known in the art. In some embodiments, the obtaining any one respective image in the set of images includes any of the embodiments disclosed herein with respect to the obtaining any other respective image in the set of images. For example, in some embodiments, a respective image in the set of images is a histological image of the tissue sample. In some embodiments, a histological image generally refers to any image that contains structural information for a biological sample and/or a biological tissue. In some embodiments, a histological image is obtained using any suitable stain, as described in further detail below.

In some embodiments, a respective image includes one or more spatial fiducials (*e.g.,* where the substrate comprises the one or more spatial fiducials). In some embodiments, a respective image does not include spatial fiducials.

Referring to Block 1008, in some embodiments, the method further comprising exposing, prior to the obtaining, the tissue sample on the substrate with each respective detectable marker in the set of detectable markers.

In some embodiments, the set of detectable markers includes at least 1, at least 2, at least 3, at least 4, at least 5, at least 6, at least 7, at least 8, at least 9, or at least 10 detectable markers. In some embodiments, the set of detectable markers includes no more than 20, no more than 10, no more than 8, no more than 5, or no more than 3 detectable markers. In some embodiments, the set of detectable markers includes from 1 to 4, from 2 to 5, from 2 to 9, from 3 to 8, from 4 to 12, or from 10 to 20 detectable markers. In some embodiments, the set of detectable markers falls within another range starting no lower than 1 detectable marker and ending no higher than 20 detectable markers.

In some embodiments, the number of detectable markers in the set of detectable markers is determined by a number of wavelengths that can be detected by an imaging apparatus. For instance, in some embodiments, the number of detectable markers in the set of detectable markers is no more than the number of excitation wavelengths **1124** (*e.g.,* imaging channels) that can be detected by a given imaging apparatus with minimal or no overlapping spectra. In some alternative embodiments, the number of detectable markers in the set of detectable markers is more than the number of excitation wavelengths **1124** (*e.g.,* imaging channels) that can be detected by a given imaging apparatus.

In some embodiments, the number of detectable markers in the set of detectable markers is determined by a number of analytes of interest to be visualized in the tissue sample. For instance, in some embodiments, the number of detectable markers in the set of detectable markers is no more than the number of analytes of interest (*e.g.,* different molecules, proteins, *etc*.) to be stained and/or visualized in the tissue sample. In some alternative embodiments, the number of detectable markers in the set of detectable markers is more than the number of analytes of interest to be stained and/or visualized in the tissue sample.

A respective detectable marker in the set of detectable markers is used to detect (*e.g.,* is specific to) a respective cellular component in the tissue sample (*e.g.,* an organelle). According to the claimed invention, the cellular component is a nucleus. In some embodiments, the cellular component is a cell membrane. In some embodiments, the cellular component is a molecule (*e.g.,* lipid, protein, nucleic acid molecule, metabolite, and/or small molecule). In some implementations, each respective detectable marker in the set of detectable markers is used to detect (*e.g.,* is specific to) one or more analytes of interest in the tissue sample. In some embodiments, each respective detectable marker in the set of detectable markers hybridizes to the one or more analytes of interest in the tissue sample. For example, a lipid dye can be used to detect lipid membranes within a tissue sample. In another example, an antibody-dye conjugate can be used to detect specific proteins within a tissue sample.

Accordingly, in some embodiments, a respective detectable marker in the set of detectable markers is a fluorescent dye attached to an antibody. Referring to Block 1010, in some embodiments, each respective detectable marker in the set of detectable markers is a different fluorescent dye attached to a different antibody (*e.g.,* where each respective antibody hybridizes to one or more analytes of interest in the tissue sample).

In some embodiments, a respective detectable marker in the set of detectable markers is specific to single-stranded and/or double-stranded nucleic acid sequences in the tissue sample (*e.g.,* an oligonucleotide probe and/or a conjugated nucleic acid). In some embodiments, each respective detectable marker in the set of detectable markers is specific to single-stranded and/or double-stranded nucleic acid sequences in the tissue sample (*e.g.,* an oligonucleotide probe and/or a conjugated nucleic acid). For instance, in some implementations, a detectable marker is an oligonucleotide probe used for *in situ hybridization.*

In some embodiments, a respective detectable marker in the set of detectable markers is non-specific for nucleic acid sequences in the tissue sample (*e.g.,* an intercalating dye). For instance, in some implementations, a detectable marker is an intercalating dye that is non-specific for nucleic acid sequences in the tissue sample, and the detectable marker detects a presence of a cell nucleus. In some such implementations, the detectable marker is 4',6-diamidino-2-phenylindole (DAPI).

In some embodiments, each respective detectable marker in the set of detectable markers is specific to a different analyte in a plurality of analytes (*e.g.,* cellular components). For instance, as described in Example 1, below, a first detectable marker (FITC) is specific for a first analyte (*e.g.,* neuronal protein NeuN) and a second detectable marker (DAPI) is specific for a different, second analyte (*e.g.,* double-stranded DNA).

Any suitable detectable marker and/or dye for detecting, visualizing, and/or hybridizing to one or more analytes of interest is contemplated for use in the present disclosure, as will be apparent to one skilled in the art. For example, in some embodiments, a respective detectable marker in the set of detectable markers is a fluorophore labeled antibody, a fluorescent label, a radioactive label, a chemiluminescent label, a colorimetric label, or a combination thereof. Referring to Block 1012, in some embodiments, each respective detectable marker in the set of detectable markers is a fluorophore labeled antibody, a fluorescent label, a radioactive label, a chemiluminescent label, a colorimetric label, or a combination thereof. Referring to Block 1014, in some embodiments, a respective detectable marker in the set of detectable markers is live/dead stain, trypan blue, periodic acid-Schiff reaction stain, Masson's trichrome, Alcian blue, van Gieson, reticulin, Azan, Giemsa, Toluidine blue, isamin blue, sudan black and osmium, acridine orange, Bismarck brown, carmine, Coomassie blue, cresyl violet, DAPI, eosin, ethidium bromide, acid fuchsine, hematoxylin, Hoechst stains, iodine, methyl green, methylene blue, neutral red, Nile blue, Nile red, osmium tetroxide, propidium iodide, rhodamine, safranin, or a combination thereof. In some embodiments, each respective detectable marker in the set of detectable markers is live/dead stain, trypan blue, periodic acid-Schiff reaction stain, Masson's trichrome, Alcian blue, van Gieson, reticulin, Azan, Giemsa, Toluidine blue, isamin blue, sudan black and osmium, acridine orange, Bismarck brown, carmine, Coomassie blue, cresyl violet, DAPI, eosin, ethidium bromide, acid fuchsine, hematoxylin, Hoechst stains, iodine, methyl green, methylene blue, neutral red, Nile blue, Nile red, osmium tetroxide, propidium iodide, rhodamine, safranin, or a combination thereof.

In some embodiments, a first detectable marker in the set of detectable markers is indicative of a particular cell type (*e.g.,* cancer cell, normal cell, healthy cell, diseased cell, *etc*.) and/or a particular disease condition (*e.g.,* a cancer stage, cancer type, tissue of origin, *etc*.). For instance, in some embodiments, different cell types can express different abundances of various analytes, including proteins, nucleic acid molecules, lipids, metabolites, and/or small molecules. Accordingly, in some embodiments, the first detectable marker comprises an analyte capture moiety that is specific to a cellular component and/or analyte (*e.g.,* protein, nucleic acid, lipid, metabolite, and/or small molecule) that is expressed in or on the particular cell type. In some such embodiments, the first detectable marker comprises an antibody-dye conjugate that is specific to a protein that is expressed in or on the particular cell type. In some embodiments, the first detectable marker comprises a nucleic acid-detectable moiety conjugate that is specific to a nucleic acid molecule (*e.g*., DNA and/or RNA) that is expressed in or on the particular cell type. In some embodiments, one or more detectable markers in the set of detectable markers is selected based on the desired cell type and/or disease condition to be characterized.

In some embodiments, the tissue sample that is exposed to the set of detectable markers is imaged using any suitable imaging technique, as will be apparent to one skilled in the art. In some embodiments, a respective image 1122 (*e.g.,* in the set of images 1120) is obtained by bright-field microscopy, immunohistochemistry, or fluorescence microscopy. Referring to Block 1016, in some embodiments, the obtaining uses fluorescence microscopy to obtain each image in the set of images. In some embodiments, each respective image in the set of images is obtained by immunofluorescence microscopy.

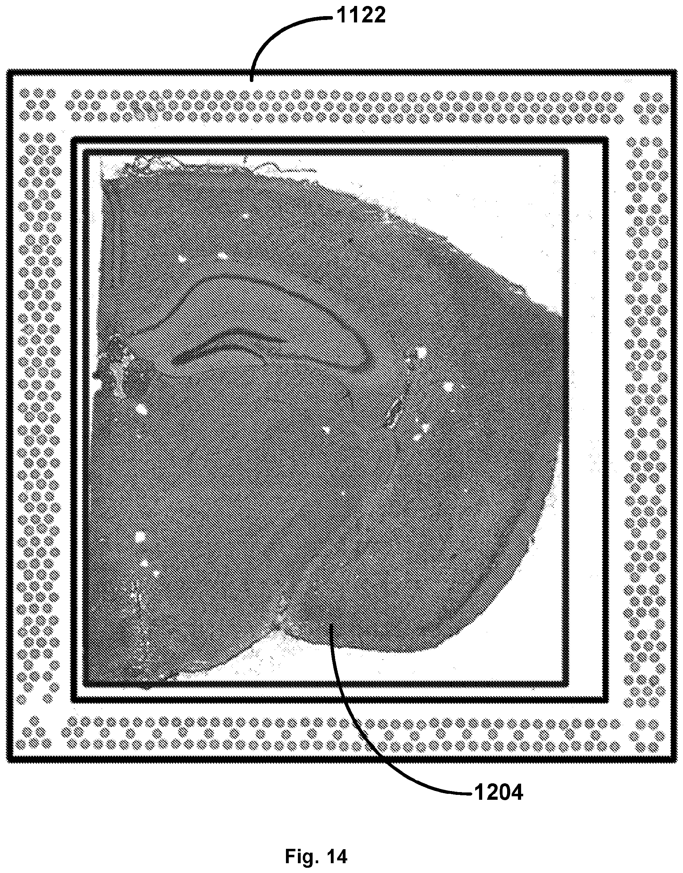

For instance, in some embodiments, a respective image is acquired using transmission light microscopy (*e.g.,* bright field transmission light microscopy, dark field transmission light microscopy, oblique illumination transmission light microscopy, dispersion staining transmission light microscopy, phase contrast transmission light microscopy, differential interference contrast transmission light microscopy, emission imaging, *etc*.). *See, e.g.,* Methods in Molecular Biology, 2018, Light Microscopy Method and Protocols, Markaki and Harz eds., Humana Press, New York, New York, ISBN-13: 978-1493983056. As an illustration, **FIG. 14** shows an example of an image **1122** of a tissue sample on a substrate in accordance with some embodiments.

In some embodiments, an image **1122** is a bright-field microscopy image in which the imaged sample appears dark on a bright background. In some such embodiments, as described above, the sample has been stained (*e.g.,* with a detectable marker). For instance, in some embodiments, the sample has been stained with Hematoxylin and Eosin and the image **1122** is a bright-field microscopy image. In some embodiments the sample has been stained with a Periodic acid-Schiff reaction stain (stains carbohydrates and carbohydrate rich macromolecules a deep red color) and the image is a bright-field microscopy image. In some embodiments the sample has been stained with a Masson's trichrome stain (nuclei and other basophilic structures are stained blue, cytoplasm, muscle, erythrocytes and keratin are stained bright-red, collagen is stained green or blue, depending on which variant of the technique is used) and the image is a bright-field microscopy image. In some embodiments, the sample has been stained with an Alcian blue stain (a mucin stain that stains certain types of mucin blue, and stains cartilage blue and can be used with H&E, and with van Gieson stains) and the image is a bright-field microscopy image. In some embodiments the sample has been stained with a van Gieson stain (stains collagen red, nuclei blue, and erythrocytes and cytoplasm yellow, and can be combined with an elastin stain that stains elastin blue/black) and the image is a bright-field microscopy image. In some embodiments the sample has been stained with a reticulin stain, an Azan stain, a Giemsa stain, a Toluidine blue stain, an isamin blue/eosin stain, a Nissl and methylene blue stain, and/or a sudan black and osmium stain and the image is a bright-field microscopy image.

In some embodiments, rather than a bright-field microscopy image of a sample, an image **1122** is an immunohistochemistry (IHC) image. IHC imaging may utilize a staining technique using antibody labels. One form of immunohistochemistry (IHC) imaging is immunofluorescence (IF) imaging. In an example of IF imaging, primary antibodies are used that specifically label a protein in the biological sample, and then a fluorescently labelled secondary antibody or other form of probe is used to bind to the primary antibody, to show up where the first (primary) antibody has bound. A light microscope, equipped with fluorescence, is used to visualize the staining. The fluorescent label is excited at one wavelength of light and emits light at a different wavelength. Using the right combination of filters, the staining pattern produced by the emitted fluorescent light is observed. In some embodiments, a tissue sample is exposed to several different primary antibodies (or other forms of probes) in order to quantify several different proteins in a biological sample. In some such embodiments, each such respective different primary antibody (or probe) is then visualized with a different fluorescence label (different channel) that fluoresces at a unique wavelength or wavelength range (relative to the other fluorescence labels used). In this way, several different proteins in the biological sample can be visualized.

More generally, in some embodiments of the present disclosure, in addition to or instead of bright-field imaging, fluorescence imaging is used to acquire a respective image of the sample. As used herein the term "fluorescence imaging" or "fluorescence microscopy" refers to imaging that relies on the excitation and re-emission of light by fluorophores, regardless of whether they are added experimentally to the sample and bound to antibodies (or other compounds) or naturally occurring features of the sample. The above-described IHC imaging, and in particular IF imaging, is just one form of fluorescence imaging. Accordingly, in some embodiments, a respective image **1122** (*e.g.,* of a biological sample) represents a respective channel in one or more channels, where each respective channel in the one or more channels represents an independent (*e.g.,* different) wavelength or a different wavelength range (*e.g.,* corresponding to a different emission wavelength). In some embodiments, a respective image **1122** comprises a plurality of instances of the respective image, where each respective instance of the respective image represents an independent (*e.g.,* different) wavelength or a different wavelength range (*e.g.,* corresponding to a different emission wavelength).

In some embodiments, where each respective image **1122** in the set of images is obtained at a different corresponding wavelength in a plurality of excitation wavelengths, the set of images forms a stack of images, where each respective image corresponds to a different "page" (*e.g.,* channel) in the stack.

In some embodiments in which fluorescence imaging is conducted, a respective image (*e.g.,* in the set of images) is acquired using Epi-illumination mode, where both the illumination and detection are performed from one side of the sample.

In some such embodiments, a respective image (*e.g.,* in the set of images) is acquired using confocal microscopy, two-photon imaging, wide-field multiphoton microscopy, single plane illumination microscopy or light sheet fluorescence microscopy. See, for example, Adaptive Optics for Biological Imaging, 2013, Kubby ed., CRC Press, Boca Raton, Florida; and Confocal and Two-Photon Microscopy: Foundations, Applications and Advances, 2002, Diaspro ed., Wiley Liss, New York, New York; and Handbook of Biological Confocal Microscopy, 2002, Pawley ed., Springer Science+Business Media, LLC, New York, New York.

In some embodiments, a respective image (*e.g.,* in the set of images) is obtained using various immunohistochemistry (IHC) probes that excite at various different wavelengths. See, *for example,* Day and Davidson, 2014, "The Fluorescent Protein Revolution (In Cellular and Clinical Imaging)," CRC Press, Taylor & Francis Group, Boca Raton, Florida; "Quantitative Imaging in Cell Biology" Methods in Cell Biology 123, 2014, Wilson and Tran, eds.; Advanced Fluorescence Reporters in Chemistry and Biology II: Molecular Constructions, Polymers and Nanoparticles (Springer Series on Fluorescence), 2010, Demchenko, ed., Springer-Verlag, Berlin, Germany; Fluorescence Spectroscopy and Microscopy: Methods and Protocols (Methods in Molecular Biology) 2014th Edition, 2014, Engelborghs and Visser, eds., HumanPress.

Accordingly, in some embodiments, the set of images includes at least 1, at least 2, at least 3, at least 4, at least 5, at least 6, at least 7, at least 8, at least 9, or at least 10 images. In some embodiments, the set of images includes no more than 20, no more than 10, no more than 8, no more than 5, or no more than 3 images. In some embodiments, the set of images includes from 1 to 4, from 2 to 5, from 2 to 9, from 3 to 8, from 4 to 12, or from 10 to 20 images. In some embodiments, the set of images falls within another range starting no lower than 1 image and ending no higher than 20 images.

In some embodiments, the obtaining the set of images is performed using at least 1, at least 2, at least 3, at least 4, at least 5, at least 6, at least 7, at least 8, at least 9, or at least 10 excitation wavelengths (*e.g.,* channels). In some embodiments, the obtaining the set of images is performed using no more than 20, no more than 10, no more than 8, no more than 5, or no more than 3 excitation wavelengths (*e.g.,* channels). In some embodiments, the obtaining the set of images is performed using from 1 to 4, from 2 to 5, from 2 to 9, from 3 to 8, from 4 to 12, or from 10 to 20 excitation wavelengths (*e.g.,* channels). In some embodiments, the obtaining the set of images is performed using another range of excitation wavelengths starting no lower than 1 excitation wavelength and ending no higher than 20 excitation wavelengths. In some embodiments, the plurality of excitation wavelengths includes at least 3 excitation wavelengths corresponding to a red illumination (*e.g.,* red channel), a green illumination (*e.g.,* green channel), and a blue illumination (*e.g.,* blue channel).

In some embodiments, one or more images in the set of images are obtained at an excitation wavelength **1124** for a corresponding detectable marker specific to a respective analyte. In other words, in some embodiments, each respective image **1122** in the set of images is obtained at a different corresponding wavelength **1124** in a plurality of excitation wavelengths, and each such wavelength corresponds to the excitation frequency of a different detectable marker (*e.g.,* containing a fluorophore) within or spatially associated with the sample. Each respective detectable marker is specific to an analyte in the tissue sample, which can be a natural substance in the sample (*e.g.,* a type of molecule that is naturally within the sample), or one that has been added to the sample. In some such embodiments, each respective detectable marker in the set of detectable markers excites at a different specific wavelength in the plurality of excitation wavelengths. Such detectable markers can be directly added to the sample, or they can be conjugated to antibodies that are specific for a particular antigen occurring within the sample, such as one that is exhibited by a particular protein. In this way, a user can use the set of images **1122** to view spatial analyte data overlayed onto fluorescence image data, thus providing information on the relationship between gene (or antibody) expression and other cellular markers (*e.g.,* proteins exhibit particular antigens).

In some embodiments, one or more images in the set of images are obtained at an excitation wavelength for one or more fiducial markers on the substrate. In some embodiments, the one or more fiducial markers are imaged at an excitation wavelength that is different from any excitation wavelength that is used to detect any detectable marker in the set of detectable markers. In some embodiments, the one or more fiducial markers are imaged at an excitation wavelength that is different from any excitation wavelength that is used to image the tissue sample. In some embodiments, the one or more fiducial markers are imaged using brightfield microscopy.

For instance, as described below in Example 1, a first excitation wavelength (*e.g.,* channel) is used to excite and obtain an emission image for a first detectable marker (FITC) that is specific for a first analyte (*e.g.,* neuronal protein NeuN), a second excitation wavelength (*e.g.,* channel) is used to excite and obtain an emission image for a second detectable marker (DAPI) that is specific for a second analyte (*e.g.,* double-stranded DNA), and a third excitation wavelength (*e.g.,* channel) is used to excite and obtain an emission image for one or more fiducial markers (*e.g.,* TRITC).

In some embodiments, one or more images in the set of images is not an emission image. In some such embodiments, one or more images in the set of images is an image of the tissue sample on the substrate, obtained using brightfield microscopy.

Additional suitable embodiments for obtaining images that are contemplated for use in the present disclosure include any of the embodiments described herein, such as those disclosed above (see, "Definitions: (A) General Definitions: Imaging") and

in U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue," and U.S. PCT Publciation No. WO2023/044071, entitled "Systems and Methods for Image Registration or Alignment".

In some embodiments, a respective image comprises a plurality of pixels. In some embodiments, the plurality of pixels comprises at least 100, at least 500, at least 1000, at least 5000, at least 10,000, at least 50,000, at least 100,000, at least 500,000, at least 1 x 10⁶, at least 2 x 10⁶, at least 3 x 10⁶, at least 5 x 10⁶, at least 8 x 10⁶, at least 1 x 10⁷, at least 1 x 10⁸, at least 1 x 10⁹, at least 1 x 10¹⁰, or at least 1 x 10¹¹ pixels. In some embodiments, the plurality of pixels comprises no more than 1 x 10¹², no more than 1 x 10¹¹, no more than 1 x 10¹⁰, no more than 1 x 10⁹, no more than 1 x 10⁸, no more than 1 x 10⁷, no more than 1 x 10⁶, no more than 100,000, no more than 10,000, or no more than 1000 pixels. In some embodiments, the plurality of pixels comprises from 1000 to 100,000, from 10,000 to 500,000, from 100,000 to 1 x 10⁶, from 500,000 to 1 x 10⁹, or from 1 x 10⁶ to 1 x 10⁸ pixels. In some embodiments, the plurality of pixels falls within another range starting no lower than 100 pixels and ending no higher than 1 x 10¹² pixels.

In some embodiments, a respective image in the set of images is represented as an array (*e.g*., matrix) comprising a plurality of pixels, such that the location of each respective pixel in the plurality of pixels in the array (e.g., matrix) corresponds to its original location in the image. In some embodiments, a respective image is represented as a vector comprising a plurality of pixels, such that each respective pixel in the plurality of pixels in the vector comprises spatial information corresponding to its original location in the image. Accordingly, in some embodiments, referring to Block 1018, each image in the set of images comprises a corresponding plurality of pixels in the form of an array of pixel values, where the array of pixel values comprises at least 100,000 pixel values.

In some embodiments, the plurality of pixels in a respective image corresponds to the location of each capture spot in a plurality of capture spots on the substrate. In some embodiments, each capture spot in the plurality of capture spots is represented by five or more, ten or more, 100 or more, 1000 or more, 10,000 or more, 50,000 or more, 100,000 or more, or 200,000 or more contiguous pixels in a respective image. In some embodiments, each capture spot in the plurality of capture spots is represented by no more than 500,000, no more than 200,000, no more than 100,000, no more than 50,000, no more than 10,000, or no more than 1000 contiguous pixels in a respective image. In some embodiments, each capture spot is represented by between 1000 and 250,000, between 100,000 and 500,000, between 10,000 and 100,000, or between 5000 and 20,000 contiguous pixels in a respective image. In some embodiments, each capture spot is represented by another range of contiguous pixels in a respective image starting no lower than 5 pixels and ending no higher than 500,000 pixels.

An image can be obtained in any electronic image file format, including but not limited to JPEG/JFIF, TIFF, Exif, PDF, EPS, GIF, BMP, PNG, PPM, PGM, PBM, PNM, WebP, HDR raster formats, HEIF, BAT, BPG, DEEP, DRW, ECW, FITS, FLIF, ICO, ILBM, IMG, PAM, PCX, PGF, JPEG XR, Layered Image File Format, PLBM, SGI, SID, CD5, CPT, PSD, PSP, XCF, PDN, CGM, SVG, PostScript, PCT, WMF, EMF, SWF, XAML, and/or RAW. In some embodiments, a respective image is obtained in any electronic color mode, including but not limited to grayscale, bitmap, indexed, RGB, CMYK, HSV, lab color, duotone, and/or multichannel. In some embodiments, the image is manipulated (*e.g.,* stitched, compressed and/or flattened).

In some embodiments, the set of images is a stack of images (e.g., monochrome RGB images).

In some embodiments, each respective image in the set of images has the same dimensions. In some embodiments, a first respective image in the set of images has different dimensions from a second respective image in the set of images.

In some embodiments, the method further comprises modifying one or more images in the set of images. In some embodiments, the modifying an image comprises adjusting a brightness of the image, adjusting a contrast of the image, flipping the image, rotating the image, cropping the image, zooming a view of the image, panning across the image, or overlaying a grid onto the respective image. In some embodiments, the modifying an image comprises preprocessing the image. In some embodiments, the method further comprises performing a normalization of pixel values within the image.

Suitable methods of image normalization and modification are contemplated, including log normalization, smoothing, noise reduction, color normalization, contrast stretching, histogram stretching, Reinhard method, Macenko method, stain color descriptor (SCD), complete color normalization and structure preserving color normalization (SPCN), as will be apparent to one skilled in the art. *See, e.g.,* Roy et al., "Novel Color Normalization Method for Hematoxylin & Eosin Stained Histopathology Images," 2019 IEEE Access 7: 2169-3536; doi: 10.1109/ACCESS.2019.2894791.

Preprocessing of images is further described in, e*.g.,* U.S. Patent Application No. 63/080547, entitled "Sample Handling Apparatus and Image Registration Methods," filed September 18, 2020; U.S. Patent Application No. 63/080,514, entitled "Sample Handling Apparatus and Fluid Delivery Methods," filed September 18, 2020; U.S. Patent Application No. 63/155,173, entitled "Sample Handling Apparatus and Image Registration Methods," filed March 1, 2021; PCT Publication No. WO202012332)A2, entitled "Imaging system hardware"; and U.S. Provisional Patent Application No. 63/245,453, entitled "Systems and Methods for Image Registration or Alignment," filed September 17, 2021.

### First spatial dataset.

Referring to Block 1020, the method further includes acquiring a first spatial dataset 1134-1 comprising, for each respective capture spot 1136 in the plurality of capture spots, for each respective detectable marker in the set of detectable markers, a measured intensity 1144 of the respective capture spot, in the corresponding image in the set of images, indexed by a spatial barcode in a plurality of spatial barcodes 1140 that represents the respective capture spot.

In some embodiments, each respective image in the set of images comprises a plurality of pixels, and the acquiring the first spatial dataset 1134-1 comprises registering each respective pixel in the plurality of pixels to the location of each capture spot in the plurality of capture spots 1136 on the substrate. In some such embodiments, registration of pixels to capture spots is performed, for each respective image in the set of images, for each respective pixel in the plurality of pixels, using a coordinate system that indicates the location of the respective pixel within a first frame of reference for the respective image, and performing a transformation that localizes the respective pixel within a second frame of reference for the plurality of capture spots.

In some embodiments, the respective image comprises one or more fiducial markers on the substrate, the second frame of reference is obtained using a substrate template that includes the spatial coordinates of each respective capture spot in the plurality of capture spots relative to the spatial coordinates of each respective fiducial marker in a plurality of reference fiducial markers, and the transformation is performed by mapping the one or more fiducial markers in the respective image to the plurality of reference fiducial markers in the substrate template. Other methods of image registration are contemplated, as described elsewhere herein (see, e.g., the section entitled "Registration," below).

Accordingly, each pixel in the image can be localized with respect to the plurality of capture spots on the substrate, and each pixel that is localized to a respective capture spot in the plurality of capture spots is assigned to the respective capture spot. Thus, in some embodiments, for each respective pixel in the plurality of pixels, a measured intensity for the respective pixel is similarly assigned to the respective capture spot. In some embodiments, for each respective image in the set of images, each respective capture spot in the plurality of capture spots corresponds to a respective set of pixels, in the plurality of pixels, that are assigned (e.g., by registration) to the respective capture spot.

In some embodiments, the method comprises, for each respective image in the set of images, repeating the registration of pixel coordinates to the plurality of capture spots, thus obtaining a corresponding set of transformations, and applying each respective transformation to each corresponding image. In some embodiments (e.g., where each respective image in the set of images has the same dimensions and/or the same orientation), the method includes performing the registration of pixel coordinates to the plurality of capture spots for a first image in the set of images, thus obtaining a first transformation, and applying the first transformation to each respective image in the set of images.

In some embodiments, the measured intensity is a value that is assigned to each respective capture spot in the plurality of capture spots. As described above, in some embodiments, for a respective image in the set of images, for each respective capture spot in the plurality of capture spots, the measured intensity is obtained by: (i) determining a pixel value for each respective pixel, in the plurality of pixels, that is assigned (e.g., by registration) to the respective capture spot, and (ii) using the pixel value of each respective pixel assigned to the respective capture spot to determine the measured intensity.

In some embodiments, the measured intensity for each respective capture spot is a count, a sum, and/or a measure of central tendency of the pixel values for the set of pixels that are assigned (e.g., by registration) to the respective capture spot. In some embodiments, the measure of central tendency includes, but is not limited to, a mean, arithmetic mean, weighted mean, midrange, midhinge, trimean, geometric mean, geometric median, Winsorized mean, median, and mode of the pixel values across the set of pixels that are assigned to the respective capture spot. In some embodiments, the determining the measured intensity for each respective capture spot comprises, for each respective image in the set of images: (i) determining a respective sub-region of the respective image that corresponds to the respective capture spot and (ii) obtaining a sum, a count, and/or a measure of central tendency for the pixel values within the respective sub-region. In some such embodiments, the determining the measured intensity for each respective capture spot comprises, for each respective image in the set of images: (i) determining a pixel radius for the respective capture spot and (ii) obtaining a measure of central tendency for the pixel values in every direction within the pixel radius of the respective capture spot. In some embodiments, the pixel radius for the respective capture spot is obtained using a known diameter for each respective capture spot in the plurality of capture spots (*see, e.g.,* the section entitled "Samples and substrates," above).

In some embodiments, the method further comprises determining a measure of dispersion of the measured intensity (*e.g.,* the pixel values across the corresponding set of pixels) of the respective capture spot. Measures of dispersion include, but are not limited to, a variance, standard deviation, standard error, and/or confidence interval.

Methods of determining pixel values for a plurality of pixels in an image are known in the art. For example, pixel values can be determined by measuring signal intensity for each respective pixel in the plurality of pixels, using any image processing tool known in the art. A range of software packages are available for processing, visualization and analysis of images, including both general image processing platforms and more specialized tools. For example, FIJI (*see, e.g.,* Schindelin et al., 2012, "Fiji: an open-source platform for biological-image analysis," Nat. Methods 9, 676-682; and Schneider et al., 2012, "NIH Image to ImageJ: 25 years of image analysis," Nat. Methods 9, 671-675) and Vaa3D (*see, e.g.,* Peng et al., 2014, "Extensible visualization and analysis for multidimensional images using Vaa3D," Nat. Protoc. 9, 193-208; and Peng et al., 2010, "V3D enables real-time 3D visualization and quantitative analysis of large-scale biological image data sets," Nat. Biotechnol. 28, 348-353) are open source, extensible platforms for image analysis and visualization. In some embodiments, the pixel values are obtained using any suitable programming language, such as R (*see, e.g.,* R Core Team 2020, "R: A language and environment for statistical computing," R Foundation for Statistical Computing, Vienna, Austria, available on the Internet at R-project.org).

In some embodiments, for each respective image in the set of images, for each respective pixel in the plurality of pixels, the respective pixel value is a numeric value that indicates the intensity of the image (*e.g.,* grayscale intensity) at the respective pixel.

In some embodiments, the pixel values and/or the measured intensity is determined using a low-resolution image, for each respective image in the set of images. In some embodiments, the pixel values and/or the measured intensity is determined using a high-resolution image, for each respective image in the set of images.

As described above, in some embodiments, each respective capture spot in the plurality of capture spots is represented by a respective spatial barcode in a plurality of spatial barcodes. Accordingly, in some such embodiments, the measured intensity for each respective capture spot in the plurality of capture spots is indexed by the respective spatial barcode, in the plurality of spatial barcodes, that represents the respective capture spot.

In some embodiments, the method includes, for each respective image in the set of images, for each respective capture spot in the plurality of capture spots, determining a corresponding measured intensity, thereby obtaining a plurality of measured intensities for the respective image. Thus, in some embodiments, the first spatial dataset includes, for each respective image in the set of images, for each respective capture spot in the plurality of capture spots (*e.g.,* at least 1000 capture spots), a corresponding value that indicates the measured intensity of the capture spot, and that is further indexed by the spatial barcode that represents the respective capture spot. In other words, each respective detectable marker (*e.g.,* antibody) corresponding to a different analyte of interest (*e.g.,* protein) has a pattern of measured intensities (*e.g*., numerical values) associated with the plurality of capture spots on the substrate, which can be visualized as different images in the set of images obtained using different respective excitation wavelengths (*e.g.,* channels).

For instance, Example 1 illustrates a first spatial dataset that includes, for each of the three excitation wavelengths (*e.g.,* channels) used to obtain each respective image in the set of images, a corresponding measured intensity (*e.g.,* value) that is assigned to each capture spot. The measured intensity can be spatially displayed as a relative brightness or a color in a range of colors assigned to all of the capture spots in the array, such as in a spatial heatmap.

### Second spatial dataset.

Referring to Block 1022, the method further includes acquiring a second spatial dataset 1134-2 comprising nucleic acid quantification data comprising, for each respective capture spot 1136 in the plurality of capture spots, for each respective nucleic acid in a plurality of nucleic acids, a corresponding representation 1138 of a number of molecules of the respective nucleic acid originating from the tissue sample while the tissue sample is overlaid on a substrate in the first orientation and localized to the respective capture spot by a corresponding spatial barcode 1140, in the plurality of spatial barcodes, associated with the respective capture spot.

In some embodiments, the first orientation for the second spatial dataset indicates that the tissue sample is overlaid on the substrate in the same position, relative to the dimensions of the substrate and/or relative to one or more fiducial markers on the substrate, as in the first spatial dataset (*e.g.,* in each respective image in the set of images). Accordingly, in some such embodiments, the tissue sample is not moved, relative to the dimensions of the substrate and/or relative to one or more fiducial markers on the substrate, between the obtaining the set of images and the performing a spatial analysis to obtain the second spatial dataset.

In some embodiments, the plurality of nucleic acids comprises five or more nucleic acids, ten or more nucleic acids, fifty or more nucleic acids, one hundred or more nucleic acids, five hundred or more nucleic acids, 1000 or more nucleic acids, 2000 or more nucleic acids, between 2000 and 100,000 nucleic acids, between 100,000 and 1 x 10⁶ nucleic acids, more than 1 x 10⁶ nucleic acids, more than 2 x 10⁶ nucleic acids, more than 3 x 10⁶ nucleic acids, more than 4 x 10⁶ nucleic acids, more than 5 x 10⁶ nucleic acids, or more than 1 x 10⁷ nucleic acids.

Referring to Block 1024, in some embodiments, the plurality of nucleic acids comprises DNA, RNA, or a combination thereof.

Referring to Block 1026, in some embodiments, the plurality of nucleic acids comprises mRNA, microRNA, piRNA, nuclear RNA, or a combination thereof. In some embodiments, the plurality of nucleic acids comprises chromatin and/or VDJ repertoire sequencing. Other suitable examples of nucleic acids are further described herein (see, for example, "(A) General Definitions: Biological samples" and "(A) General Definitions: Nucleic acid and Nucleotide," above).

In some embodiments, the acquiring the second spatial dataset comprises obtaining a plurality of sequence reads, in electronic form, from the plurality of capture spots, where each respective capture spot in the plurality of capture spots includes a corresponding set of capture probes that directly or indirectly associates with one or more nucleic acids from the tissue sample, the plurality of sequence reads comprises sequence reads corresponding to all or portions of the plurality of nucleic acids, and each respective sequence read in the plurality of sequence reads includes a spatial barcode of the corresponding capture spot in the plurality of capture spots or a complement thereof. In some such embodiments, the acquiring the second spatial dataset further includes using all or a subset of the plurality of spatial barcodes to localize respective sequence reads in the plurality of sequence reads to corresponding capture spots in the plurality of capture spots, thereby dividing the plurality of sequence reads into a plurality of subsets of sequence reads, each respective subset of sequence reads corresponding to a different capture spot in the plurality of capture spots. In some embodiments, because of the number of sequence reads in the plurality of sequence reads and the number of capture spots in the plurality of capture spots, this localization requires 10,000 or more computations, 100,000 or more computations, 1 x 10⁶ or more computations, 2 x 10⁶ or more computations, 3 x 10⁶ or more computations, 4 x 10⁶ or more computations, 5 x 10⁶ or more computations, 6 x 10⁶ or more computations, more than 1 x 10⁷ computations, or more than 1 x 10⁸ computations. Moreover, in some embodiments, in order to provide real time results, these computations are performed in less than one hour, less than thirty minutes, less than 10 minutes, less than 5 minute, or less than one minute. Thus, in typical embodiments, the localization of respective sequence reads in the plurality of sequence reads to corresponding capture spots in the plurality of capture spots cannot be mentally performed.

For instance, referring to Block 1028, in some embodiments the acquiring the second spatial dataset comprises obtaining a plurality of sequence reads, in electronic form, from the plurality of capture spots, where each respective capture spot in the plurality of capture spots includes a corresponding set of 1000 or more capture probes, 2000 or more capture probes, 10,000 or more capture probes, 100,000 or capture more probes, 1 x 10⁶ or more capture probes, 2 x 10⁶ or more capture probes, 5 x 10⁶ capture probes, or 1 x 10⁷ or more capture probes that directly or indirectly associates with one or more nucleic acids from the tissue sample. In such embodiments the plurality of sequence reads comprises sequence reads corresponding to all or portions of the plurality of nucleic acids, the plurality of sequence reads comprises at least 10,000 sequence reads, and each respective sequence read in the plurality of sequence reads includes a spatial barcode of the corresponding capture spot in the plurality of capture spots or a complement thereof. In some such embodiments, the acquiring the second spatial dataset further includes using all or a subset of the plurality of spatial barcodes to localize respective sequence reads in the plurality of sequence reads to corresponding capture spots in the plurality of capture spots, thereby dividing the plurality of sequence reads into a plurality of subsets of sequence reads, each respective subset of sequence reads corresponding to a different capture spot in the plurality of capture spots.

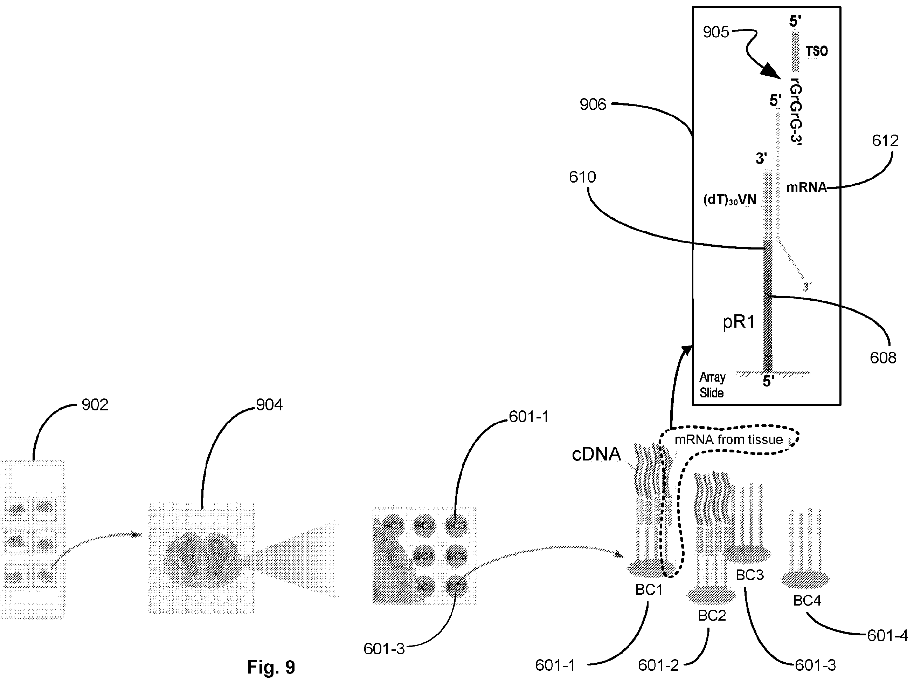

As illustrated in **FIG. 9**, in an example embodiment, a substrate (*e.g.,* array slide **902)** containing marked capture spot arrays **904** is used for placement and imaging of thin tissue sections of a biological sample. Each capture spot array **904** contains a plurality of capture spots **601** (*e.g.,* **601-1, 601-2, 601-3, 601-4)** comprising barcoded capture probes (*e.g.,* barcoded capture spots **1136).** A method of spatial analyte analysis is performed, in which the tissue section is permeabilized and a plurality of analytes for the biological sample (*e.g.,* mRNAs from the tissue) are contacted (*e.g.,* directly or indirectly) with the barcoded capture probes. As shown in more detail in panel **906,** a method of spatial analyte analysis includes performing a reverse transcription step (*e.g.,* using template switching oligo **905)** to generate nucleic acid molecules including, for a particular capture probe **602,** the spatial barcode **608** of the respective probe, a unique UMI identifier **610** of the respective probe, and a nucleic acid sequence corresponding to the respective analyte **612** contacted with the respective probe. The inclusion of the UMI **610** and the spatial barcode **608** in nucleic acid molecules and/or sequence reads corresponding to the contacted analyte ensures that the spatial location of the analyte within the tissue is captured at the level of capture spot **601** (*e.g.,* **1136)** resolution.

In some embodiments, the plurality of sequence reads comprises 10,000 or more sequence reads, 50,000 or more sequence reads, 100,000 or more sequence reads, or 1 x 10⁶ or more sequence reads. In some embodiments, the plurality of sequence reads comprises at least 100,000, at least 200,000, at least 500,000, at least 800,000, at least 1 x 10⁶, at least 2 x 10⁶, at least 5 x 10⁶, at least 8 x 10⁶, at least 1 x 10⁷, or at least 1 x 10⁸ sequence reads. In some embodiments, the plurality of sequence reads comprises no more than 1 x 10⁹, no more than 1 x 10⁸, no more than 1 x 10⁷, no more than 1 x 10⁶, no more than 500,000, no more than 200,000 or no more than 100,000 sequence reads. In some embodiments, the plurality of sequence reads comprises from 10,000 to 1 x 10⁷, from 100,000 to 1 x 10⁸, from 1 x 10⁵ to 1 x 10⁸, or from 10,000 to 500,000 sequence reads. In some embodiments, the plurality of sequence reads falls within another range starting no lower than 10,000 sequence reads and ending no higher than 1 x 10⁹ sequence reads.

Sequencing reads (also "reads" herein) may include a string of nucleic acid bases corresponding to a sequence of a nucleic acid molecule that has been sequenced. In general, sequence reads can be obtained from, for example, nucleic acid molecules such as deoxyribonucleic acid (DNA) or ribonucleic acid (RNA), including variants or derivatives thereof (e.g., single stranded DNA or DNA/RNA hybrids, and nucleic acid molecules with a nucleotide analog). A wide variety of different sequencing methods can be used to obtain the plurality of sequence reads. Sequencing can be performed by various commercial systems. Referring to Block 1030, the obtaining the plurality of sequence reads comprises high-throughput sequencing.

In some embodiments, the plurality of sequence reads is obtained using a sequencing device such as, without limitation, a sequencing system by Illumina^{®}, Pacific Biosciences (PacBio^{®}), Oxford Nanopore^{®}, or Life Technologies (Ion Torrent^{®}). Alternatively, or in addition, the plurality of sequence reads may be obtained by sequencing using nucleic acid amplification, polymerase chain reaction (PCR) (e.g., digital PCR and droplet digital PCR (ddPCR), quantitative PCR, real time PCR, multiplex PCR, PCR-based singleplex methods, and/or emulsion PCR), and/or isothermal amplification. Apparatuses suitable for obtaining the sequencing information of a spatial dataset are further described in, e.g., U.S. Patent Application No. 63/080547, entitled "Sample Handling Apparatus and Image Registration Methods," filed September 18, 2020; U.S. Patent Application No. 63/080,514, entitled "Sample Handling Apparatus and Fluid Delivery Methods," filed September 18, 2020; U.S. Patent Application No. 63/155,173, entitled "Sample Handling Apparatus and Image Registration Methods," filed March 1, 2021; and PCT Publication No. WO2020123320A2, entitled "Imaging system hardware".

Other examples of methods for sequencing include, but are not limited to, DNA hybridization methods (e.g., Southern blotting), restriction enzyme digestion methods, Sanger sequencing methods, next-generation sequencing methods (e.g., single-molecule real-time sequencing, nanopore sequencing, and Polony sequencing), ligation methods, and microarray methods. Additional examples of sequencing methods that can be used include targeted sequencing, single molecule real-time sequencing, exon sequencing, electron microscopy-based sequencing, panel sequencing, transistor-mediated sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, co-amplification at lower denaturation temperature-PCR (COLD-PCR), sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD^{™} sequencing, MS-PET sequencing, and any combinations thereof.

In some embodiments, each capture probe in a respective capture spot includes a poly-A sequence or a poly-T sequence and a unique spatial barcode that characterizes the respective capture spot. In some embodiments, each capture probe in a respective capture spot includes the same spatial barcode from the plurality of spatial barcodes. In some embodiments, each capture probe in a respective capture spot includes a different spatial barcode from the plurality of spatial barcodes. In some embodiments, each capture probe in the corresponding set of capture probes of a respective capture spot includes the same spatial barcode from the plurality of spatial barcodes. As described above, in some embodiments, the unique spatial barcode encodes a unique predetermined value selected from the set {1, ..., 1024}, {1, ..., 4096}, {1, ..., 16384}, {1, ..., 65536}, {1, ..., 262144}, {1, ..., 1048576}, {1, ..., 4194304}, {1, ..., 16777216}, {1, ..., 67108864}, or {1, ..., 1 x 1012}.

In some embodiments, the corresponding representation of a number of molecules of the respective nucleic acid originating from the tissue sample while the tissue sample is overlaid on the substrate in the first orientation and localized to the respective capture spot by a corresponding spatial barcode, in the plurality of barcodes, associated with the respective capture spot is a count of a number of unique sequence reads in the plurality of sequence reads that have the spatial barcode associated with the respective capture spot. In some embodiments, the corresponding representation of a number of molecules of the respective nucleic acid is a normalized count of the number of unique sequence reads in the plurality of sequence reads that have the spatial barcode associated with the respective capture spot. In some embodiments, the corresponding representation of a number of molecules of the respective nucleic acid is a UMI count.

Additional methods for obtaining spatial analyte data that are contemplated for use in the present disclosure, including but not limited to sequencing, sequence reads, and methods of processing and analyzing the same, are further described in U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and U.S. Patent Publication No. US2023-0167495A1, entitled "Systems and Methods for Identifying Regions of Aneuploidy in a Tissue".

### Registration.

As described above, in some embodiments, the method further comprises performing a registration to overlay each respective image in the set of images (e.g., the first spatial dataset) onto a frame of reference for the plurality of capture spots on the substrate. In some embodiments, the method further comprises using the barcoded nucleic acid spatial analyte data to obtain a frame of reference for the spatial analyte data from the tissue sample (e.g., the second spatial dataset), where the frame of reference is known with respect to the capture spots on the substrate.

Thus, in some such embodiments, the image data for the tissue sample (e.g., the first spatial dataset) can be overlayed onto spatial analyte data for the plurality of nucleic acid analytes of the tissue sample (e.g., the second spatial dataset) using the frame of reference for the plurality of capture spots on the substrate.

In some embodiments, the registration is fiducial registration.

Generally, fiducial registration (e.g., fiducial alignment) can be used to determine a position of an element in a spatial dataset (e.g., an array of capture spots) with respect to the coordinate system of an image of the tissue sample (e.g., in the set of images), thus obtaining a frame of reference of the spatial dataset that is known with respect to the image.

In some embodiments, the fiducial registration is performed at a computing system, such as a system **1100.** For instance, in some embodiments, the computing system determines one or more spatial fiducials located on the substrate. In some embodiments, the one or more spatial fiducials are determined using computer vision and/or image processing functionality provided in an image processing pipeline configured within a sample handling apparatus. For example, as described above, in some embodiments, an image **1122** is aligned to the plurality of capture spots **1136** on a substrate by a procedure that comprises analyzing the array of pixel values to identify one or more spatial fiducials **1130** of the respective image. In some embodiments, the one or more spatial fiducials include a high contrast or uniquely shaped mark to aid in determination of the spatial fiducial via the computer vision and/or image processing functionality provided in an image processing pipeline, or other methods.

In some embodiments, the one or more spatial fiducials **1130** of the respective image **1122** are aligned with a corresponding one or more reference spatial fiducials using an alignment algorithm to obtain a transformation between the one or more spatial fiducials **1130** of the respective image **1122** and the corresponding one or more reference spatial fiducials. The transformation and a coordinate system corresponding to the one or more reference spatial fiducials are then used to locate a corresponding position in the respective image of each capture spot in the plurality of capture spots.

**FIG. 14** illustrates an image **1122** of a tissue **1204** on a substrate, where the image includes a plurality of spatial fiducials, in accordance with some embodiments. The spatial fiducials are arranged along the external border of the substrate, surrounding a capture spot array and the tissue. In some such embodiments, the spatial fiducials comprise patterned spots, and the patterned spots indicate the edges and corners of the capture spot array. In some such embodiments, a different pattern of spatial fiducials is provided at each corner, allowing the image to be correlated with spatial information using any orientation (e.g., rotated and/or mirror image). **FIG. 7C** further illustrates an image of a plurality of fiducial markers on a substrate, where the fiducial markers are arranged along the external border of a sample area, surrounding a capture spot array upon which a tissue is placed. Accordingly, in some embodiments, the frame of reference of the first spatial dataset is known with respect to the capture spot array, based on the one or more spatial fiducials of one or more images in the set of images.

In some embodiments, one or more imaging algorithms (e.g., an image segmentation algorithm) can be configured to determine where a biological sample, such as tissue, has been placed, thus generating a respective pattern for the biological sample, and aligning the respective pattern with the one or more spatial fiducials. In some embodiments, tissue detection (e.g., image segmentation) is used to identify which capture spots, and therefore which capture probes, in the spatial dataset will be used for spatial analyte analysis. In some embodiments, fiducial alignment is performed to determine where in the image an individual capture spot resides, since each user may set a slightly different field of view when imaging the sample area.

Methods and embodiments for fiducial registration contemplated for use in the present disclosure are further described in U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; U.S. Patent Application No. 63/080547, entitled "Sample Handling Apparatus and Image Registration Methods," filed September 18, 2020; U.S. Patent Application No. 63/080,514, entitled "Sample Handling Apparatus and Fluid Delivery Methods," filed September 18, 2020; U.S. Patent Application No. 63/155,173, entitled "Sample Handling Apparatus and Image Registration Methods," filed March 1, 2021; PCT Publication No. WO2020123320A2, entitled "Imaging system hardware"; and PCT Publication No. WO2023044071, entitled "Systems and Methods for Image Registration or Alignment".

Additional methods and embodiments for image segmentation are contemplated for use in the present disclosure, as further described in U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; and PCT Publication No. WO2023044071, entitled "Systems and Methods for Image Registration or Alignment".

### Displaying spatial datasets.

Referring to Block 1032, the method further includes spatially displaying all or a portion of the first spatial dataset 1134-1 and a corresponding all or a portion of the second spatial dataset 1134-2 co-registered to each other by the plurality of spatial barcodes 1140 on a display.

In some embodiments, the display includes visualization tools that can be configured to provide the first spatial dataset, the second spatial dataset, and/or any features or overlays thereof as described herein, in one or more visual formats. In some embodiments, the first spatial dataset, the second spatial dataset, and/or any features or overlays thereof as described herein, are provided in a GUI of a display of a sample handling apparatus. In some embodiments, the visualization tools can be configured on a remote computing device that is communicatively coupled to the sample handling apparatus, such that the first spatial dataset, the second spatial dataset, and/or any features or overlays thereof as described herein, can be visualized and/or manipulated on the remote computing device.

In some embodiments, the visualization tools are configured to provide a user input system and user interface, such as a desktop application that provides interactive visualization functionality to perform any of the workflows or processes described herein. In some embodiments, the visualization tools include a browser that can be configured to enable users to evaluate and interact with different views of the spatial analyte data (e.g., the first spatial dataset and/or the second spatial dataset) to quickly gain insights into the underlying biology of the samples being analyzed. The browser can be configured to evaluate significant analytes (e.g., genes), characterize and refine clusters of data, and to perform differential analysis (e.g., expression analysis) within the spatial context of an image and/or a spatial dataset.

In some embodiments, the visualization tools are configured to read from and write to files generated by a spatial analyte analysis and/or image analysis workflow. The files can be configured to include tiled and untiled versions of images and analyte data, including but not limited to, analyte expression data for all barcoded locations on a substrate or slide, alignment data associated with alignment of a sample or portions of the sample and the barcoded locations of an array, and analyte expression-based clustering information for the barcoded locations. The analyte expression-based clustering information can include t-Distributed Stochastic Neighbor Embedding (t-SNE) and Uniform Manifold Approximation and Projection (UMAP) projections.

As described above, in some embodiments, the first spatial dataset includes, for each respective image in the set of images, for each respective capture spot in the plurality of capture spots, a corresponding value that indicates the measured intensity of the respective capture spot. Accordingly, as described below in Example 1, in an example embodiment, the first spatial dataset that includes, for each of the three excitation wavelengths (e.g., channels) used to obtain each respective image in the set of images, a corresponding measured intensity (e.g., value) that is assigned to each capture spot. The measured intensity (e.g., value) of each capture spot can be spatially displayed as a relative brightness or a color in a range of colors assigned to all of the capture spots in the array, such as in a spatial heatmap.

Similarly, in some embodiments, the second spatial dataset can be visualized where the representation of the number of molecules of each respective nucleic acid in the plurality of nucleic acids localized to each respective capture spot in the plurality of capture spots by a corresponding spatial barcode (e.g., each UMI count) is a value that is assigned to each capture spot. The value can be spatially displayed as a relative brightness or a color in a range of colors assigned to all of the capture spots in the array, such as in a spatial heatmap.

Other representations of visualization are possible, for instance, using dimension reduction techniques (e.g., latent component analysis and/or clustering). For example, in some embodiments, the display includes a presentation of the first spatial dataset and/or the second spatial dataset organized with respect to clusters. In some embodiments, the presentation can provide representative clusters as violin plots, although a number of other nonlimiting plot types can be envisioned. In some embodiments, the dimension reduction technique is t-SNE and/or UMAP. In some embodiments, a dimension reduction technique is used to reduce the principal component values of the first and/or second spatial datasets to a corresponding plurality of two-dimensional datapoints. For example, in some implementations, the dimension reduction technique is Sammon mapping, curvilinear components analysis, stochastic neighbor embedding, Isomap, maximum variance unfolding, locally linear embedding, and/or Laplacian Eigenmaps. These techniques are described in, e.g., van der Maaten and Hinton, 2008, "Visualizing High-Dimensional Data Using t-SNE," Journal of Machine Learning Research 9, 2579-2605. In some embodiments, the user has the option to select the dimension reduction technique. In some embodiments, the user has the option to select t-SNE, UMAP, Sammon mapping, curvilinear components analysis, stochastic neighbor embedding, Isomap, maximum variance unfolding, locally linear embedding, or Laplacian Eigenmaps.

Additional methods of combining and displaying two or more spatial datasets are possible, as described in, e.g., PCT Publication No. WO2021163630A1, entitled "Systems and Methods for Joint Interactive Visualization of Gene Expression and DNA Chromatin Accessibility"; and U.S. Patent Publication No. US2021-0381056A1, entitled, "Systems and Methods for Joint Interactive Visualization of Gene Expression and DNA Chromatin Accessibility".

Referring to Block 1034, in some embodiments, the spatially displaying is interactive.

For example, in some embodiments, the display includes image setting functionality configured to adjust or configure settings associated with any of the workflows or processes described herein, including but not limited to fiducial display, scale display, rotation, and/or resetting the image data. In some embodiments, the display includes one or more image manipulation tools, such as a pointer to select data or menu items, a lasso to select data, and a pen to annotate or mark data. The analyte data can be provided in a primary viewing panel.

Referring to Block 1036, in some embodiments, the method further includes, responsive to a user interaction, performing an action on the all or the portion of the first spatial dataset and the corresponding all or the portion of the second spatial dataset co-registered to each other selected from the group consisting of: (i) zooming, (ii) panning, and (iii) adjusting an opacity of all or a portion of the first spatial dataset or the corresponding all or a portion of the second spatial dataset.

In some embodiments, both the first and second spatial datasets are displayed as an overlay. For instance, in some embodiments, the method further includes visualizing, on a display, a visual representation of the first spatial dataset and/or a visual representation of the second spatial dataset overlayed onto one or more images in the set of images. In some embodiments, the method further comprises visualizing, on a display, a visual representation of the first spatial dataset overlayed onto a visual representation of the second spatial dataset, both of which are further overlayed onto one or more images in the set of images.

In some embodiments, the display includes secondary viewing panels. The secondary viewing panels can provide one or more projections of the spatial analyte data (e.g., the first spatial dataset and/or the second spatial dataset) provided in the primary viewing panel. For example, the secondary viewing panel can provide a spatial projection of the analyte data so that a user can interact with the spatial opacity and magnification settings of the data. The secondary viewing panel can provide an additional projection of the spatial analyte data (e.g., the first spatial dataset and/or the second spatial dataset) other than or in addition to that shown on the primary viewing panel.

The primary viewing panel and secondary viewing panels can each individually be configured with image manipulation tools including, but not limited to, image resize functionality, image cropping functionality, image zoom functionality, image capture functionality, tile view functionality, list view functionality, or the like.

In some embodiments, the first and second spatial datasets are displayed as one or more linked windows, such that any manipulation of spatial data from the first spatial dataset in a first window modifies the spatial data from the second spatial dataset in a second window. For instance, to allow users to see common characteristics or compare the first and second spatial datasets at once, one aspect of the present disclosure makes use of novel linked windows.

In some such embodiments, a user can add multiple instances of display of the first spatial dataset and/or the second spatial dataset. In some embodiments, each instance of display is a different projection of the first spatial dataset and/or the second spatial dataset (e.g., any of the projections and/or visual representations disclosed above). In some embodiments, each respective projection is displayed as a smaller window within a larger user interface (e.g., a main window). In some such implementations, actions taken in the main window, such as changing the active category, or showing expression or accessibility for a particular feature, will propagate to the linked windows. In some embodiments, a user can toggle between a plurality of instances of display (e.g., linked windows and/or projections). Advantageously, using linked windows avoids having to jump back and forth, making the investigation and analysis fluid and intuitive.

In some embodiments, the first and second spatial datasets are linked such that display of all or a portion of the first spatial dataset is dependent on selection of all or a portion of the second spatial dataset. In some embodiments, the first and second spatial datasets are linked such that display of all or a portion of the second spatial dataset is dependent on selection of all or a portion of the first spatial dataset.

In some embodiments, linked windows can also be used to illustrate the quantification of any combination of analytes, arranged in two-dimensional space using dimension reduction algorithms such as t-SNE or UMAP, including any combination of nucleic acids (e.g., DNA and/or RNA), intracellular proteins (e.g., transcription factors), cell methylation status, various forms of accessible chromatin (e.g., ATAC-seq, DNase-seq, and/or MNase-seq), metabolites, barcoded labelling agents (e.g., the oligonucleotide tagged antibodies), V(D)J sequences of immune cell receptors (e.g., T-cell receptor), perturbation agents (e.g., a CRISPR crRNA/sgRNA, TALEN, zinc finger nucleases, and/or antisense oligonucleotides.

Visualization and applications of spatial analyte data, including spatial analyte data for one or more spatial datasets overlayed on biological sample images, that are contemplated for use in the present disclosure are further described in U.S. Patent No. 11,501,440; U.S. Patent Publication No. US20210150707A1, entitled "SYSTEMS AND METHODS FOR TISSUE CLASSIFICATION"; U.S. Patent No. 11,514,575; U.S. Patent Publication No. US2021-0155982A1, entitled "Spatial Analysis of Analytes"; U.S. Patent Application No. 63/080547, entitled "Sample Handling Apparatus and Image Registration Methods," filed September 18, 2020; U.S. Patent Application No. 63/080,514, entitled "Sample Handling Apparatus and Fluid Delivery Methods," filed September 18, 2020; U.S. Patent Application No. 63/155,173, entitled "Sample Handling Apparatus and Image Registration Methods," filed March 1, 2021; PCT Publication No. WO2020123320A2, entitled "Imaging system hardware," PCT Publication No. WO2021163630A1, entitled "Systems and Methods for Joint Interactive Visualization of Gene Expression and DNA Chromatin Accessibility,"; U.S. Patent Publication No. US2021-0381056A1, entitled, "Systems and Methods for Joint Interactive Visualization of Gene Expression and DNA Chromatin Accessibility"; and PCT Publication No. WO2023/044071, entitled "Systems and Methods for Image Registration or Alignment".

### Example applications of spatial analyte analysis.

In some embodiments, the spatially displaying comprises displaying the corresponding all or a portion of the second spatial dataset overlaid onto one or more representations of all or a portion of the first spatial dataset, corresponding to each respective detectable marker in the selected subset of detectable markers. In some such embodiments, the spatially displaying comprises displaying the corresponding all or a portion of the second spatial dataset overlaid onto a stack of images (and/or representations of the first spatial dataset corresponding to each image in the stack of images), where each respective image in the stack of images corresponds to a respective detectable marker in the selected subset of detectable markers. In some such implementations, the spatially displaying comprises displaying a multi-"page" image, in which each respective "page" corresponds to a different excitation wavelength and/or a different spatial dataset.

As described above, in some implementations, a user can toggle through different "pages" of the image stack to view an overlay of any one of the various representations of the tissue sample with any other representation (e.g., obtained from barcoded spatial analyte data of the first spatial dataset, barcoded spatial analyte data of the second spatial dataset, and/or the set of images). Alternatively, all or a subset of the representations of the tissue sample (e.g., obtained from barcoded spatial analyte data of the first spatial dataset, barcoded spatial analyte data of the second spatial dataset, and/or the set of images) can be superimposed simultaneously. Analyte data can thus be viewed spatially by localizing analyte abundance levels obtained from the first spatial dataset, the second spatial dataset, and/or a respective image within a shared frame of reference for the tissue sample.

In some embodiments, co-registration of the first spatial dataset and the second spatial dataset is used to associate one or more first species of analytes (e.g., polynucleotides, etc.) from the tissue sample with one or more second species of analytes (e.g., polypeptides, etc.), and/or with one or more physical properties, of the tissue sample. For example, the one or more first species of analytes can be associated with locations of the one or more second species of analytes in the tissue sample. Such information (e.g., genetic information such as DNA sequence information, transcriptome information such as sequences of transcripts, or both) can be used in association with other spatial information (e.g., proteomic information obtained from imaging). Additionally, a cell surface protein of a cell can be associated with one or more physical properties of the cell (e.g., a shape, size, activity, or a type of the cell). In some embodiments, the one or more physical properties can be characterized by imaging the cell. For instance, in some embodiments, the first spatial dataset is obtained using immunofluorescence imaging for a protein of interest, the second spatial dataset comprises quantitative nucleic acid spatial data, and the co-registration of the first spatial dataset and the second spatial dataset is used to visualize and compare colocalization of nucleic acid quantification with immunofluorescence staining of the protein of interest.

In some embodiments, the spatially displaying all or a portion of the first spatial dataset 1134-1 and a corresponding all or a portion of the second spatial dataset 1134-2 co-registered to each other by the plurality of spatial barcodes 1140 comprises viewing on the display only the subset of nucleic acid quantification data (e.g., UMI counts) that are co-expressed (e.g., overlaid) on measured intensities of one or more detectable markers in the set of detectable markers of interest (e.g., protein abundance and/or localization).

In some embodiments, the method includes selecting a detectable marker of interest (e.g., corresponding to a respective excitation wavelength in a plurality of excitation wavelengths) and filtering the nucleic acid data to view a corresponding subset of nucleic acid molecules that are co-expressed with the selected detectable marker. In some such embodiments, the detectable marker is specific for an analyte and/or cell component of interest (e.g., a protein) in a particular tissue sample (e.g., a cell type such as a cancer cell and/or a sample that is undergoing a particular treatment or biological process), and the co-registration is used to determine the extent to which, if any, the analyte of interest is co-expressed with one or more nucleic acids of interest (e.g., genes).

In some embodiments, the nucleic acid quantification data can be filtered such that only the subset of the second spatial dataset that is co-expressed with a selected subset of detectable markers is displayed. In some embodiments, the selected subset of detectable markers includes at least 1, at least 2, at least 3, at least 4, at least 5, at least 6, at least 7, at least 8, at least 9, or at least 10 detectable markers. In some embodiments, the selected subset of detectable markers includes no more than 20, no more than 10, no more than 8, no more than 5, or no more than 3 detectable markers. In some embodiments, the selected subset of detectable markers includes from 1 to 4, from 2 to 5, from 2 to 9, from 3 to 8, from 4 to 12, or from 10 to 20 detectable markers. In some embodiments, the selected subset of detectable markers falls within another range starting no lower than 1 detectable marker and ending no higher than 20 detectable markers.

In some embodiments, the spatially displaying all or a portion of the first spatial dataset 1134-1 and a corresponding all or a portion of the second spatial dataset 1134-2 co-registered to each other by the plurality of spatial barcodes 1140 comprises viewing on the display only the measured intensities of detectable markers (e.g., protein abundance and/or localization) that are co-expressed (e.g., overlaid) on nucleic acid quantification data (e.g., UMI counts) for nucleic acid analytes (e.g., target genes) of interest.

In some such embodiments, the method includes selecting one or more nucleic acid analytes of interest (e.g., genes) and filtering the measured intensity data to view only the corresponding subset of measured intensity data (e.g., immunofluorescence corresponding to one or more detectable markers) that are co-expressed with the selected nucleic acid analytes. In some embodiments, a respective nucleic acid analyte of interest is a biomarker (e.g., a gene) having known expression in a particular sample type (e.g., a cell type such as a cancer cell and/or a sample that is undergoing a particular treatment or biological process), the co-registration is used to determine the extent to which, if any, the nucleic acid analyte is co-expressed with one or more analytes that are measurable by imaging and/or staining (e.g., a protein that is characterized by immunofluorescence staining).

In some embodiments, the measured intensity data can be filtered such that only the subset of the first spatial dataset that is co-expressed with a selected one or more nucleic acid analytes of interest is displayed. In some embodiments, the one or more nucleic acid analytes of interest includes at least 2, at least 5, at least 10, at least 20, at least 50, at least 80, at least 100, at least 200, at least 300, or at least 500 nucleic acid analytes. In some embodiments, the one or more nucleic acid analytes of interest includes no more than 1000, no more than 500, no more than 100, no more than 50, or no more than 10 nucleic acid analytes. In some embodiments, the one or more nucleic acid analytes of interest includes from 10 to 40, from 20 to 50, from 2 to 90, from 30 to 80, from 50 to 200, or from 2 to 20 nucleic acid analytes. In some embodiments, the one or more nucleic acid analytes of interest falls within another range starting no lower than 2 nucleic acid analytes and ending no higher than 20 nucleic acid analytes.

In some embodiments, the selection of a detectable marker of interest comprises determining whether the detectable marker satisfies a minimum intensity at one or more capture spots in the plurality of capture spots. Accordingly, referring to Block 1038, in some embodiments, a first detectable marker in the set of detectable markers is indicative of a particular cell type, and the method further comprises removing from the display those portions of the first spatial dataset and the second spatial dataset that are not associated with capture spots in the plurality of capture spots that exhibit at least a threshold amount of the first detectable marker. Referring to Block 1040, in some embodiments, the first detectable marker comprises a dye-labeled antibody to a protein that is expressed in or on the particular cell type.

In some embodiments, the method includes selecting a detectable marker of interest (e.g., based on the measured intensity of the respective detectable marker corresponding to a respective excitation wavelength in a plurality of excitation wavelengths), filtering the nucleic acid data to view a corresponding subset of nucleic acid molecules that are co-expressed with the selected detectable marker, and further removing from the display those portions of the first spatial dataset and the second spatial dataset that are not associated with capture spots in the plurality of capture spots that exhibit at least a threshold amount of the nucleic acid quantification data (e.g., genes) co-expressed with the selected detectable marker. Thus, in some such embodiments, the spatially displaying comprises filtering on based on minimum threshold amounts of both the measured intensities of the detectable marker and the nucleic acid quantification data.

In some embodiments, the selection of a nucleic acid of interest comprises determining whether the nucleic acid satisfies a minimum intensity at one or more capture spots in the plurality of capture spots. Accordingly, in some embodiments, a first nucleic acid in the plurality of nucleic acids is indicative of a particular cell type, and the method further comprises removing from the display those portions of the first spatial dataset and the second spatial dataset that are not associated with capture spots in the plurality of capture spots that exhibit at least a threshold amount of the first nucleic acid.

In some embodiments, the method includes selecting one or more nucleic acid analytes of interest (e.g., genes), filtering the measured intensity data to view only the corresponding subset of measured intensity data (e.g., immunofluorescence corresponding to one or more detectable markers) that are co-expressed with the selected nucleic acid analytes, and further removing from the display those portions of the first spatial dataset and the second spatial dataset that are not associated with capture spots in the plurality of capture spots that exhibit at least a threshold amount of the measured intensity data (e.g., detectable markers) co-expressed with the selected nucleic acid. Thus, in some such embodiments, the spatially displaying comprises filtering based on minimum threshold amounts of both the measured intensities of the detectable marker and the nucleic acid quantification data.

Referring to Block 1042, in some embodiments, the method further includes displaying of all or the portion of the first spatial dataset and the corresponding all or the portion of the second spatial dataset co-registered to each other to characterize a biological condition in a subject. This is useful because, as described above, in some embodiments, tissue samples can be characterized by the presence, absence, relative abundance, and/or expression profiles of one or more analytes. Such information can be used to assess a cell type, a disease condition, a treatment, and/or a biological process of the tissue sample. See, for example, the section entitled "Samples and substrates," above. Advantageously, co-expression can be informative for characterizing biological conditions through specific combinations of analyte abundance (e.g., expression of one or more genes and one or more proteins), and/or where the expression of a first analyte is validated by the abundance of a second analyte (e.g., low levels of mRNA that can be validated by presence of the protein product). In some embodiments, profiles of individual cells or populations of cells in the first and/or the second spatial dataset, combined or individually, can be compared to profiles from other cells, e.g., "normal" cells, to identify variations in analytes, which can provide diagnostically relevant information. In some embodiments, these profiles can be useful in the diagnosis of a variety of disorders that are characterized by variations in cell surface receptors, such as cancer and other disorders.

In some embodiments the display of all or a portion of the first spatial dataset and the corresponding all or a portion of the second spatial dataset co-registered to each other provides an extent to which (i) one or more detectable markers in the set of detectable markers and (ii) the nucleic acid quantification data for respective nucleic acids in the plurality of nucleic acids are colocalized in the tissue sample.

In some embodiments the display of all or a portion of the first spatial dataset and the corresponding all or a portion of the second spatial dataset co-registered to each other provides an extent to which (i) one or more detectable markers in the set of detectable markers and (ii) the nucleic acid quantification data for respective nucleic acids in the plurality of nucleic acids colocalize to a common component of a plurality of cells within the tissue sample. In some such embodiments, the common component is a cell membrane, cytoplasm, a nucleus, a nuclear envelope, a nucleolus, an endoplasmic reticulum, a ribosome, a Golgi apparatus, a mitochondria, a lysosome, a vacuole, a peroxisome, a cytoskeleton, and/or a centriole.

In embodiments where the detectable markers are specific to expression of specific proteins, the colocalization of the one or more detectable markers with the nucleic acid quantification data in any of the forgoing embodiments is used in some such embodiments to confirm (through both gene expression and resulting protein gene product abundance in precise locations in the tissue) the precise tissue locations where such specific proteins are synthesized. Such information is then used to further understanding of the roles and functions of proteins within different tissues.

In embodiments where the detectable markers are specific to expression of specific proteins, the extent to which (i) one or more detectable markers in the set of detectable markers and (ii) the nucleic acid quantification data for respective nucleic acids in the plurality of nucleic acids colocalize to a common portion of a tissue in any of the forgoing embodiments can be used, for example, to identify a function of a protein, associate a protein with a particular biological pathway, validate gene expression data, validate the accuracy of gene expression data obtained through techniques like RNA sequencing, elucidate a regulatory process that controls gene expression, elucidate whether gene expression and protein abundance patterns are altered in diseased cells or tissues, provide context for disease research by helping identify whether gene expression and protein abundance patterns are altered in diseased cells or tissues, and/or discover new biomarkers for various diseases.

In embodiments where the detectable markers are specific to expression of specific proteins, the display of all or a portion of the first spatial dataset and the corresponding all or a portion of the second spatial dataset co-registered to each other provides a measure of stability and turnover rates of proteins. For instance, in such colocalization analysis, the nucleic acid quantification data provides expression levels of the gene or genes while a detectable marker in the set of detectable markers is for the protein gene products of the gene or genes. In some such embodiments, such colocalization compares colocalized gene expression (of the gene, nucleic acid analyte) with protein abundance (non-nucleic acid analyte, protein gene product of the gene) to provide insights into the stability and turnover rates of the protein gene products of the genes. For instance, certain proteins might be produced at high mRNA levels, but their abundance might be low due to rapid degradation. Understanding protein turnover, through such colocalization studies, is important for understanding cellular homeostasis and the dynamics of protein regulation.

In some embodiments, the display of all or a portion of the first spatial dataset and the corresponding all or a portion of the second spatial dataset co-registered to each other is used in drug discovery and development. For instance, through determining which genes (localized by the second spatial dataset) are colocalized with disease-related proteins and/or diseased portions of a tissue (localized by the first spatial dataset), potential drug targets and design therapies that modulate the expression or function of these genes are developed.

Thus, the colocalization provided by the present disclosure enhances the understanding of gene regulation, cellular processes, and disease mechanisms.

According to the claimed invention, referring to Block 1044, a first detectable marker in the set of detectable markers is indicative of a presence of a cell nucleus, and the method further comprises using a measured intensity of the first detectable marker in each capture spot in the plurality of capture spots as indicated within the first spatial dataset to determine a corresponding estimate of a number of cells in each respective capture spot in the plurality of capture spots. For instance, in some such embodiments, the first detectable marker is an intercalating, non-specific nucleic acid dye (e.g., DAPI) that is used to estimate cell count based on the detection of cell nuclei in the tissue sample. Referring to Block 1046, in some embodiments, the method further includes using the corresponding estimate of a number of cells in each respective capture spot in the plurality of capture spots to exclude, in a clustering or dimension reduction of the second spatial dataset, nucleic acid quantification data from capture spots in the plurality of capture spots that fail to satisfy a cell number threshold. For instance, in some implementations, an estimate of cell count can be used to normalize nucleic acid quantification data from the second spatial dataset. This allows for more accurate quantification of unique spatially barcoded nucleic acid molecules associated with capture spots, particularly in cases where a first capture spot is contacted with a greater number of cells than a second capture spot. In such cases, higher nucleic acid molecule counts (e.g., UMI counts) resulting from differences in cell count per capture spot can be misinterpreted as higher nucleic acid expression per cell.

In some embodiments, a first detectable marker in the set of detectable markers is indicative of a presence of a cell nucleus, and the method further includes using the indicated cell nuclei to perform cell segmentation. In some such embodiments, the cell segmentation is performed by a procedure comprising estimating a radius of length n around each respective nucleus and segmenting, for each circular closed-form shape of radius n around the respective nucleus, a corresponding cell. In some embodiments, the radius of length n is from 0.3 to 5 microns. In some embodiments, the radius of length n is from 0.5 to 3 microns.

In some embodiments, the cell segmentation further includes validating the estimation of the circular closed-form shape of each respective cell with the measured intensity values of a second detectable marker. For example, in some embodiments, a second detectable marker in the set of detectable markers is indicative of a presence of a cell membrane, and the cell segmentation further comprises using the indication of the cell membrane to validate the estimated radius n around each respective nucleus indicated by the first detectable marker.

Accordingly, referring to Block 1048, in some embodiments, a first detectable marker in the set of detectable markers is indicative of a presence of cell nucleus, a second detectable marker in the set of detectable markers is indicative of a presence of cell membrane, and the method further comprises (i) using a measured intensity of the first detectable marker in each capture spot in the plurality of capture spots as indicated within the first spatial dataset to determine a corresponding estimate of a number of cells in each respective capture spot in the plurality of capture spots, and (ii) using a pattern of abundance of the second detectable marker in each capture spot in the plurality of capture spots as indicated within the first spatial dataset to validate the corresponding estimate of the number of cells in each respective capture spot in the plurality of capture spots. In some such embodiments, the first detectable marker is DAPI, and the second detectable marker is a lipid dye.

In some embodiments, a first detectable marker in the set of detectable markers is indicative of a presence of a cell membrane, and the method further includes using the indicated cell membranes to perform cell segmentation.

### Additional embodiments.

Another aspect of the present disclosure provides a computer system comprising one or more processors, memory, and one or more programs, where the one or more programs are stored in the memory and configured to be executed by the one or more processors. The one or more programs include instructions for analyzing a tissue sample by a method comprising obtaining a set of images of the tissue sample while the tissue sample is overlaid on a substrate in a first orientation, where the substrate comprises a plurality of capture spots (e.g., at least 1000 capture spots), and each respective image in the set of images is an emission image of the tissue sample upon excitation of the tissue sample at a corresponding excitation wavelength of a corresponding detectable marker, in a set of one or more detectable markers, associated with the respective image. The method further includes acquiring a first spatial dataset comprising, for each respective capture spot in the plurality of capture spots, for each respective detectable marker in the set of detectable markers, a measured intensity of the respective capture spot, in the corresponding image in the set of images, indexed by a spatial barcode in a plurality of spatial barcodes that represents the respective capture spot. The method further includes acquiring a second spatial dataset comprising nucleic acid quantification data comprising, for each respective capture spot in the plurality of capture spots, for each respective nucleic acid in a plurality of nucleic acids, a corresponding representation of a number of molecules of the respective nucleic acid originating from the tissue sample while the tissue sample is overlaid on a substrate in the first orientation and localized to the respective capture spot by a corresponding spatial barcode, in the plurality of spatial barcodes, associated with the respective capture spot. The method further comprises spatially displaying all or a portion of the first spatial dataset and a corresponding all or a portion of the second spatial dataset co-registered to each other by the plurality of spatial barcodes on a display.

Another aspect of the present disclosure provides a computer readable storage medium storing one or more programs, the one or more programs comprising instructions, which when executed by an electronic device with one or more processors and a memory cause the electronic device to perform a method for analyzing a tissue sample, comprising obtaining a set of images of the tissue sample while the tissue sample is overlaid on a substrate in a first orientation, where the substrate comprises a plurality of capture spots (e.g., at least 1000 capture spots), and each respective image in the set of images is an emission image of the tissue sample upon excitation of the tissue sample at a corresponding excitation wavelength of a corresponding detectable marker, in a set of detectable markers, associated with the respective image. The method further includes acquiring a first spatial dataset comprising, for each respective capture spot in the plurality of capture spots, for each respective detectable marker in the set of detectable markers, a measured intensity of the respective capture spot, in the corresponding image in the set of images, indexed by a spatial barcode in the plurality of spatial barcodes that represents the respective capture spot. The method further includes acquiring a second spatial dataset comprising nucleic acid quantification data comprising, for each respective capture spot in the plurality of capture spots, for each respective nucleic acid in a plurality of nucleic acids, a corresponding representation of a number of molecules of the respective nucleic acid originating from the tissue sample while the tissue sample is overlaid on a substrate in the first orientation and localized to the respective capture spot by a corresponding spatial barcode, in the plurality of spatial barcodes, associated with the respective capture spot. The method further comprises spatially displaying all or a portion of the first spatial dataset and a corresponding all or a portion of the second spatial dataset co-registered to each other by the plurality of spatial barcodes on a display.

Another aspect of the present disclosure provides a computer system comprising one or more processors, memory, and one or more programs, where the one or more programs are stored in the memory and configured to be executed by the one or more processors. The one or more programs include instructions for any of the methods, workflows, processes, or embodiments disclosed herein, and/or any substitutions, modifications, additions, deletions, and/or combinations thereof, as will be apparent to one skilled in the art.

Another aspect of the present disclosure provides a computer readable storage medium storing one or more programs, the one or more programs comprising instructions, which when executed by an electronic device with one or more processors and a memory cause the electronic device to perform any of the methods, workflows, processes, or embodiments disclosed herein, and/or any substitutions, modifications, additions, deletions, and/or combinations thereof, as will be apparent to one skilled in the art.

### Examples

### Example 1: Determining Fluorescence Intensity of Capture Spots.

An example tissue analysis was performed in which immunofluorescence intensity was estimated at a plurality of capture spots in an array. A set of images were obtained of an adult mouse brain coronal tissue section on a substrate, where the tissue section was stained with a first detectable marker specific for a first analyte (neuronal protein NeuN) and a second detectable marker specific for a second analyte (double stranded DNA). FIGS. 7A-C illustrate each image in the set of images, including a first emission image obtained at a first excitation wavelength for the first detectable marker (FITC; FIG. 7A), a second emission image obtained at a second excitation wavelength for the second detectable marker (DAPI; FIG. 7B), and a third emission image obtained at a third excitation wavelength for a third detectable marker (TRITC; FIG. 7C). As illustrated in FIG. 7C, the substrate further comprised a plurality of fiducial markers, which imaged at the third excitation wavelength. Each image in the set of images further included a plurality of pixels, each respective pixel having a respective pixel value.

The height and width of each image in the set of images were obtained, and each image was preprocessed for analysis and visualization. Images were further downsized from high resolution to low resolution, and scale factors were obtained in order to map the coordinate positions of each respective pixel in the plurality of pixels in each high resolution image to a corresponding coordinate position of a respective pixel in the corresponding low resolution image. Moreover, for each image in the set of images, the corresponding plurality of pixels was aligned with the capture spots in the array, thus associating each capture spot in the array with a subset of pixels in the plurality of pixels. Alignment of images with capture spots can be performed, as described above, using one or more fiducial markers, such as those illustrated in FIG. 7C.

Measured intensities (e.g., of fluorescence) at each capture spot were obtained by determining a spot diameter for the respective capture spot, determining the pixel intensity for each respective pixel assigned to the respective capture spot, and obtaining an average of the pixel intensities across the subset of pixels associated with the respective capture spot. Measured intensities could be obtained using either high resolution images or low resolution images.

Accordingly, a first spatial dataset was generated including, for each respective capture spot in the plurality of capture spots, an identity of a respective spatial barcode associated with the capture spot, the coordinate position of the respective capture spot (e.g., row and column) in the array of capture spots, the coordinate position of the respective capture spot relative to a frame of reference for the set of images, one or more scale factors, one or more dimensions for the respective capture spot, and, for each respective image in the set of images, a corresponding measured intensity at the respective capture spot. Thus, the measured intensities for each capture spot were recorded as values in the first spatial dataset according to excitation wavelength (e.g., an array of values for each channel corresponding to each respective image in the set of images).

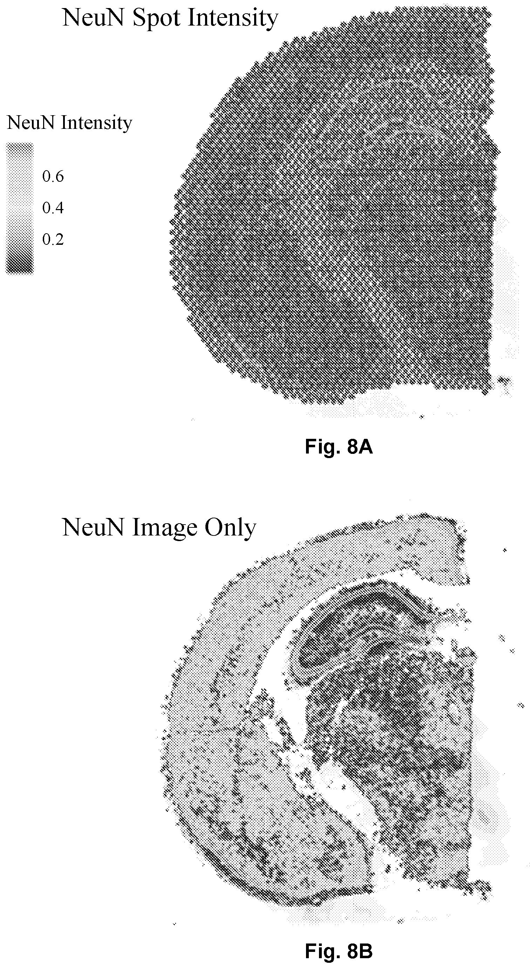

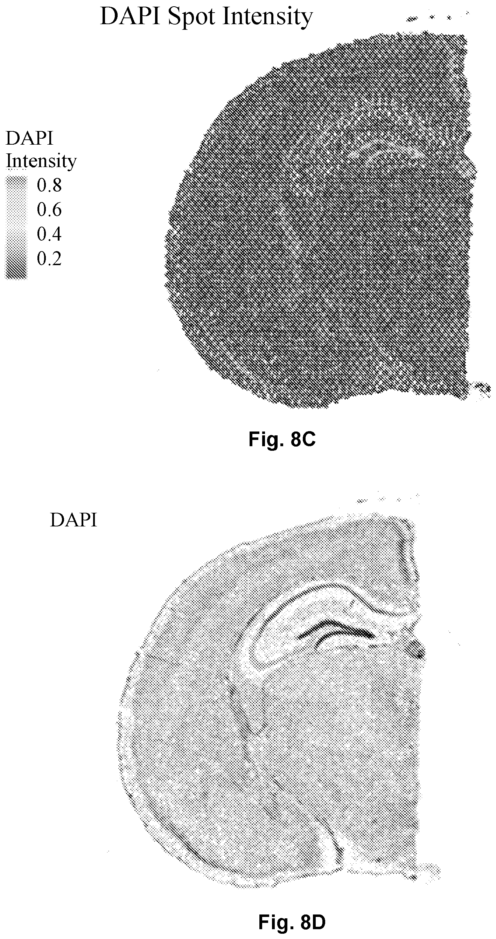

A color palette was selected for visual display, where measured intensities were represented using a range of colors in a spatial heatmap, as illustrated in grayscale in FIGS. 8A-D. Immunofluorescence intensities were plotted with and without capture spot assignments for the first emission image (FIGS. 8A: NeuN Spot Intensity and 8B: NeuN Image Only) and the second emission image (FIGS. 8C: DAPI Spot Intensity and 8D: DAPI Image Only).

### REFERENCES CITED AND ALTERNATIVE EMBODIMENTS

The present invention can be implemented as a computer program product that comprises a computer program mechanism embedded in a nontransitory computer readable storage medium. For instance, the computer program product could contain the program modules shown in **FIGS. 11A** and **11B****,** and/or described in **FIGS. 10A****,** **10B****,** **10C****,** **10D****,** and **10E****.** These program modules can be stored on a CD-ROM, DVD, magnetic disk storage product, USB key, or any other non-transitory computer readable data or program storage product.

As used herein, the terms "about" or "approximately" refer to an acceptable error range for a particular value as determined by one of ordinary skill in the art, which can depend in part on how the value is measured or determined, e.g., the limitations of the measurement system. For example, "about" can mean within 1 or more than 1 standard deviation, per the practice in the art. "About" can mean a range of ±20%, ±10%, ±5%, or ±1% of a given value. The term "about" or "approximately" can mean within an order of magnitude, within 5-fold, or within 2-fold, of a value. Where particular values are described in the application and claims, unless otherwise stated the term "about" meaning within an acceptable error range for the particular value should be assumed. The term "about" can have the meaning as commonly understood by one of ordinary skill in the art. The term "about" can refer to ±10%. The term "about" can refer to ±5%.

Where values are described in terms of ranges, it should be understood that the description includes the disclosure of all possible sub-ranges within such ranges, as well as specific numerical values that fall within such ranges irrespective of whether a specific numerical value or specific sub-range is expressly stated. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention. As used herein, the term "between" used in a range is intended to include the recited endpoints. For example, a number "between X and Y" can be X, Y, or any value from X to Y.

The term "each," when used in reference to a collection of items, is intended to identify an individual item in the collection but does not necessarily refer to every item in the collection, unless expressly stated otherwise, or unless the context of the usage clearly indicates otherwise.

As used herein, the singular forms "a," "an," and "the" include the plural forms as well, unless the context clearly dictates otherwise. It is further noted that the claims may be drafted to exclude any optional element. As such, this statement is intended to serve as antecedent basis for use of such exclusive terminology as "solely," "only," and the like in connection with the recitation of claim elements or use of a "negative" limitation. Furthermore, to the extent that the terms "including," "includes," "having," "has," "with," or variants thereof are used in either the detailed description and/or the claims, such terms are intended to be inclusive in a manner similar to the term "comprising."

The specific embodiments described herein are offered by way of example only. The embodiments were chosen and described in order to best explain the principles of the invention and its practical applications, to thereby enable others skilled in the art to best utilize the invention and various embodiments with various modifications as are suited to the particular use contemplated. The invention is to be limited only by the terms of the appended claims.

## Claims

1. A method for analyzing a tissue sample (1204), the method comprising:
using a computer system (1100) comprising one or more processing cores (1102) and a memory (1112):
obtaining a set of images (1122) of the tissue sample while the tissue sample is overlaid on a substrate (902) in a first orientation, wherein the substrate comprises a plurality of capture spots (601/1136), the plurality of capture spots comprises at least 1000 capture spots, and each respective image in the set of images is an emission image of the tissue sample upon excitation of the tissue sample at a corresponding excitation wavelength (1124) of a corresponding detectable marker, in a set of one or more detectable markers, associated with the respective image;
acquiring a first spatial dataset comprising (1134-1), for each respective capture spot in the plurality of capture spots, for each respective detectable marker in the set of detectable markers, a measured intensity (1144) of the respective capture spot, in the corresponding image in the set of images, indexed by a spatial barcode (1140) in a plurality of spatial barcodes that represents the respective capture spot;
acquiring a second spatial dataset (1134-2) comprising nucleic acid quantification data comprising, for each respective capture spot in the plurality of capture spots, for each respective nucleic acid in a plurality of nucleic acids, a corresponding representation of a number of molecules of the respective nucleic acid originating from the tissue sample while the tissue sample is overlaid on a substrate in the first orientation and localized to the respective capture spot by a corresponding spatial barcode, in the plurality of spatial barcodes, associated with the respective capture spot; and
spatially displaying all or a portion of the first spatial dataset and a corresponding all or a portion of the second spatial dataset co-registered to each other by the plurality of spatial barcodes on a display (1108), wherein
a first detectable marker in the set of detectable markers is indicative of a presence of a cell nucleus, and wherein the method further comprises using a measured intensity of the first detectable marker in each capture spot in the plurality of capture spots as indicated within the first spatial dataset to determine a corresponding estimate of a number of cells in each respective capture spot in the plurality of capture spots.

2. The method of claim 1, wherein the obtaining uses fluorescence microscopy to obtain each image in the set of images.

3. The method of claim 1 or 2, the method further comprising exposing, prior to the obtaining, the tissue sample on the substrate with each respective detectable marker in the set of detectable markers.

4. The method of any one of claims 1-3, wherein each respective detectable marker in the set of detectable markers is a fluorophore labeled antibody, a fluorescent label, a radioactive label, a chemiluminescent label, a colorimetric label, or a combination thereof.

5. The method of any one of claims 1-4, wherein a respective detectable marker in the set of detectable markers is live/dead stain, trypan blue, periodic acid-Schiff reaction stain, Masson's trichrome, Alcian blue, van Gieson, reticulin, Azan, Giemsa, Toluidine blue, isamin blue, sudan black and osmium, acridine orange, Bismarck brown, carmine, Coomassie blue, cresyl violet, DAPI, eosin, ethidium bromide, acid fuchsine, hematoxylin, Hoechst stains, iodine, methyl green, methylene blue, neutral red, Nile blue, Nile red, osmium tetroxide, propidium iodide, rhodamine, safranin, or a combination thereof.

6. The method of any one of claims 1-5, wherein the acquiring the second spatial dataset comprises:
obtaining a plurality of sequence reads, in electronic form, from the plurality of capture spots, wherein:
each respective capture spot in the plurality of capture spots includes a corresponding set of 1000 or more capture probes that directly or indirectly associates with one or more nucleic acids from the tissue sample,
the plurality of sequence reads comprises sequence reads corresponding to all or portions of the plurality of nucleic acids,
the plurality of sequence reads comprises at least 10,000 sequence reads, and
each respective sequence read in the plurality of sequence reads includes a spatial barcode of the corresponding capture spot in the plurality of capture spots or a complement thereof;
using all or a subset of the plurality of spatial barcodes to localize respective sequence reads in the plurality of sequence reads to corresponding capture spots in the plurality of capture spots, thereby dividing the plurality of sequence reads into a plurality of subsets of sequence reads, each respective subset of sequence reads corresponding to a different capture spot in the plurality of capture spots.

7. The method of claim 6, wherein each capture probe in the respective capture spot includes a poly-A sequence or a poly-T sequence and a unique spatial barcode that characterizes the respective capture spot,
each capture probe in the respective capture spot includes the same spatial barcode from the plurality of spatial barcodes, or
each capture probe in the respective capture spot includes a different spatial barcode from the plurality of spatial barcodes.

8. The method of claim 6 or 7, wherein each capture probe in the corresponding set of capture probes of a respective capture spot includes the same spatial barcode from the plurality of spatial barcodes.

9. The method of claim 8, wherein the corresponding representation of a number of molecules of the respective nucleic acid originating from the tissue sample while the tissue sample is overlaid on the substrate in the first orientation and localized to the respective capture spot by a corresponding spatial barcode, in the plurality of barcodes, associated with the respective capture spot is a count of a number of unique sequence reads in the plurality of sequence reads that have the spatial barcode associated with the respective capture spot.

10. The method of any one of claims 1-9, wherein the plurality of nucleic acids comprises DNA, RNA, or a combination thereof.

11. The method of any one of claims 1-9, wherein the plurality of nucleic acids comprises mRNA, microRNA, piRNA, nuclear RNA, or a combination thereof.

12. The method of any one of claims 1-11, wherein the tissue sample is a sectioned tissue sample that has a depth of 30 microns or less, a depth of 10 microns or less, or a depth of between 4 microns and 30 microns.

13. The method of any one of claims 1-12, wherein each respective capture spot in the plurality of capture spots is contained within a 2 micron by 2 micron square on the substrate, a distance between a center of each respective capture spot to a neighboring capture spot in the plurality of capture spots on the substrate is between 2 microns and 8 microns, or a shape of each capture spot in the plurality of capture spots on the substrate is a closed-form shape, optionally wherein the closed-form shape is elliptic or circular and each capture spot in the plurality of capture spots has a diameter of between 3 microns and 90 microns or optionally wherein the closed-form shape is elliptic or circular and each capture spot in the plurality of capture spots has a diameter of between 3 microns and 20 microns.

14. The method of any one of claims 1-13, wherein
a second detectable marker in the set of detectable markers is indicative of a presence of cell membrane, and wherein the method further comprises:
using a pattern of abundance of the second detectable marker in each capture spot in the plurality of capture spots as indicated within the first spatial dataset to validate the corresponding estimate of the number of cells in each respective capture spot in the plurality of capture spots.

15. A system (1100) for analyzing a tissue sample with a computer system comprising:
one or more processors;
a memory; and
one or more programs, wherein the one or more programs are stored in the memory and configured to be executed by the one or more processors, the one or more programs including instructions for performing the method for analyzing a tissue sample of any one of claims 1-14.

16. A computer readable storage medium storing one or more programs, the one or more programs comprising instructions, which when executed by the system (1100) for analyzing a tissue sample according to claim 15 cause the the system (1100) for analyzing a tissue sample to perform the method for analyzing a tissue sample of any one of claims 1-14.

## Patentansprüche

1. Verfahren zum Analysieren einer Gewebeprobe (1204), wobei das Verfahren umfasst:
unter Verwendung eines Computersystems (1100), umfassend einen oder mehrere Prozessorkerne (1102) und einen Speicher (1112):
Erhalten eines Satzes von Bildern (1122) der Gewebeprobe, während die Gewebeprobe in einer ersten Ausrichtung einem Substrat (902) überlagert ist, wobei das Substrat eine Vielzahl von Erfassungspunkten (601/1136) umfasst, die Vielzahl von Erfassungspunkten mindestens 1000 Erfassungspunkte umfasst und jedes jeweilige Bild in dem Satz von Bildern ein Emissionsbild der Gewebeprobe bei Anregung der Gewebeprobe bei einer entsprechenden Anregungswellenlänge (1124) eines entsprechenden detektierbaren Markers, aus einem Satz von einem oder mehreren detektierbaren Markern, ist, der mit dem jeweiligen Bild assoziiert ist;
Erfassen eines ersten räumlichen Datensatzes (1134-1), der für jeden jeweiligen Erfassungspunkt aus der Vielzahl von Erfassungspunkten für jeden jeweiligen detektierbaren Marker in dem Satz von detektierbaren Markern eine gemessene Intensität (1144) des jeweiligen Erfassungspunkts, in dem entsprechenden Bild in dem Satz von Bildern, umfasst, indexiert durch einen räumlichen Barcode (1140) aus einer Vielzahl von räumlichen Barcodes, der den jeweiligen Erfassungspunkt darstellt;
Erfassen eines zweiten räumlichen Datensatzes (1134-2), der Nukleinsäure-Quantifizierungsdaten umfasst, die für jeden jeweiligen Erfassungspunkt aus der Vielzahl von Erfassungspunkten, für jede jeweilige Nukleinsäure aus einer Vielzahl von Nukleinsäuren, eine entsprechende Darstellung einer Anzahl von Molekülen der jeweiligen Nukleinsäure umfassen, die von der Gewebeprobe stammen, während die Gewebeprobe in der ersten Ausrichtung einem Substrat überlagert ist und durch einen entsprechenden räumlichen Barcode aus der Vielzahl von räumlichen Barcodes, der mit dem jeweiligen Erfassungspunkt assoziiert ist, dem jeweiligen Erfassungspunkt assoziiert wird; und
räumliches Anzeigen der Gesamtheit oder eines Abschnitts des ersten räumlichen Datensatzes und einer entsprechenden Gesamtheit oder eines Abschnitts des zweiten räumlichen Datensatzes, die durch die Vielzahl von räumlichen Barcodes auf einer Anzeige (1108) zueinander ko-registriert sind, wobei
ein erster detektierbarer Marker in dem Satz von detektierbaren Markern auf ein Vorhandensein eines Zellkerns hinweist, und wobei das Verfahren ferner die Verwendung einer gemessenen Intensität des ersten detektierbaren Markers in jedem Erfassungspunkt aus der Vielzahl von Erfassungspunkten, wie innerhalb des ersten räumlichen Datensatzes angegeben, umfasst, um eine entsprechende Schätzung einer Anzahl von Zellen in jedem jeweiligen Erfassungspunkt aus der Vielzahl von Erfassungspunkten zu bestimmen.

2. Verfahren nach Anspruch 1, wobei das Erhalten Fluoreszenzmikroskopie verwendet, um jedes Bild in dem Satz von Bildern zu erhalten.

3. Verfahren nach Anspruch 1 oder 2, wobei das Verfahren, vor dem Erhalten, ferner das Exponieren der Gewebeprobe auf dem Substrat mit jedem jeweiligen detektierbaren Marker in dem Satz von detektierbaren Markern umfasst.

4. Verfahren nach einem der Ansprüche 1 bis 3, wobei jeder jeweilige detektierbare Marker in dem Satz von detektierbaren Markern ein Antikörper mit Fluorophor-Markierung, eine Fluoreszenzmarkierung, eine radioaktive Markierung, eine chemilumineszierende Markierung, eine kolorimetrische Markierung oder eine Kombination davon ist.

5. Verfahren nach einem der Ansprüche 1 bis 4, wobei ein jeweiliger detektierbarer Marker in dem Satz von detektierbaren Markern ein Lebend/Tot-Färbemittel, Trypanblau, Periodsäure-Schiff-Reaktionsfärbemittel, Masson-Trichrom, Alcianblau, van Gieson, Retikulin, Azan, Giemsa, Toluidinblau, Isaminblau, Sudanschwarz und Osmium, Acridinorange, Bismarckbraun, Karmin, Coomassieblau, Kresylviolett, DAPI, Eosin, Ethidiumbromid, saures Fuchsin, Hämatoxylin, Hoechst-Färbemittel, Jod, Methylgrün, Methylenblau, Neutralrot, Nilblau, Nilrot, Osmiumtetroxid, Propidiumiodid, Rhodamin, Safranin oder eine Kombination davon ist.

6. Verfahren nach einem der Ansprüche 1 bis 5, wobei das Erfassen des zweiten räumlichen Datensatzes umfasst:
Erhalten einer Vielzahl von Sequenz-Reads, in elektronischer Form, aus der Vielzahl von Erfassungspunkten, wobei:
jeder jeweilige Erfassungspunkt aus der Vielzahl von Erfassungspunkten einen entsprechenden Satz von 1000 oder mehr Erfassungssonden beinhaltet, die direkt oder indirekt mit einer oder mehreren Nukleinsäuren aus der Gewebeprobe assoziieren,
die Vielzahl von Sequenz-Reads Sequenz-Reads umfasst, die der Gesamtheit oder Abschnitten der Vielzahl von Nukleinsäuren entsprechen,
die Vielzahl von Sequenz-Reads mindestens 10.000 Sequenz-Reads umfasst, und
jede entsprechende Sequenz-Read aus der Vielzahl von Sequenz-Reads einen räumlichen Barcode des entsprechenden Erfassungspunkts aus der Vielzahl von Erfassungspunkten oder ein Komplement davon beinhaltet;
Verwenden der Gesamtheit oder einer Teilmenge der Vielzahl von räumlichen Barcodes, um jeweilige Sequenz-Reads aus einer Vielzahl von Sequenz-Reads räumlich zu entsprechenden Erfassungspunkten aus der Vielzahl von Erfassungspunkten zuzuordnen, wodurch die Vielzahl von Sequenz-Reads in eine Vielzahl von Teilmengen von Sequenz-Reads unterteilt wird, wobei jede jeweilige Teilmenge von Sequenz-Reads einem unterschiedlichen Erfassungspunkt aus der Vielzahl von Erfassungspunkten entspricht.

7. Verfahren nach Anspruch 6, wobei jede Erfassungssonde in dem jeweiligen Erfassungspunkt eine Poly-A-Sequenz oder eine Poly-T-Sequenz und einen eindeutigen räumlichen Barcode, der den jeweiligen Erfassungspunkt charakterisiert, beinhaltet,
jede Erfassungssonde in dem jeweiligen Erfassungspunkt den gleichen räumlichen Barcode aus der Vielzahl von räumlichen Barcodes beinhaltet, oder
jede Erfassungssonde in dem jeweiligen Erfassungspunkt einen unterschiedlichen räumlichen Barcode aus der Vielzahl von räumlichen Barcodes beinhaltet.

8. Verfahren nach Anspruch 6 oder 7, wobei jede Erfassungssonde in dem entsprechenden Satz von Erfassungssonden eines jeweiligen Erfassungspunkts denselben räumlichen Barcode aus der Vielzahl von räumlichen Barcodes beinhaltet.

9. Verfahren nach Anspruch 8, wobei die entsprechende Darstellung einer Anzahl von Molekülen der jeweiligen Nukleinsäure, die von der Gewebeprobe stammt, während die Gewebeprobe in einer ersten Ausrichtung einem Substrat überlagert ist und durch einen entsprechenden räumlichen Barcode, aus der Vielzahl von Barcodes, der mit dem jeweiligen Erfassungspunkt assoziiert ist, dem jeweiligen Erfassungspunkt räumlich zugeordnet wird, eine Zählung der Anzahl von eindeutigen Sequenz-Reads aus der Vielzahl von Sequenz-Reads ist, die den mit dem jeweiligen Erfassungspunkt assoziierten räumlichen Barcode aufweisen.

10. Verfahren nach einem der Ansprüche 1 bis 9, wobei die Vielzahl von Nukleinsäuren DNA, RNA oder eine Kombination davon umfasst.

11. Verfahren nach einem der Ansprüche 1 bis 9, wobei die Vielzahl von Nukleinsäuren mRNA, MikroRNA, piRNA, nukleare RNA oder eine Kombination davon umfasst.

12. Verfahren nach einem der Ansprüche 1 bis 11, wobei die Gewebeprobe eine geschnittene Gewebeprobe ist, die eine Tiefe von 30 Mikrometer oder weniger, eine Tiefe von 10 Mikrometer oder weniger oder eine Tiefe zwischen 4 Mikrometer und 30 Mikrometer aufweist.

13. Verfahren nach einem der Ansprüche 1 bis 12, wobei jeder jeweilige Erfassungspunkt aus der Vielzahl von Erfassungspunkten innerhalb eines 2 Mikrometer mal 2 Mikrometer großen Quadrats auf dem Substrat enthalten ist, ein Abstand zwischen einer Mitte von jedem jeweiligen Erfassungspunkt bis zu einem benachbarten Erfassungspunkt aus der Vielzahl von Erfassungspunkten auf dem Substrat zwischen 2 Mikrometer und 8 Mikrometer liegt oder eine Form jedes Erfassungspunkts aus der Vielzahl von Erfassungspunkten auf dem Substrat eine geschlossene Form ist, wobei die geschlossene Form optional elliptisch oder kreisförmig ist und jeder Erfassungspunkt aus der Vielzahl von Erfassungspunkten einen Durchmesser von zwischen 3 Mikrometer und 90 Mikrometer aufweist, oder wobei die geschlossene Form optional elliptisch oder kreisförmig ist und jeder Erfassungspunkt aus der Vielzahl von Erfassungspunkten einen Durchmesser von zwischen 3 Mikrometer und 20 Mikrometer aufweist.

14. Verfahren nach einem der Ansprüche 1 bis 13, wobei
ein zweiter detektierbarer Marker in dem Satz detektierbarer Marker indikativ für das Vorhandensein
einer Zellmembran ist, und wobei das Verfahren ferner umfasst:
Verwenden eines Häufigkeitsmusters des zweiten detektierbaren Markers in jedem Erfassungspunkt aus der Vielzahl von Erfassungspunkten, wie innerhalb des ersten räumlichen Datensatzes angegeben, um die entsprechende Schätzung der Anzahl von Zellen in jedem jeweiligen Erfassungspunkt aus der Vielzahl von Erfassungspunkten zu validieren.

15. System (1100) zum Analysieren einer Gewebeprobe mit einem Computersystem, umfassend:
einen oder mehrere Prozessoren;
einen Speicher; und
ein oder mehrere Programme, wobei das eine oder die mehreren Programme in dem Speicher gespeichert und eingerichtet sind, durch den einen oder die mehreren Prozessoren ausgeführt zu werden, wobei das eine oder die mehreren Programme Anweisungen zum Durchführen eines Verfahrens zum Analysieren einer Gewebeprobe nach einem der Ansprüche 1 bis 14 beinhalten.

16. Computerlesbares Speichermedium, das ein oder mehrere Programme speichert, wobei das eine oder die mehreren Programme Anweisungen umfassen, die, wenn sie durch das System (1100) zum Analysieren einer Gewebeprobe nach Anspruch 15 ausgeführt werden, das System (1100) zum Analysieren einer Gewebeprobe veranlassen, das Verfahren zum Analysieren einer Gewebeprobe nach einem der Ansprüche 1 bis 14 durchzuführen.

## Revendications

1. Procédé pour l'analyse d'un échantillon de tissu (1204), le procédé comprenant :
l'utilisation d'un système informatique (1100) comprenant un ou plusieurs cœurs de traitement (1102) et une mémoire (1112) :
l'obtention d'un ensemble d'images (1122) de l'échantillon de tissu tandis que l'échantillon de tissu est superposé sur un substrat (902) dans une première orientation, dans lequel le substrat comprend une pluralité de points de capture (601/1136), la pluralité de points de capture comprend au moins 1000 points de capture, et chaque image respective dans l'ensemble d'images est une image d'émission de l'échantillon de tissu lors de l'excitation de l'échantillon de tissu à une longueur d'onde d'excitation (1124) correspondante d'un marqueur détectable correspondant, dans un ensemble d'un ou plusieurs marqueurs détectables, associé à l'image respective ;
l'acquisition d'un premier ensemble de données spatiales comprenant (1134-1), pour chaque point de capture respectif dans la pluralité de points de capture, pour chaque marqueur détectable respectif dans l'ensemble de marqueurs détectables, une intensité mesurée (1144) du point de capture respectif, dans l'image correspondante dans l'ensemble d'images, indexée par un code à barres spatial (1140) dans une pluralité de codes à barres spatiaux qui représente le point de capture respectif ;
l'acquisition d'un deuxième ensemble de données spatiales (1134-2) comprenant des données de quantification d'acides nucléiques comprenant, pour chaque point de capture respectif dans la pluralité de points de capture, pour chaque acide nucléique respectif dans une pluralité d'acides nucléiques, une représentation correspondante d'un nombre de molécules de l'acide nucléique respectif provenant de l'échantillon de tissu tandis que l'échantillon de tissu est superposé sur un substrat dans la première orientation et localisé par rapport au point de capture respectif par un code à barres spatial correspondant, dans la pluralité de codes à barres spatiaux, associé au point de capture respectif ; et
l'affichage spatial de la totalité ou d'une portion du premier ensemble de données spatiales et de la totalité ou d'une portion correspondante du deuxième ensemble de données spatiales co-enregistrées les unes par rapport aux autres par la pluralité de codes à barres spatiaux sur un affichage (1108), dans lequel
un premier marqueur détectable dans l'ensemble de marqueurs détectables indique la présence d'un noyau cellulaire, et dans lequel le procédé comprend en outre l'utilisation d'une intensité mesurée du premier marqueur détectable dans chaque point de capture dans la pluralité de points de capture comme indiqué au sein du premier ensemble de données spatiales pour déterminer une estimation correspondante d'un nombre de cellules dans chaque point de capture respectif dans la pluralité de points de capture.

2. Procédé selon la revendication 1, dans lequel l'obtention utilise la microscopie à fluorescence pour obtenir chaque image dans l'ensemble d'images.

3. Procédé selon la revendication 1 ou 2, le procédé comprenant en outre l'exposition, avant l'obtention, de l'échantillon de tissu sur le substrat avec chaque marqueur détectable respectif dans l'ensemble de marqueurs détectables.

4. Procédé selon l'une quelconque des revendications 1 à 3, dans lequel chaque marqueur détectable respectif dans l'ensemble de marqueurs détectables est un anticorps étiqueté par un fluorophore, une étiquette fluorescente, une étiquette radioactive, une étiquette chimioluminescente, une étiquette colorimétrique ou une combinaison de ceux-ci.

5. Procédé selon l'une quelconque des revendications 1 à 4, dans lequel un marqueur détectable respectif dans l'ensemble de marqueurs détectables est un colorant de cellules mortes live/dead, le bleu trypan, un colorant à l'acide périodique de Schiff, le trichrome de Masson, le bleu Alcian, le van Gieson, la réticuline, l'Azan, le Giemsa, le bleu de toluidine, le bleu d'isamine, le soudan noir et l'osmium, l'orange d'acridine, le brun Bismarck, le carmin, le bleu de Coomassie, le violet de crésyl, le DAPI, l'éosine, le bromure d'éthidium, la fuchsine acide, l'hématoxyline, les colorants Hoechst, l'iode, le vert de méthyle, le bleu de méthylène, le rouge neutre, le bleu de Nil, le rouge de Nil, le tétroxyde d'osmium, l'iodure de propidium, la rhodamine, la safranine, ou une combinaison de ceux-ci.

6. Procédé selon l'une quelconque des revendications 1 à 5, dans lequel l'acquisition du deuxième ensemble de données spatial comprend :
l'obtention d'une pluralité de lectures de séquences, sous forme électronique, à partir de l'ensemble de points de capture, dans lequel :
chaque point de capture respectif dans la pluralité de points de capture comporte un ensemble correspondant de 1000 sondes de capture ou plus qui s'associent directement ou indirectement à un ou plusieurs acides nucléiques provenant de l'échantillon de tissu,
la pluralité de lectures de séquences comprend des lectures de séquences correspondant à la totalité ou à des portions de la pluralité d'acides nucléiques,
la pluralité de lectures de séquences comprend au moins 10 000 lectures de séquences, et
chaque lecture de séquences respective dans la pluralité de lectures de séquences comporte un code à barres spatial du point de capture correspondant dans la pluralité de points de capture ou un complément de celui-ci ;
l'utilisation de la totalité ou d'un sous-ensemble parmi la pluralité de codes à barres spatiaux pour localiser des lectures de séquences respectives dans la pluralité de lectures de séquences à des points de capture correspondants dans la pluralité de points de capture, divisant ainsi la pluralité de lectures de séquences en une pluralité de sous-ensembles de lectures de séquences, chaque sous-ensemble respectif de lectures de séquences correspondant à un point de capture différent dans la pluralité de points de capture.

7. Procédé selon la revendication 6, dans lequel chaque sonde de capture dans le point de capture respectif comporte une séquence poly-A ou une séquence poly-T et un code à barres spatial unique qui caractérise le point de capture respectif,
chaque sonde de capture dans le point de capture respectif comporte le même code à barres spatial parmi la pluralité de codes à barres spatiaux, ou
chaque sonde de capture dans le point de capture respectif comporte un code à barres spatial différent parmi la pluralité de codes à barres spatiaux.

8. Procédé selon la revendication 6 ou 7, dans lequel chaque sonde de capture dans l'ensemble correspondant de sondes de capture d'un point de capture respectif comporte le même code à barres spatial parmi la pluralité de codes à barres spatiaux.

9. Procédé selon la revendication 8, dans lequel la représentation correspondante d'un nombre de molécules de l'acide nucléique respectif provenant de l'échantillon de tissu tandis que l'échantillon de tissu est superposé sur le substrat dans la première orientation et localisé par rapport au point de capture respectif par un code à barres spatial correspondant, dans la pluralité de codes à barres, associé au point de capture respectif est un comptage d'un nombre de lectures de séquences uniques dans la pluralité de lectures de séquences qui ont le code à barres spatial associé au point de capture respectif.

10. Procédé selon l'une quelconque des revendications 1 à 9, dans lequel la pluralité d'acides nucléiques comprend ADN, ARN ou une combinaison de ceux-ci.

11. Procédé selon l'une quelconque des revendications 1 à 9, dans lequel la pluralité d'acides nucléiques comprend ARNm, microARN, ARNpi, ARN nucléaire, ou une combinaison de ceux-ci.

12. Procédé selon l'une quelconque des revendications 1 à 11, dans lequel l'échantillon de tissu est un échantillon de tissu sectionné qui a une profondeur de 30 microns ou moins, une profondeur de 10 microns ou moins, ou une profondeur d'entre 4 microns et 30 microns.

13. Procédé selon l'une quelconque des revendications 1 à 12, dans lequel chaque point de capture respectif dans la pluralité de points de capture est contenu au sein d'un carré de 2 microns par 2 microns sur le substrat, une distance entre un centre de chaque point de capture respectif et un point de capture voisin dans la pluralité de points de capture sur le substrat est entre 2 microns et 8 microns, ou une forme de chaque point de capture dans la pluralité de points de capture sur le substrat est une forme fermée, facultativement dans lequel la forme fermée est elliptique ou circulaire et chaque point de capture dans la pluralité de points de capture a un diamètre d'entre 3 microns et 90 microns, ou facultativement dans lequel la forme fermée est elliptique ou circulaire et chaque point de capture dans la pluralité de points de capture a un diamètre d'entre 3 microns et 20 microns.

14. Procédé selon l'une quelconque des revendications 1 à 13, dans lequel
un deuxième marqueur détectable dans l'ensemble de marqueurs détectables indique une présence de membrane cellulaire, et dans lequel le procédé comprend en outre :
l'utilisation d'un motif d'abondance du deuxième marqueur détectable dans chaque point de capture dans la pluralité de points de capture comme indiqué au sein du premier ensemble de données spatiales pour valider l'estimation correspondante du nombre de cellules dans chaque point de capture respectif dans la pluralité de points de capture.

15. Système (1100) pour l'analyse d'un échantillon de tissu avec un système informatique comprenant :
un ou plusieurs processeurs ;
une mémoire ; et
un ou plusieurs programmes, dans lequel les un ou plusieurs programmes sont stockés dans la mémoire et configurés pour être exécutés par les un ou plusieurs processeurs, les un ou plusieurs programmes comportant des instructions pour la réalisation du procédé d'analyse d'un échantillon de tissu de l'une quelconque des revendications 1 à 14.

16. Support de stockage lisible par ordinateur stockant un ou plusieurs programmes, les un ou plusieurs programmes comprenant des instructions qui, lorsqu'elles sont exécutées par le système (1100) pour analyser un échantillon de tissu selon la revendication 15, amènent le système (1100) destiné à analyser un échantillon de tissu à réaliser le procédé d'analyse d'un échantillon de tissu de l'une quelconque des revendications 1 à 14.
